(19) **Europäisches Patentamt / European Patent Office / Office européen des brevets**

(11) **EP 3 282 256 A1**

(12) **EUROPEAN PATENT APPLICATION**
published in accordance with Art. 153(4) EPC

(43) Date of publication:
**14.02.2018 Bulletin 2018/07**

(21) Application number: **16776705.2**

(22) Date of filing: **08.04.2016**

(51) Int Cl.:
**G01N 33/68** (2006.01)  **G01N 21/27** (2006.01)
**G01N 33/48** (2006.01)  **G01N 33/483** (2006.01)
**G01N 33/53** (2006.01)

(86) International application number:
**PCT/JP2016/061609**

(87) International publication number:
**WO 2016/163539 (13.10.2016 Gazette 2016/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.04.2015 JP 2015081175**

(71) Applicants:
  • **Social Welfare Organization Saiseikai Imperial Gift Foundation, Inc.**
    **Tokyo 108-0073 (JP)**
  • **Sumitomo Dainippon Pharma Co., Ltd.**
    **Osaka-shi**
    **Osaka 541-8524 (JP)**

(72) Inventors:
  • **OKANOUE Takeshi**
    **Suita-shi**
    **Osaka 564-0013 (JP)**

  • **YOSHIMURA Keito**
    **Osaka-shi**
    **Osaka 554-0022 (JP)**
  • **IWASAKI Tsuyoshi**
    **Osaka-shi**
    **Osaka 554-0022 (JP)**
  • **YAMAZAKI Kazuto**
    **Osaka-shi**
    **Osaka 554-0022 (JP)**
  • **EBISE Hayao**
    **Osaka-shi**
    **Osaka 554-0022 (JP)**
  • **ICHIHARA Junji**
    **Suita-shi**
    **Osaka 564-0053 (JP)**

(74) Representative: **Hoffmann Eitle**
    **Patent- und Rechtsanwälte PartmbB**
    **Arabellastraße 30**
    **81925 München (DE)**

(54) **METHOD FOR DISCRIMINATING SYMPTOM OF HEPATIC DISEASE**

(57)  Provided is a noninvasive method for determining the presence of a symptom of a hepatic disease such as nonalcoholic fatty liver disease with a higher diagnostic accuracy. A method for discriminate between nonalcoholic fatty liver (NAFL) and nonalcoholic steatohepatitis (NASH), the method comprising: (1) a step of measuring the quantities of marker molecules contained in blood collected from a subject; (2) a step of determining an index value from a normalized score calculated based on the quantities of the marker molecules belonging to the same group; and (3) a step of determining that the subject is possibly affected with NASH in the case that the index value is larger than a reference value.

**EP 3 282 256 A1**

## Description

### Technical Field

The present invention relates to a method for determining the presence of a symptom of a hepatic disease. In addition, the present invention relates to a method for discriminating between nonalcoholic fatty liver (NAFL) and non-alcoholic steatohepatitis (NASH), and a method for determining the presence of hepatic fibrosis.

### Background Art

Nonalcoholic fatty liver disease (NAFLD) is a hepatic disease closely relating to lifestyle diseases including diabetes and obesity. Along with the recent increase of the number of individuals affected with a lifestyle disease, the number of NAFLD patients is increasing in the world. NAFLD ranges from nonalcoholic fatty liver (NAFL) with a good prognosis to nonalcoholic steatohepatitis (NASH) with a poor prognosis. NASH is known to cause inflammation, degeneration or hepatocellular necrosis, or fibrosis. When NASH has progressed, hepatic cirrhosis or liver cancer may be caused. In addition, the number of NASH patients is recently increasing. Thus, diagnosis of NASH and suitable treatment thereof are strongly required.

### Citation List

### Non Patent Literature

Non Patent Literature 1: Poynard, T. et al. Diagnostic value of biochemical markers (NashTest) for the prediction of non alcoholo steato hepatitis in patients with non-alcoholic fatty liver disease. BMC Gastroenterol. 6, 34 (2006).
Non Patent Literature 2: Younossi, Z. M. et al. A novel diagnostic biomarker panel for obesity-related nonalcoholic steatohepatitis (NASH). Obes. Surg. 18, 1430-7 (2008).
Non Patent Literature 3: Sumida, Y. et al. A simple clinical scoring system using ferritin, fasting insulin, and type IV collagen 7S for predicting steatohepatitis in nonalcoholic fatty liver disease. J. Gastroenterol. 46, 257-68 (2011).

### Summary of Invention

### Technical Problem

Currently, only histopathology through a liver biopsy is approved in the clinical practice guidelines (Evidence-based Clinical Practice Guidelines for NAFLD/NASH 2014, edited by The Japanese Society of Gastroenterology, Nankodo Co., Ltd., 2014) as a method for definitive diagnosis of NASH. A liver biopsy is a highly invasive technique, in which a liver tissue is sampled by puncturing. Since a liver biopsy typically requires hospitalization and high cost, for example, a liver biopsy is not applied in many cases even when an individual is suspected to be affected with NASH. Thus, a liver biopsy is not an adequate method for diagnosing many patients. Because of such a circumstance, development of a diagnosis method (determination method) with a noninvasive diagnostic marker for NASH (a marker for diagnosing NASH) without need of a liver biopsy has been looked forward to.

Hepatic fibrosis, one of manifestations of NASH, has a large impact on a prognosis of NASH (J Hepatol. 2005 Jan; 42 (1): 132-8), and thus it is important to determine the presence of hepatic fibrosis in diagnosis and treatment of NASH. Currently, a liver biopsy is needed for definitive diagnosis also in determination of the presence or absence of hepatic fibrosis or evaluation of the degree of progression thereof. Because of such a circumstance, development of a method for determining the presence of NASH with hepatic fibrosis and a technique for evaluating the degree of progression thereof also has been looked forward to.

Many noninvasive diagnostic markers for NASH have been previously reported. In the current clinical practice guidelines, "steatosis, inflammation, hepatocyte disorder (ballooning degeneration)" are listed as the features of NASH. In addition, NASH includes cases with hepatic fibrosis. For diagnosis of NAFL-NASH, Matteoni classification (Matteoni, C., Younossi, Z. & Gramlich, T. Nonalcoholic fatty liver disease: a spectrum of clinical and pathological severity. Gastroenterology 1413-1419 (1999).) based on pathological findings from such a liver biopsy is widely used. Most of the existing diagnosis methods for NASH are based on such symptoms characteristic to NASH, and use combination of markers for inflammation, markers for hepatocyte disorder, markers for fibrosis, and so on. Among the existing markers, markers for which the true diagnostic accuracy has been confirmed through a verification based on independent patient groups are only NashTest (Non Patent Literature 1), NASH Diagnostics (Non Patent Literature 2), and NAFIC score (Non Patent Literature 3). In general, the diagnostic accuracy for NASH is indicated by an area under a receiver operating characteristic curve (ROC) (area under the Receiver Operating Characteristic Curve: AUROC) of the sensitivity and specificity with respect to how accurately NASH patients can be diagnosed among NAFLD patients. The values of AUROC of NAFL-NASH diagnosis (diagnosis based on pathological findings) for learning/verification data in the above-mentioned three existing diagnosis methods are 0.69/0.80, 0.91/0.70, and 0.85/0.78, respectively. These diagnosis methods are not approved as being applicable to definitive diagnosis for NASH in the current clinical practice guidelines, and a still higher diagnostic accuracy is required to provide an alternative to definitive diagnosis through a liver biopsy.

The present invention was made in view of the above-described circumstances, and an object of the present invention is to provide a noninvasive method for determining the disease type or determining the presence of a symptom of a hepatic disease such as nonalcoholic fatty liver disease with a higher diagnostic accuracy.

**Solution to Problem**

The present inventors diligently studied, and found that use of a group of marker molecules biologically controlled by each other enables determination of the presence of a symptom of a hepatic disease such as nonalcoholic fatty liver disease in a noninvasive method with a diagnostic accuracy higher than those of conventional methods, and thus completed the present invention.

Specifically, provided is a method comprising:

(1) a step of measuring the quantity of a marker molecule contained in blood collected from a subject, wherein the marker molecule to be measured is

(A) at least one marker molecule in any one group of the following Group 1, the following Group 2, the following Group 3, and the following Group 4:

Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2,
Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40,
Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone, and
Group 4 consisting of AREG, BDNF, CD40-L, EREG, FGF2, HBEGF, IGFBP2, MMP9, PAI-1, PDGFB, PLGF, SAP, TGFA, TGFB1, THBS1, and VTN,
or,

(B) at least one marker molecule selected from one of two groups selected from the Group 1, the Group 2, the Group 3, and the Group 4 and at least one marker molecule selected from the other;

(2) a step of determining an index value from a normalized score calculated based on the quantities of the marker molecules belonging to the same group; and
(3) a step of determining that the subject is possibly affected with nonalcoholic steatohepatitis (NASH) or that the subject possibly has a symptom of hepatic fibrosis by comparing the index value with a reference value.

More specifically, the present invention relates to the following.

[1] A method for discriminating between nonalcoholic fatty liver (NAFL) and nonalcoholic steatohepatitis (NASH), the method comprising:

(1) a step of measuring the quantities of marker molecules contained in blood collected from a subject, wherein the marker molecules to be measured are

(A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2,
at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40,
at least two marker molecules selected from Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone,
or

at least two marker molecules selected from Group 4 consisting of AREG, BDNF, CD40-L, EREG, FGF2, HBEGF, IGFBP2, MMP9, PAI-1, PDGFB, PLGF, SAP, TGFA, TGFB1, THBS1, and VTN,
or,
(B) at least two marker molecules selected from one of two groups selected from the Group 1, the Group 2, the Group 3, and the Group 4 and at least two marker molecules selected from the other;

(2) a step of determining an index value from a normalized score calculated based on the quantities of the marker molecules, wherein the normalized score based on the quantities of the marker molecules belonging to the same group is calculated by using the following formula 1:

$$M = \frac{1}{N} \sum_{i=1}^{N} \frac{1}{1 + exp\left(-\alpha_i \times \dfrac{m_i - mean(m_i)}{sd(m_i)}\right)}$$

$$\alpha_i = \begin{cases} 1 & \left(mean\left(m_i^{NASH}\right) > mean\left(m_i^{NAFL}\right)\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL, and,
in the case that the marker molecules correspond to the (A), the index value is the normalized score, and
in the case that the marker molecules correspond to the (B), the index value is calculated from normalized scores for the two groups; and
(3) a step of determining that the subject is affected with or possibly affected with NASH in the case that the index value is larger than a reference value.

[2] The method according to the above [1], wherein, in the case of the (B), the index value is calculated from normalized scores for the two groups by using a linear equation or a nonlinear equation.
[3] A method for discriminating between nonalcoholic fatty liver (NAFL) and nonalcoholic steatohepatitis (NASH), the method comprising:

(1) a step of measuring the quantities of marker molecules contained in blood collected from a subject, wherein the marker molecules to be measured are

(A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2,
at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40,
at least two marker molecules selected from Group 3 consisting of AST, PSAT, LEP, ICAM1, CK-18, GSTA1, ALT, INS^IVD, INS^MAP, 6Ckine, AGT, CRP, CXCL10, FABP4, G6PI, HSP-60, P3NP, and testosterone,
or
at least two marker molecules selected from Group 4 consisting of AREG, BDNF, CD40-L, EREG, FGF2, HBEGF, IGFBP2, MMP9, PAI-1, PDGFB, PLGF, SAP, TGFA, TGFB1, THBS 1, and VTN,
or
(B) at least two marker molecules selected from one of two groups selected from the Group 1, the Group 2, the Group 3, and the Group 4 and at least two marker molecules selected from the other;

(2) a step of determining an index value from a normalized score calculated based on the quantities of the marker molecules, wherein the normalized score based on the quantities of the marker molecules belonging to the same group is calculated by using the following formula 1:

$$M = \frac{1}{N} \sum_{i=1}^{N} \frac{1}{1 + exp\left(-\alpha_i \times \frac{m_i - mean(m_i)}{sd(m_i)}\right)}$$

$$\alpha_i = \begin{cases} 1 & \left(mean(m_i^{NASH}) > mean(m_i^{NAFL})\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL, and,
in the case that the marker molecules correspond to the (A), the index value is the normalized score, and
in the case that the marker molecules correspond to the (B), the index value is calculated from normalized scores for the two groups by using a method with an equation for mean calculation, a method with a linear regression equation, a method with a linear discriminant, a method with a logistic regression equation, a method with a nonlinear equation for mean calculation, a method with a nonlinear regression equation, a method with a nonlinear discriminant, or a method with a nonlinear logistic regression equation; and
(3) a step of determining that the subject is affected with or possibly affected with NASH in the case that the index value is larger than a reference value.

[4] The method according to any one of the above [1] to [3], wherein, in the case of the (B), the index value is calculated from normalized scores for the two groups by using the following formula 2, as a method with a logistic regression equation:

$$I = a_1 \times M_{a1} + a_2 \times M_{a2} \text{ (formula 2)}$$

wherein I denotes an index value; $M_{a1}$ and $M_{a2}$ denote a normalized score for one of the two groups and a normalized score for the other, respectively; and $a_1$ and $a_2$ each denote a constant determined in logistic regression analysis.
[5] The method according to any one of the above [1] to [3], wherein, in the case of the (B), the index value is calculated from normalized scores for the two groups by using any one of the following Decision trees 1 to 6, as a method with a nonlinear discriminant:

Decision tree 1: in the case that the marker molecules are selected from the Group 1 and Group 2, if $M1_a \geq 0.476$, then I = 2, if $M1_a < 0.476$ and $M2_a \geq 0.466$, then I = 1, and if $M1_a < 0.476$ and $M2_a < 0.466$, then I = 0;
Decision tree 2: in the case that the marker molecules are selected from the Group 1 and Group 3, if $M3_a \geq 0.409$ and $M1_a \geq 0.45$, then I = 2, if $M3_a \geq 0.409$ and $M1_a < 0.45$, then I = 1, and if $M3_a < 0.409$, then I = 0;
Decision tree 3: in the case that the marker molecules are selected from the Group 1 and Group 4, if $M4_a \geq 0.547$, then I = 2, if $M4_a < 0.547$ and $M1_a \geq 0.468$, then I = 1, and if $M4_a < 0.547$ and $M1_a < 0.468$, then I = 0;
Decision tree 4: in the case that the marker molecules are selected from the Group 2 and Group 3, if $M3_a \geq 0.409$ and $M2_a \geq 0.412$, then I = 2, if $M3_a \geq 0.409$ and $M2_a < 0.412$, then I = 1, and if $M3_a < 0.409$, then I = 0;
Decision tree 5: in the case that the marker molecules are selected from the Group 2 and Group 4, if $M4_a \geq 0.547$, then I = 2, if $M4_a < 0.547$ and $M2_a \geq 0.413$, then I = 1, and if $M4_a < 0.547$ and $M2_a < 0.413$, then I = 0; and

Decision tree 6: in the case that the marker molecules are selected from the Group 3 and Group 4, if $M3_a \geq$ 0.409, then I = 2, if $M3_a < 0.409$ and $M4_a \geq 0.588$, then I = 1, and if $M3_a < 0.409$ and $M4_a < 0.588$, then I = 0,

wherein I denotes an index value; and $M1_a$, $M2_a$, $M3_a$, and $M4_a$ denote normalized scores for the Group 1, Group 2, Group 3, and Group 4, respectively.

[6] The method according to any one of the above [1] to [3], wherein, in the case of the (A), the marker molecules are PROS1 and CLU, each belonging to the Group 1.

[7] The method according to any one of the above [1] to [3], wherein, in the case of the (A), the marker molecules are VCAM1 and HA; VCAM1 and CTSD; VCAM1 and COL4; VCAM1 and COL4-7S; HA and COL4-7S; HA and CTSD; HA and COL4; CTSD and COL4; or CTSD and COL4-7S, each belonging to the Group 2.

[8] The method according to any one of the above [1] to [3], wherein, in the case of the (A), the marker molecules are AST and PSAT; AST and LEP; AST and ICAM1; AST, PSAT, and LEP; AST, PSAT, and ICAM1; AST, LEP, and CK-18; AST, ICAM1, and GSTA1; or AST, PSAT, and 6Ckine, each belonging to the Group 3.

[9] The method according to any one of the above [1] to [5], wherein, in the case of combination of the Group 1 and the Group 3 in the (B), the marker molecules selected from the Group 1 are PROS1 and CLU each belonging to the Group 1, and the marker molecules selected from the Group 3 are AST and PSAT; AST and LEP; AST and ICAM1; AST, PSAT, and LEP; AST, PSAT, and ICAM1; AST, LEP, and CK-18; AST, ICAM1, and GSTA1; or AST, PSAT, and 6Ckine.

[10] A method for determining the presence of hepatic fibrosis, the method comprising:

a step of measuring the quantities or quantity of marker molecules or a marker molecule contained in blood collected from a subject, wherein the marker molecules or marker molecule to be measured are/is at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40, or VCAM1;

a step of determining that the subject has or possibly has a symptom of hepatic fibrosis in the case that a normalized score calculated based on the quantities or quantity of the marker molecules or maker molecule is larger than a reference value, wherein the normalized score is calculated by using the following formula 1:

$$M = \frac{1}{N}\sum_{i=1}^{N}\frac{1}{1 + exp\left(-\alpha_i \times \frac{m_i - mean(m_i)}{sd(m_i)}\right)}$$

$$\alpha_i = \begin{cases} 1 & \left(mean(m_i^{NASH}) > mean(m_i^{NAFL})\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL.

[11] The method for determining the presence of hepatic fibrosis according to the above [10], wherein the marker molecules or marker molecule are/is VCAM1, HA, CTSD, COL4, COL4-7S, ALB, AFP, AXL, CCL19, CGB, CSF1, FAS, Mac-2bp, CA19-9, NRCAM, OPG, VWF, and YKL-40; VCAM1; VCAM1 and HA; VCAM1 and CTSD; VCAM1 and COL4; VCAM1 and COL4-7S; HA and CTSD; CTSD and COL4; HA and COL4; HA and COL4-7S; or CTSD and COL4-7S.

[12] The method according to the above [10] or [11], wherein determination of the presence of hepatic fibrosis is based on discrimination between symptoms of Type-1 to Type-3 and a symptom of Type-4 in Matteoni classification, or discrimination between a symptom of Stage 0, Stage 1, or Stage 2 and a symptom of Stage 3 or Stage 4 in Stages of fibrosis.

[13] A method for determining the degree of progression of a symptom of a hepatic disease, the method comprising:

a step of measuring the quantity or quantities of a marker molecule or marker molecules contained in blood collected from a subject, wherein the marker molecule or marker molecules to be measured is/are VCAM1; VCAM1 and HA; HA and COL4; VCAM1 and COL4; HA and COL4-7S; or VCAM1 and COL4-7S; and

a step of determining that the degree of progression of hepatic fibrosis, the degree of progression of hepatocellular ballooning degeneration, or the degree of progression of hepatic inflammation in the subject is higher or possibly higher as a normalized score calculated based on the quantity or quantities of the marker molecule or molecules is higher, wherein the normalized score is calculated by using the following formula 1:

$$M = \frac{1}{N} \sum_{i=1}^{N} \frac{1}{1 + exp\left(-\alpha_i \times \dfrac{m_i - mean(m_i)}{sd(m_i)}\right)}$$

$$\alpha_i = \begin{cases} 1 & \left(mean\left(m_i^{NASH}\right) > mean\left(m_i^{NAFL}\right)\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule in patients presenting with NASH; and mean ($M_i^{NAFL}$) denotes a predetermined mean of the quantity of each marker molecule in patients presenting with NAFL.

[14] The method according to [13], wherein the marker molecule or marker molecules to be measured is/are VCAM1; VCAM1 and HA; VCAM1 and COL4; HA and COL4-7S; or VCAM1 and COL4-7S.

[15] A method for determining the degree of progression of a symptom of nonalcoholic fatty liver disease (NAFLD), the method comprising:

a step of measuring the quantity or quantities of a marker molecule or marker molecules contained in blood collected from a subject, wherein the marker molecule or marker molecules to be measured is/are VCAM1; VCAM1 and HA; HA and COL4; VCAM1 and COL4; HA and COL4-7S; or VCAM1 and COL4-7S; and

a step of determining that the degree of progression of hepatic fibrosis, the degree of progression of the activity of nonalcoholic fatty liver disease, the degree of progression of hepatocellular ballooning degeneration, or the degree of progression of hepatic inflammation in the subject is higher or possibly higher as a normalized score calculated based on the quantity or quantities of the marker molecule or molecules is higher, wherein the normalized score is calculated by using the following formula 1:

$$M = \frac{1}{N}\sum_{i=1}^{N} \frac{1}{1 + exp\left(-\alpha_i \times \dfrac{m_i - mean(\mathrm{m}_i)}{sd(\mathrm{m}_i)}\right)}$$

$$\alpha_i = \begin{cases} 1 & \left(mean(\mathrm{m}_i^{NASH}) > mean(\mathrm{m}_i^{NAFL})\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule in patients presenting with NASH; and mean ($m_i^{NAFL}$) denotes a predetermined mean of the quantity of each marker molecule in patients presenting with NAFL.

[16] The method according to [15], wherein the marker molecule or marker molecules to be measured is/are VCAM1; VCAM1 and HA; VCAM1 and COL4; HA and COL4-7S; or VCAM1 and COL4-7S.

[17] A kit for use in the method according to any one of the above [1] to [16], the kit comprising:

a reagent for detecting the marker molecule or marker molecules; and
an instruction for performing the method.

[18] A method for discriminating between nonalcoholic fatty liver (NAFL) and nonalcoholic steatohepatitis (NASH), the method comprising:

a step of measuring the quantity of VCAM1 contained in blood collected from a subject; and
a step of determining that the subject is affected with or possibly affected with NASH in the case that the quantity of VCAM1 is larger than a reference value.

[19] A method for determining the presence of hepatic fibrosis, the method comprising:

a step of measuring the quantity of VCAM1 contained in blood collected from a subject; and
a step of determining that the subject has or possibly has a symptom of hepatic fibrosis in the case that the quantity of VCAM1 is larger than a reference value.

[20] A method for determining the degree of progression of a symptom of a hepatic disease, the method comprising:

a step of measuring the quantity of VCAM1 contained in blood collected from a subject; and
a step of determining that the degree of progression of hepatic fibrosis, the degree of progression of hepatocellular ballooning degeneration, or the degree of progression of hepatic inflammation in the subject is higher or possibly higher as the quantity of VCAM1 is larger.

[21] A method for determining the degree of progression of a symptom of nonalcoholic fatty liver disease (NAFLD), the method comprising:

a step of measuring the quantity of VCAM1 contained in blood collected from a subject; and
a step of determining that the degree of hepatic fibrosis, the degree of progression of the activity of nonalcoholic fatty liver disease, the degree of progression of hepatocellular ballooning degeneration, or the degree of progression of hepatic inflammation in the subject is higher or possibly higher as the quantity of VCAM1 is larger.

[22] A kit for use in the method according to any one of the above [18] to [21], the kit comprising:

a reagent for detecting VCAM 1.

[23] A program for use in the method according to any one of the above [1] to [16], wherein the program allows a computer to execute:

a normalization step of calculating a normalized score or normalized scores from measurement data on the quantity or quantities of the marker molecule or marker molecules contained in blood collected from a subject by using the formula 1 and then determining an index value from the normalized score or normalized scores calculated;
a comparison step of comparing the index value determined with a reference value; and
a determination step of determining whether the subject has a symptom of a hepatic disease based on a result of the comparison.

[24] A program for use in the method according to any one of the above [18] to [21], wherein the program allows a computer to execute:

a comparison step of comparing the quantity of VCAM1 contained in blood collected from a subject with a reference value; and
a determination step of determining whether the subject has a symptom of a hepatic disease based on a result of the comparison.

[25] The program according to the above [23] or [24], wherein the program allows a computer to further execute an acquisition step of acquiring measurement data on the quantity or quantities of the marker molecule or marker molecules contained in blood collected from a subject or the quantity of the VCAM1 contained in blood collected from a subject.
[26] A measurement apparatus for use in the method according to any one of the above [1] to [16] and [18] to [21], the measuring apparatus comprising:

a sample-setting part configured to set a sample therein; a light source part configured to irradiate the sample with light; and a detection part configured to detect light transmitted through the sample.

[27] A hepatic disease determination system for use in the method according to any one of the above [1] to [16], the hepatic disease determination system comprising:

a measurement apparatus configured to measure the quantity or quantities of the marker molecule or marker molecules contained in blood collected from a subject; and a hepatic disease determination apparatus configured to determine the presence of a symptom of a hepatic disease based on the quantity or quantities of the marker molecule or marker molecules measured, wherein
the measuring apparatus comprises a sample-setting part configured to set a sample therein, a light source part configured to irradiate the sample with light, and a detection part configured to detect light transmitted through the sample, and
the hepatic disease determination apparatus comprises an acquisition unit configured to acquire measurement data from the measurement apparatus, a normalization unit configured to calculate a normalized score or normalized scores from the measurement data by using the formula 1 and then determine an index value from the normalized score or normalized scores calculated, a comparison unit configured to compare the index value determined with a reference value, and a determination unit configured to determine whether the subject has a symptom of a hepatic disease based on a result of the comparison.

[28] A hepatic disease determination system for use in the method according to any one the above [18] to [21], the hepatic disease determination system comprising:

a measurement apparatus configured to measure the quantity of VCAM1 contained in blood collected from a subject; and a hepatic disease determination apparatus configured to determine the presence of a symptom of a hepatic disease based on the quantity of VCAM1 measured, wherein
the measuring apparatus comprises a sample-setting part configured to set a sample therein, a light source part configured to irradiate the sample with light, and a detection part configured to detect light transmitted through the sample, and
the hepatic disease determination apparatus comprises an acquisition unit configured to acquire measurement data from the measurement apparatus, a comparison unit configured to compare the quantity of VCAM1 with a reference value, and a determination unit configured to determine whether the subject has a symptom of a hepatic disease based on a result of the comparison.

[29] A diagnostic reagent for determining whether a subject is affected with or possibly affected with nonalcoholic steatohepatitis (NASH), a subject has or possibly has a symptom of hepatic fibrosis, the degree of progression of hepatic fibrosis is high or possibly high, the degree of progression of a symptom of hepatocellular ballooning degeneration is high or possibly high, the degree of progression of hepatic inflammation is high or possibly high, a subject has or possibly has a symptom of hepatomegaly, a subject has or possibly has a symptom of hepatocellular necrosis, a subject has or possibly has a symptom of hepatocellular apoptosis, a subject has or possibly has a symptom of hepatocellular degeneration, or a subject has or possibly has a symptom of hepatic steatosis, the diagnostic reagent comprising a reagent for detecting VCAM1.

[30] The diagnostic reagent according to the above [29], wherein the reagent for detecting VCAM1 is an antibody.

[31] Use of a reagent for detecting VCAM1, for in-vitro detection of nonalcoholic steatohepatitis (NASH), hepatic fibrosis, hepatocellular ballooning degeneration, hepatic inflammation, hepatomegaly, hepatocellular necrosis, hepatocellular apoptosis, hepatocellular degeneration, or hepatic steatosis.

[32] The use according to the above [31], wherein an additional diagnostic reagent is concomitantly used.

[33] The use according to the above [32], wherein the additional diagnostic reagent is a diagnostic reagent for detecting HA, COL4, or COL4-7S.

[34] A program or a recording medium storing the program, wherein the program allows a computer to execute:

a comparison step of comparing an index value determined from measurement data on the quantity or quantities of a marker molecule or marker molecules contained in blood collected from a subject with a reference value, the marker molecule or marker molecules including VCAM1; and

a determination step of determining whether the subject has a symptom of a hepatic disease based on a result of the comparison.

[35] The program or recording medium storing the program according to the above [34], wherein the program allows a computer to further execute an acquisition step of acquiring measurement data on the quantity or quantities of the marker molecule or marker molecules contained in blood collected from a subject.

[36] The program or recording medium storing the program according to the above [34] or [35], wherein the program allows a computer to further execute a normalization step of calculating a normalized score or normalized scores from the measurement data and then determining an index value from the normalized score or normalized scores calculated.

[37] The program or recording medium storing the program according to any one of the above [34] to [36], wherein the marker molecule or marker molecules further include HA, COL4, or COL4-7S.

The present invention further relates to the following.

[38] A method for discriminating between nonalcoholic fatty liver (NAFL) and nonalcoholic steatohepatitis (NASH), or a method for collecting data for diagnosis of NAFL and NASH, wherein the method comprising:

(1) a step of measuring the quantities of marker molecules contained in blood collected from a subject, wherein the marker molecules are

(A) all marker molecules belonging to Group 1 consisting of PROS 1, CLU, ANG, APOC3, APOD, CFHR1, cortisol, EGFR, HPN, IGFBP3, IL1B, IL23A, MET, MMP10, tetranectin, TTR, VDBP, and VEGFR-2, all marker molecules belonging to Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40,
all marker molecules belonging to Group 3 consisting of AST, PSAT, LEP, ICAM1, CK-18, GSTA1, ALT, $INS^{IVD}$, $INS^{MAP}$, 6Ckine, AGT, CRP, CXCL10, FABP4, G6PI, HSP-60, P3NP, and testosterone, or
all marker molecules belonging to Group 4 consisting of AREG, BDNF, CD40-L, EREG, FGF2, HBEGF, IGFBP2, MMP9, PAI-1, PDGFB, PLGF, SAP, TGFA, TGFB1, THBS1, and VTN,
or
(B) all marker molecules belonging to two groups selected from the Group 1, the Group 2, the Group 3, and the Group 4;

(2) a step of determining an index value from a normalized score calculated based on the quantities of the marker molecules belonging to the same group, wherein the normalized score is calculated by using the following formula 1:

$$M = \frac{1}{N} \sum_{i=1}^{N} \frac{1}{1 + exp\left(-\alpha_i \times \dfrac{m_i - mean(m_i)}{sd(m_i)}\right)}$$

$$\alpha_i = \begin{cases} 1 & \left(mean\left(m_i^{NASH}\right) > mean\left(m_i^{NAFL}\right)\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL, and,

in the case that the marker molecules correspond to the (A), the index value is the normalized score, and

in the case that the marker molecules correspond to the (B), the index value is calculated by using the following formula 2:

$$I = a_1 \times M_{a1} + a_2 \times M_{a2} \text{ (formula 2)}$$

wherein I denotes an index value; $M_{a1}$ and $M_{a2}$ denote a normalized score for one of the two groups and a normalized score for the other, respectively; and $a_1$ and $a_2$ each denote a constant determined in logistic regression analysis; and

(3) a step of determining that the subject is affected with or possibly affected with NASH in the case that the index value is larger than a reference value, or a step of collecting data to determine whether the subject is affected with NASH based on a possibility that the subject is affected with NASH in the case that the index value is larger than a reference value.

[39] The method according to the above [38], wherein, in the case of the (A), the marker molecules are all marker molecules belonging to the Group 1.

[40] The method according to the above [38], wherein, in the case of the (A), the marker molecules are all marker molecules belonging to the Group 2.

[41] The method according to the above [38], wherein, in the case of the (A), the marker molecules are all marker molecules belonging to the Group 3.

[42] The method according to the above [38], wherein, in the case of the (A), the marker molecules are all marker molecules belonging to the Group 4.

[43] The method according to the above [38], wherein, in the case of the (B), the marker molecules are all marker molecules selected from two groups selected from the Group 1, the Group 2, the Group 3, and the Group 4.

[44] The method according to the above [2], wherein, in the case of the (B), the linear equation is an equation for mean calculation, a linear regression equation, a linear discriminant, or a logistic regression equation.

[45] The method according to the above [44], wherein, in the case of the (B), the equation for mean calculation is for arithmetic averaging.

[46] The method according to the above [44], wherein, in the case of the (B), the linear discriminant is for linear discriminant analysis.

[47] The method according to the above [2], wherein, in the case of the (B), the nonlinear equation is a nonlinear equation for mean calculation, a nonlinear regression equation, a nonlinear discriminant, or a nonlinear logistic regression equation.

[48] The method according to the above [47], wherein, in the case of the (B), the nonlinear equation for mean calculation is for geometric averaging.

[49] The method according to the above [47], wherein, in the case of the (B), the nonlinear regression equation is the product of linear expressions or a neural network.

[50] The method according to the above [47], wherein, in the case of the (B), the nonlinear discriminant is a decision tree or a support vector machine.

[51] A method for determining an effect of a therapeutic drug for nonalcoholic steatohepatitis (NASH), the method comprising:

a step of measuring the quantity or quantities of a marker molecule or marker molecules contained in blood collected from an NASH patient before and after application of the therapeutic drug to the NASH patient, wherein the marker molecule or marker molecules to be measured is/are VCAM1;, VCAM1 and HA; HA and COL4; VCAM1 and COL4; HA and COL4-7S; or VCAM1 and COL4-7S; and

a step of calculating a normalized score based on the quantity or quantities of the marker molecule or marker molecules before and after the application of the therapeutic drug and then determining that an effect of the application of the therapeutic drug is possibly present in the case that the normalized score after the application is lower than the normalized score before the application, wherein each normalized score is calculated by using the following formula 1:

$$M = \frac{1}{N}\sum_{i=1}^{N}\frac{1}{1 + exp\left(-\alpha_i \times \frac{m_i - mean(m_i)}{sd(m_i)}\right)}$$

$$\alpha_i = \begin{cases} 1 & \left(mean(m_i^{NASH}) > mean(m_i^{NAFL})\right) \\ -1 & (otherwise) \end{cases}$$

(formula 1)

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule in patients presenting with NASH; and mean ($m_i^{NAFL}$) denotes a predetermined mean of the quantity of each marker molecule in patients presenting with NAFL.

[52] The method according to [51], wherein the marker molecule or marker molecules to be measured is/are VCAM1; VCAM1 and HA; VCAM1 and COL4; or HA and COL4-7S.

[53] A method for determining an effect of a therapeutic drug for nonalcoholic steatohepatitis (NASH), the method comprising:

a step of measuring the quantity of VCAM1 contained in blood collected from an NASH patient before and after application of the therapeutic drug to the NASH patient; and

a step of determining that an effect of the application of the therapeutic drug is possibly present in the case that the quantity of VCAM1 after the application is lower than the quantity of VCAM1 before the application.

[54] A method for determining an effect of a therapeutic drug for nonalcoholic fatty liver (NAFL) or nonalcoholic steatohepatitis (NASH), the method comprising:

(1) a step of measuring the quantities of marker molecules contained in blood collected from a subject of an NAFL or NASH patient before and after application of the therapeutic drug to the NAFL or NASH patient, wherein the marker molecules to be measured are

(A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2,

at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40,

at least two marker molecules selected from Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone, or

at least two marker molecules selected from Group 4 consisting of AREG, BDNF, CD40-L, EREG, FGF2, HBEGF, IGFBP2, MMP9, PAI-1, PDGFB, PLGF, SAP, TGFA, TGFB1, THBS1, and VTN, or,

(B) at least two marker molecules selected from one of two groups selected from the Group 1, the Group 2, the Group 3, and the Group 4 and at least two marker molecules selected from the other;

(2) a step of determining an index value from a normalized score calculated based on the quantities of the marker molecules before and after application of the therapeutic drug to the NAFL or NASH patient, wherein the normalized score based on the quantities of the marker molecules belonging to the same group is calculated by using the following formula 1:

$$M = \frac{1}{N} \sum_{i=1}^{N} \frac{1}{1 + exp\left(-\alpha_i \times \dfrac{m_i - mean(\mathrm{m}_i)}{sd(\mathrm{m}_i)}\right)}$$

$$\alpha_i = \begin{cases} 1 & \left(mean(\mathrm{m}_i^{NASH}) > mean(\mathrm{m}_i^{NAFL})\right) \\ -1 & (otherwise) \end{cases}$$

$$\text{(formula 1)}$$

wherein M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL, and,

in the case that the marker molecules correspond to the (A), the index value is the normalized score, and

in the case that the marker molecules correspond to the (B), the index value is calculated from normalized scores for the two groups; and

(3) a step of determining that an effect of the application of the therapeutic drug is possibly present in the case that the normalized score after the application is lower than the normalized score before the application.

**Advantageous Effects of Invention**

According to the present invention, a noninvasive method for determining the presence of a symptom of a hepatic disease such as nonalcoholic fatty liver disease with a higher diagnostic accuracy can be provided.

**Brief Description of Drawings**

[Fig. 1] Fig. 1 is graphs showing the normalized scores or index values in the NAFL patients and the NASH patients when each of the marker molecules was used.

[Fig. 2-1] Fig. 2-1 is graphs showing the normalized scores in the NAFL patients and the NASH patients when each of the marker molecules (Measurement method 1) was used.

[Fig. 2-2] Fig. 2-2 is graphs showing the normalized scores or measured values of VCAM1 in the NAFL patients and the NASH patients when each of the marker molecules (Measurement method 2) was used.

[Fig. 3] Fig. 3 is graphs showing the normalized scores in the NAFL patients and the NASH patients when each of the marker molecules was used.

[Fig. 4] Fig. 4 is graphs showing the index values in the NAFL patients and the NASH patients when each of the marker molecules was used.

[Fig. 5-1] Fig. 5-1 is graphs showing the normalized scores in the patients with Type-1 to Type-3 symptoms and in the patients with a Type-4 symptom on Matteoni classification when each of the marker molecules (Measurement method 1) was used.

[Fig. 5-2] Fig. 5-2 is graphs showing the normalized scores or measured values of VCAM1 in the patients with Type-1 to Type-3 symptoms and in the patients with a Type-4 symptom on Matteoni classification when each of the marker molecules (Measurement method 2) was used.

[Fig. 6-1] Fig. 6-1 is graphs showing the normalized scores in the patients with Stage 0, Stage 1 or Stage 2 symptom (F0-2) and in the patients with Stage 3 or Stage 4 symptom (F3-4) on the "Stage", which is the index for the degree of progression of hepatic fibrosis, when each of the marker molecules (Measurement method 1) was used.

[Fig. 6-2] Fig. 6-2 is graphs showing the normalized scores or measured values of VCAM1 in the patients with Stage 0, Stage 1 or Stage 2 symptom (F0-2) and in the patients with Stage 3 or Stage 4 symptom (F3-4) on the "Stage", which is the index for the degree of progression of hepatic fibrosis, when each of the marker molecules (Measurement method 2) was used.

[Fig. 7] Fig. 7 is graphs showing the correlation between the normalized score and the "Stage", which is the index for the degree of progression of hepatic fibrosis, when each of the marker molecules was used.

[Fig. 8] Fig. 8 is graphs showing the correlation between the normalized score and the pathological diagnosis score of lobular inflammation based on a liver biopsy, which is the index for the degree of progression of inflammation, when each of the marker molecules was used.

[Fig. 9] Fig. 9 is graphs showing the correlation between the normalized score and the pathological diagnosis score of ballooning degeneration based on a liver biopsy, which is the index for the degree of progression of hepatocellular ballooning degeneration, when each of the marker molecules was used.

[Fig. 10] Fig. 10 is graphs showing the correlation between the normalized score and the NAFLD Activity Score (NAS), which is the index for activity of NAFLD, when each of the marker molecules was used.

[Fig. 11] Fig. 11 is a schematic view showing an embodiment of the liver disease determination system.

[Fig. 12] Fig. 12 is a diagrammatic view showing the functional structure of the liver disease determination apparatus.

[Fig. 13] Fig. 13 is a flow chart of a method for determining a hepatic disease.

[Fig. 14] Fig. 14 is a graph showing the correlation between the normalized score and the "Stage", which is the index for the degree of progression of hepatic fibrosis, when the normalized score of "HA, COL4" was used.

[Fig. 15] Fig. 15 is a graph showing the correlation between the normalized score and the pathological diagnosis score of lobular inflammation based on a liver biopsy, which is the index for the degree of progression of inflammation, when the normalized score of "HA, COL4" was used.

[Fig. 16] Fig. 16 is a graph showing the correlation between the normalized score and the pathological diagnosis score of ballooning degeneration based on a liver biopsy, which is the index for the degree of progression of hepatocellular ballooning degeneration, when the normalized score of "HA, COL4" was used.

[Fig. 17] Fig. 17 is a graph showing the correlation between the normalized score and the NAFLD Activity Score (NAS), which is the index for activity of NAFLD, when the normalized score of "HA, COL4" was used.

Description of Embodiments

Hereinafter, suitable embodiments of the present invention will be described in detail. However, the present invention is never limited to the following embodiments.

The method for discriminating between nonalcoholic fatty liver (NAFL) and nonalcoholic steatohepatitis (NASH) according to the present embodiment (hereinafter, occasionally referred to as "discrimination method") includes:

(1) a step of measuring the quantities of marker molecules contained in blood collected from a subject;
(2) a step of determining an index value from a normalized score calculated based on the quantities of the marker molecules belonging to the same group; and
(3) a step of determining that the subject is presenting with or possibly presenting with NASH in the case that the index value is larger than a reference value.

As with nonalcoholic fatty liver (NAFL), nonalcoholic steatohepatitis (NASH) is a disease classified as nonalcoholic fatty liver disease (NAFLD). NAFLD is defined as a fatty liver disease unrelated to excessive alcohol intake among hepatic diseases excluding viral hepatic diseases and autoimmune hepatic diseases. NASH is defined as "a case that hepatocellular ballooning degeneration with inflammation is found in addition to macrovesicular steatosis of hepatocytes" in pathological diagnosis. NASH corresponds to a case classified as Type-3 or Type-4 in the Matteoni classification. It is known that NASH can cause hepatic cirrhosis or liver cancer when the symptoms have progressed. NASH is characterized by hepatic fibrosis, hepatocellular ballooning degeneration, hepatic inflammation, hepatomegaly, hepatocellular necrosis, hepatocellular apoptosis, hepatocellular degeneration, hepatic steatosis, and so on. On the other hand, NAFL is a disease which is one of NAFLD excluding NASH and based on macrovesicular steatosis of hepatocytes. NAFL corresponds to a case classified as Type-1 or Type-2 in the Matteoni classification. NAFL is known to give a better prognosis than NASH. Now, the steps will be described.

(1) Step of measuring quantities of marker molecules contained in blood collected from subject

Examples of the blood collected from a subject include whole blood, serum, and plasma, and serum or plasma is preferably used.

Next, the marker molecules will be described. The present inventors diligently examined the relation between the quantities of approximately 260 biomolecules and difference in symptoms of NAFL and NASH for serum samples obtained from NAFL patients and NASH patients, and found four clusters (occasionally referred to as "groups", "factor modules", or "modules") of a plurality of biomolecules which are biologically related to each other at a high level and the abundances of which are interrelated. Now, the groups will be described.

The marker molecules belonging to Group 1 (occasionally referred to as "Module 1") are marker molecules relating to a defensive response to fatty liver, and the Group 1 consists of 18 marker molecules: PROS1 (vitamin K-dependent protein S), CLU (clusterin), ANG (angiogenin), APOC3 (apolipoprotein C-III), APOD (apolipoprotein D), CFHR1 (complement factor H-related protein 1), cortisol, EGFR (epidermal growth factor receptor), HPN (hepsin), IGFBP3 (insulin-like growth factor binding protein type 3), IL1B (interleukin 1$\beta$), IL23A (interleukin 23), MET (hepatocyte growth factor receptor), MMP10 (matrix metalloproteinase 10), tetranectin, TTR (transthyretin), VDBP (vitamin D-binding protein), and VEGFR-2 (vascular endothelial cell growth factor receptor 2).

The marker molecules belonging to Group 2 (occasionally referred to as "Module 2") are marker molecules relating to a defensive response of the immune system to hepatocellular damage, and the Group 2 consists of 18 marker molecules: VCAM1 (vascular cell adhesion molecule 1), HA (hyaluronic acid), CTSD (cathepsin D), COL4 (collagen 4), COL4-7S (type 4 collagen 7S), ALB (albumin), AFP ($\alpha$ fetoprotein), AXL (AXL receptor tyrosine kinase), CCL19 (chemokine (C-C motif) ligand 19), CGB (human chorionic gonadotropin $\beta$), CSF1 (macrophage colony-stimulating factor 1), FAS (FASLG receptor), Mac-2bp (Mac-2 binding protein), CA19-9 (carbohydrate antigen 19-9), NRCAM (neural cell adhesion molecule), OPG (osteoprotegerin), VWF (von Willebrand factor), and YKL-40.

The marker molecules belonging to Group 3 (occasionally referred to as "Module 3") are marker molecules relating to the innate immune response to hepatitis, and the Group 3 consists of 18 marker molecules: AST (aspartate aminotransferase), PSAT (phosphoserine aminotransferase), LEP (leptin), ICAM1 (intercellular adhesion molecule 1), CK-18 (CK18 M30 fragment), GSTA1 (glutathione S-transferase alpha), ALT (alanine aminotransferase), INS$^{IVD}$ (insulin, measured by using CLIA), INS$^{MAP}$ (insulin, measured by using a Human MAP method (the multiple immunoassay service Human MAP provided by Myriad RBM, Inc., the same is applied hereinafter)), 6Ckine, AGT (angiotensinogen), CRP (C-reactive protein), CXCL10 (C-X-C motif chemokine 10, interferon gamma induced protein 10), FABP4 (fatty acid-binding protein 4, adipocyte), G6PI (glucose-6-phosphate isomerase), HSP-60 (heat shock protein 60), P3NP (procollagen 3 N-terminal peptide), and testosterone.

The marker molecules belonging to Group 4 (occasionally referred to as "Module 4") are marker molecules relating to cell growth in association with hepatocellular damage and hepatic fibrosis, and the Group 4 consists of 16 marker molecules: AREG (amphiregulin), BDNF (brain-derived neurotrophic factor), CD40-L (CD40 ligand), EREG (epiregulin), FGF2 (basic fibroblast growth factor), HBEGF (heparin-binding EGF-like growth factor), IGFBP2 (insulin-like growth factor-binding protein 2), MMP9 (matrix metalloproteinase 9), PAI-1 (plasminogen activator inhibitor 1), PDGFB (platelet-derived growth factor BB), PLGF (placental growth factor), SAP (serum amyloid P component), TGFA (transforming growth factor $\alpha$), TGFB1 (latency-associated peptide of transforming growth factor beta 1), THBS1 (thrombospondin 1), and VTN (vitronectin).

Examples of the marker molecules to be measured include:

(A1) at least two marker molecules selected from the Group 1, at least two marker molecules selected from the Group 2, at least two marker molecules selected from the Group 3, or at least two marker molecules selected from the Group 4, or

(B1) at least two marker molecules selected from one of two groups selected from the Group 1, the Group 2, the Group 3, and the Group 4, and at least two marker molecules selected from the other.

In the case of the (B1), examples of the marker molecules to be measured include at least two marker molecules selected from the Group 1 and at least two marker molecules selected from the Group 2; at least two marker molecules selected from the Group 1 and at least two marker molecules selected from the Group 3; at least two marker molecules selected from the Group 1 and at least two marker molecules selected from the Group 4; at least two marker molecules selected from the Group 2 and at least two marker molecules selected from the Group 3; at least two marker molecules selected from the Group 2 and at least two marker molecules selected from the Group 4; and at least two marker molecules selected from the Group 3 and at least two marker molecules selected from the Group 4.

In another aspect of the present embodiment, examples of the marker molecules to be measured include:

(A2) all marker molecules belonging to the Group 1, all marker molecules belonging to the Group 2, all marker molecules belonging to the Group 3, or all marker molecules belonging to the Group 4, or
(B2) all marker molecules belonging to two groups selected from the Group 1, the Group 2, the Group 3, and the Group 4.

In the case of the (B2), examples of the marker molecules to be measured include all marker molecules belonging to the Group 1 and the Group 2; all marker molecules belonging to the Group 1 and the Group 3; all marker molecules belonging to the Group 1 and the Group 4; all marker molecules belonging to the Group 2 and the Group 3; all marker molecules belonging to the Group 2 and the Group 4; and all marker molecules belonging to the Group 3 and the Group 4, and the marker molecules to be measured are preferably all marker molecules belonging to the Group 1 and the Group 3.

To measure the quantities of the above-described marker molecules, any known method capable of measuring the quantity in blood can be used without any limitation. Examples of the method for measuring the quantities of the marker molecules include ELISA, chemiluminescence immunoassay (CLIA), a latex agglutination method, radioimmunoassay, turbidimetric immunoassay, an enzyme activity measurement method, a dye-binding method, a Luminex method, Western blotting, a Human MAP method, and mass spectrometry, and preferred examples include ELISA, CLIA, a latex agglutination method, radioimmunoassay, turbidimetric immunoassay, an enzyme activity measurement method, a dye-binding method, a Luminex method, and a Human MAP method.

(2) Step of determining index value from normalized score calculated on basis of quantities of marker molecules belonging to same group

The substep of calculating a normalized score based on the quantities of the marker molecules belonging to the same group includes a sub-substep of normalizing data derived from the quantities of the marker molecules measured, a sub-substep of converting the distribution of the normalized data, and a sub-substep of selecting a representative value determined from the converted distribution as a normalized score.

In the sub-substep of normalizing data derived from the quantities of the marker molecules measured, any known method can be used without any limitation. Examples of the method for normalizing the data include a method of performing proportional conversion so as to set the root-mean-square to 1, a method of performing linear conversion so as to set the mean comparable to the variance, and a method of setting the maximum value and minimum value to 1 and 0, respectively, and preferred examples include a method of performing linear conversion so as to set the mean comparable to the variance.

In the sub-substep of converting the distribution of the normalized data, any known method can be used without any limitation. Examples of the method for converting the distribution of the normalized data include conversion with a logarithmic function and conversion with a sigmoid function, and preferred examples include conversion with a standard sigmoid function. This sub-substep can be omitted.

In the sub-substep of selecting a representative value determined from the converted distribution as a normalized score, any known method can be used without any limitation. Examples of the representative value include common statistics, i.e., a mean, median, mode, maximum, minimum, and any percentile, and preferred examples include a mean and median.

Preferred examples of the method for calculating a normalized score with combination of these sub-substeps include a method including a sub-substep of normalizing the data by using a method of performing linear conversion so as to set the mean comparable to the variance, a sub-substep of converting the distribution of the data by using a standard sigmoid function, and a sub-substep of selecting the mean determined from the converted distribution as a representative value. In this method, a normalized score is calculated based on the quantities of the marker molecules belonging to the same group by using the following formula 1.

$$M = \frac{1}{N} \sum_{i=1}^{N} \frac{1}{1 + exp\left(-\alpha_i \times \frac{m_i - mean(\mathrm{m}_i)}{sd(\mathrm{m}_i)}\right)}$$

$$\alpha_i = \begin{cases} 1 & \left(mean\left(\mathrm{m}_i^{NASH}\right) > mean\left(\mathrm{m}_i^{NAFL}\right)\right) \\ -1 & (otherwise) \end{cases}$$

(formula 1)

In the formula 1, M denotes a normalized score; N denotes the number of marker molecules; $m_i$ denotes the quantity of each marker molecule measured; mean ($m_i$) denotes a predetermined mean of the quantity of each marker molecule; sd ($m_i$) denotes a predetermined standard deviation for each marker molecule; mean ($m_i^{NASH}$) denotes a predetermined mean of the quantity of each marker molecule for patients affected with NASH; and mean ($m_i^{NAFL}$) denotes a predetermined mean of the quantity of each marker molecule for patients affected with NAFL.

Here, mean ($m_i$) is a mean of the quantity of each marker molecule, for which, for example, a mean obtained by measurement for specimens from individuals affected with NAFLD, a mean for individuals affected with NAFLD based on known measurement data, a mean for local residents including an individual to be determined or evaluated based on known measurement data, or a mean for a race to be determined or evaluated based on known measurement data can be used. When a mean for individuals affected with NAFLD is used, for example, a simple average for the whole of an ensemble of the affected individuals, a simple average of a mean for individuals affected with NAFL and a mean for NASH patients in an ensemble of the affected individuals, or a weighted average obtained by applying a weight to each of a mean for individuals affected with NAFL and a mean for NASH patients followed by averaging the resultants can be used. In the case that a mean for individuals affected with NAFLD based on known measurement data is used, for example, a simple average for individuals affected with NAFLD based on known measurement data, a simple average of a mean for individuals affected with NAFL and a mean for NASH patients based on known measurement data, or a weighted average obtained by applying a weight to each of a mean for individuals affected with NAFL and a mean for patients affected with NASH based on known measurement data followed by averaging the resultants can be used. For the known measurement data, for example, measurements of biomolecules published in an article, values listed in a package insert of an in vitro diagnostic reagent, or values listed in clinical practice guidelines can be used. Data from a plurality of origins can be used in combination. Preferably, a simple average for the whole of an ensemble of individuals affected with NAFLD or a simple average of a mean for individuals affected with NAFL and a mean for NASH patients in an ensemble of individuals affected with NAFLD, each obtained by measurement for specimens from individuals affected with NAFLD, is used, for example. More preferably, a simple average of a mean for individuals affected with NAFL and a mean for NASH patients in an ensemble of individuals affected with NAFLD, each mean obtained by measurement for specimens from individuals affected with NAFLD, is used, for example.

Here, sd ($m_i$) is a standard deviation of the quantity of each marker molecule, for which, for example, a standard deviation obtained by measurement for specimens from individuals affected with NAFLD, a standard deviation for individuals affected with NAFLD based on known measurement data, a standard deviation for local residents including an individual to be determined or evaluated based on known measurement data, or a standard deviation for a race to be determined or evaluated based on known measurement data can be used. When a standard deviation for individuals affected with NAFLD is used, for example, a standard deviation for the whole of an ensemble of the affected individuals, a simple average of a standard deviation for individuals affected with NAFL and a standard deviation for NASH patients in an ensemble of the affected individuals, or a weighted average obtained by applying a weight to each of a standard deviation for individuals affected with NAFL and a standard deviation for NASH patients followed by averaging the resultants can be used. In the case that a standard deviation for individuals affected with NAFLD based on known measurement data is used, for example, a standard deviation for individuals affected with NAFLD based on known measurement data, a simple average of a standard deviation for individuals affected with NAFL and a standard deviation for NASH patients based on known measurement data, or a weighted average obtained by applying a weight to each of a standard deviation for individuals affected with NAFL and a standard deviation for patients affected with NASH based on known measurement data followed by averaging the resultants can be used. For the known measurement data, for example, measurements of biomolecules published in an article, values listed in a package insert of an in vitro diagnostic reagent, or values listed in clinical practice guidelines can be used. Data from a plurality of origins can be used in combination. Preferably, a standard deviation for the whole of an ensemble of individuals affected with NAFLD or a simple average of a standard deviation for individuals affected with NAFL and a standard deviation for NASH patients in an ensemble of individuals affected with NAFLD, each obtained by measurement for specimens from individuals affected with NAFLD, is used, for example. More preferably, a simple average of a standard deviation for individuals affected with NAFL and a standard deviation for NASH patients in an ensemble of individuals affected with NAFLD, each standard deviation obtained by measurement for specimens from individuals affected with NAFLD, is used, for example.

When values of mean $(m_i)$, sd $(m_i)$, mean $(m_i^{NASH})$, and mean $(m_i^{NAFL})$ for a given marker molecule are derived from different methods for measuring the quantity of a marker molecule, different values obtained by any of the different measurement methods may be used for setting mean $(m_i)$, sd $(m_i)$, mean $(m_i^{NASH})$, and mean $(m_i^{NAFL})$. When any relation is found between measurements derived from different measurement methods, values of mean $(m_i)$, sd $(m_i)$, mean $(m_i^{NASH})$, and mean $(m_i^{NAFL})$ set by using one measurement method may be substituted in a given relational equation to convert into values obtained by another measurement method.

Values of mean $(m_i)$, sd $(m_i)$, mean $(m_i^{NASH})$, and mean $(m_i^{NAFL})$ may be appropriately set according to a race or locality targeted for measurement. Specific examples of values of mean $(m_i)$, sd $(m_i)$, mean $(m_i^{NASH})$, and mean $(m_i^{NAFL})$ include values listed later in Tables 5 to 8 and 41.

In the case that one normalized score is calculated (in the case that the marker molecules correspond to the (A)), the normalized score is directly used as an index value.

In the case that two or more normalized scores are calculated (in the case that the marker molecules correspond to the (B)), an index value can be determined by using a plurality of normalized scores. In the step of determining an index value from a plurality of normalized scores, any known method can be used without any limitation. Examples of the method for determining an index value from a plurality of normalized scores include a method with a linear equation and a method with a nonlinear equation. Examples of the method with a linear equation include a method with an equation for mean calculation, a method with a linear regression equation, a method with a linear discriminant, and a method with a logistic regression equation. Examples of the method with an equation for mean calculation include a method with arithmetic averaging. Examples of the method with a linear discriminant include a method with linear discriminant analysis. Examples of the method with a nonlinear equation include a method with a nonlinear equation for mean calculation, a method with a nonlinear regression equation, a method with a nonlinear discriminant, and a method with a nonlinear logistic regression equation. Examples of the method with a nonlinear equation for mean calculation include a method with geometric averaging. Examples of the method with a nonlinear regression equation include a method with multiplication of linear expressions and a method with a neural network. Examples of the method with a nonlinear discriminant include a method with a decision tree and a method with a support vector machine. Examples of preferred methods include a method with a decision tree and a method with a logistic regression equation.

Examples of more preferred methods include a method with a logistic regression equation, and a value calculated by using the following formula 2 is used as a preferred index value. In the formula 2, I denotes an index value; $M_{a1}$ and $M_{a2}$ denote a normalized score for one of the two groups ("first normalized score") and a normalized score for the other ("second normalized score"), respectively; and $a_1$ and $a_2$ each denote a constant determined so as to minimize the error in discrimination between NAFL and NASH.

$$I = a_1 \times M_{a1} + a_2 \times M_{a2} \text{ (formula 2)}$$

Values of $a_1$ and $a_2$ may be determined by using any method as long as $a_1$ and $a_2$ are determined so as to minimize the error in discrimination between NAFL and NASH, and, for example, can be determined by using linear discriminant analysis or logistic regression analysis. Specific examples of $a_1$ and $a_2$ include values listed later in Table 9.

In the case that an index value is determined from a plurality of normalized scores by using a decision tree, for example, the following Decision trees 1 to 6 are used. When an index value is determined by using the Decision trees, a value listed later in Table 11 in Examples is used as a reference value.

Decision tree 1: in the case that the marker molecules are selected from the Group 1 and Group 2, if $M_{1a} \geq 0.476$, then I = 2, if $M1_a < 0.476$ and $M2_a \geq 0.466$, then I = 1, and if $M1_a < 0.476$ and $M2_a < 0.466$, then I = 0;

Decision tree 2: in the case that the marker molecules are selected from the Group 1 and Group 3, if $M3_a \geq 0.409$ and $M1_a \geq 0.45$, then I = 2, if $M3_a \geq 0.409$ and $M1_a < 0.45$, then I = 1, and if $M3_a < 0.409$, then I = 0;

Decision tree 3: in the case that the marker molecules are selected from the Group 1 and Group 4, if $M4_a \geq 0.547$, then I = 2, if $M4_a < 0.547$ and $M1_a \geq 0.468$, then I = 1, and if $M4_a < 0.547$ and $M1_a < 0.468$, then I = 0;

Decision tree 4: in the case that the marker molecules are selected from the Group 2 and Group 3, if $M3_a \geq 0.409$ and $M2_a \geq 0.412$, then I = 2, if $M3_a \geq 0.409$ and $M2_a < 0.412$, then I = 1, and if $M3_a < 0.409$, then I = 0;

Decision tree 5: in the case that the marker molecules are selected from the Group 2 and Group 4, if $M4_a \geq 0.547$,

then I = 2, if $M4_a < 0.547$ and $M2_a \geq 0.413$, then I = 1, and if $M4_a < 0.547$ and $M2_a < 0.413$, then I = 0; and

Decision tree 6: in the case that the marker molecules are selected from the Group 3 and Group 4, if $M3_a \geq 0.409$, then I = 2, if $M3_a < 0.409$ and $M4_a > 0.588$, then I = 1, and if $M3_a < 0.409$ and $M4_a < 0.588$, then I = 0,

wherein I denotes an index value; and $M1_a$, $M2_a$, $M3_a$, and $M4_a$ denote normalized scores for the Group 1, Group 2, Group 3, and Group 4, respectively.

(3) Step of determining that subject is possibly affected with NASH in case that index value is larger than reference value

The "reference value" in the present specification refers to a value as a reference to discriminate between two conditions ("cutoff value"), and can be arbitrarily set. The reference value may be an index value for individuals affected with NAFL, or a preset value. In the case of a preset value, an index value such that sensitivity and specificity required by a user are achieved can be used for setting the reference value, based on a relation between a reference value and sensitivity (accuracy for individuals affected with NASH) and specificity (accuracy for individuals affected with NAFL) each estimated from an ROC curve derived from index values possessed by individuals affected with NAFL and individuals affected with NASH.

Examples of the reference value for determining whether a subject is possibly affected with NASH include the following values. The following exemplary reference values are those in the case that a normalized score is directly used as an index value, and those in the case that an index value determined from a plurality of normalized scores by using the above (formula 2) is used.

In the case that the marker molecules are at least two marker molecules belonging to the Group 1, the reference value is, for example, a value set to 0.149 to 0.907.

In the case that the marker molecules are at least two marker molecules belonging to the Group 2, the reference value is, for example, a value set to 0.087 to 0.848.

In the case that the marker molecules are at least two marker molecules belonging to the Group 3, the reference value is, for example, a value set to 0.164 to 0.909.

In the case that the marker molecules are at least two marker molecules belonging to the Group 4, the reference value is, for example, a value set to 0.073 to 0.854.

In the case that the marker molecules are all marker molecules belonging to the Group 1, the reference value is, for example, a value set to 0.359 to 0.586, and is preferably a value set to 0.359 to 0.486 from the viewpoint of improvement of the accuracy for individuals affected with NASH, and is preferably a value set to 0.486 to 0.586 from the viewpoint of improvement of the accuracy for individuals affected with NAFL.

In the case that the marker molecules are all marker molecules belonging to the Group 2, the reference value is, for example, a value set to 0.357 to 0.509, and is preferably a value set to 0.357 to 0.419 from the viewpoint of improvement of the accuracy for individuals affected with NASH, and is preferably a value set to 0.419 to 0.509 from the viewpoint of improvement of the accuracy for individuals affected with NAFL.

In the case that the marker molecules are all marker molecules belonging to the Group 3, the reference value is, for example, a value set to 0.378 to 0.545, and is preferably a value set to 0.378 to 0.45 from the viewpoint of improvement of the accuracy for individuals affected with NASH, and is preferably a value set to 0.45 to 0.545 from the viewpoint of improvement of the accuracy for individuals affected with NAFL.

In the case that the marker molecules are all marker molecules belonging to the Group 4, the reference value is, for example, a value set to 0.272 to 0.583, and is preferably a value set to 0.272 to 0.489 from the viewpoint of improvement of the accuracy for individuals affected with NASH, and is preferably a value set to 0.489 to 0.583 from the viewpoint of improvement of the accuracy for individuals affected with NAFL.

In the case that the marker molecules are at least two marker molecules belonging to the Group 1 and at least two marker molecules belonging to the Group 2, the reference value is, for example, a value set to 3.438 to 25.735.

In the case that the marker molecules are at least two marker molecules belonging to the Group 1 and at least two marker molecules belonging to the Group 3, the reference value is, for example, a value set to 5.987 to 34.702.

In the case that the marker molecules are at least two marker molecules belonging to the Group 1 and at least two marker molecules belonging to the Group 4, the reference value is, for example, a value set to 1.932 to 14.239.

In the case that the marker molecules are at least two marker molecules belonging to the Group 2 and at least two marker molecules belonging to the Group 3, the reference value is, for example, a value set to 2.836 to 19.664.

In the case that the marker molecules are at least two marker molecules belonging to the Group 2 and at least two marker molecules belonging to the Group 4, the reference value is, for example, a value set to 1.387 to 14.334.

In the case that the marker molecules are at least two marker molecules belonging to the Group 3 and at least two marker molecules belonging to the Group 4, the reference value is, for example, a value set to 3.044 to 20.554.

In the case that the marker molecules are all marker molecules belonging to the Group 1 and the Group 2, the reference value is, for example, a value set to 12.025 to 15.396, and is preferably a value set to 12.025 to 13.795 from the viewpoint of improvement of the accuracy for individuals affected with NASH, and is preferably a value set to 13.795 to 15.396 from the viewpoint of improvement of the accuracy for individuals affected with NAFL.

In the case that the marker molecules are all marker molecules belonging to the Group 1 and the Group 3, the reference value is, for example, a value set to 15.915 to 20.712, and is preferably a value set to 15.915 to 18.297 from the viewpoint of improvement of the accuracy for individuals affected with NASH, and is preferably a value set to 18.297 to 20.712 from the viewpoint of improvement of the accuracy for individuals affected with NAFL.

In the case that the marker molecules are all marker molecules belonging to the Group 1 and the Group 4, the reference value is, for example, a value set to 6.219 to 8.967, and is preferably a value set to 6.219 to 7.533 from the viewpoint of improvement of the accuracy for individuals affected with NASH, and is preferably a value set to 7.533 to 8.967 from the viewpoint of improvement of the accuracy for individuals affected with NAFL.

In the case that the marker molecules are all marker molecules belonging to the Group 2 and the Group 3, the reference value is, for example, a value set to 8.497 to 11.466, and is preferably a value set to 8.497 to 10.129, and is preferably a value set to 10.129 to 11.466 from the viewpoint of improvement of the accuracy for individuals affected with NAFL.

In the case that the marker molecules are all marker molecules belonging to the Group 2 and the Group 4, the reference value is, for example, a value set to 5.889 to 8.934, and is preferably a value set to 5.889 to 7.445 from the viewpoint of improvement of the accuracy for individuals affected with NASH, and is preferably a value set to 7.445 to 8.934 from the viewpoint of improvement of the accuracy for individuals affected with NAFL.

In the case that the marker molecules are all marker molecules belonging to the Group 3 and the Group 4, the reference value is, for example, a value set to 9.023 to 12.548, and is preferably a value set to 9.023 to 10.665 from the viewpoint of improvement of the accuracy for individuals affected with NASH, and is preferably a value set to 10.665 to 12.548 from the viewpoint of improvement of the accuracy for individuals affected with NAFL.

As described above, the discrimination method according to the present embodiment has, in one aspect, a significant advantageous feature that groups (modules) each composed of a plurality of biomolecules (16 to 18 biomolecules) which are biologically related to each other at a high level and the abundances of which in blood are interrelated are used, and not the quantity of an individual marker molecule but a normalized score based on measurement of all marker molecules in a given group (module) is used. Some methods for discriminating between NAFL and NASH based on measurement of a plurality of marker molecules have been previously reported. However, the methods are all those based on measurement of a few marker molecules. Such a method based on measurement of a few marker molecules tends to be susceptible to variation or bias among measurements of the quantity of each maker molecule. The discrimination method according to the present embodiment is less susceptible to error and variation in measurement of individual marker molecules in that the discrimination method is based on measurement of more marker molecules and a representative value (normalized score) for a module composed of marker molecules which are biologically related to each other at a high level and the abundances of which in blood are interrelated is used, and thus enables discrimination with a higher diagnostic accuracy.

In conventional methods for discriminating between NAFL and NASH with a plurality of marker molecules, a method in which a weight is applied to each of measurements of a plurality of marker molecules and then the resultants are summed up, or a method in which measurements of a plurality of marker molecules are multiplied or divided is used. However, these methods have a relatively large tendency to cause excessive adaptation to a group of specimens used for construction of the discrimination method, i.e., learning. Since the discrimination method according to the present embodiment uses a statistic such as a mean and a median of normalized measurements of marker molecules as an index value, and, for this reason, learning is not performed in this step, the discrimination method according to the present embodiment has an advantage that excessive adaptation to a group of specimens used for learning is avoided. In view of these two points, the discrimination method according to the present embodiment is a method for discriminating between NAFL and NASH with an excellent advantageous feature compared to conventional arts.

In another aspect of the present embodiment, the marker molecule or marker molecules to be measured may be one or more marker molecules belonging to a given group (module). Selection of marker molecules representative of each module enables simple discrimination with a high diagnostic accuracy maintained. Marker molecules representative of each module can be appropriately selected without any limitation. Regarding the number of the marker molecules to be selected, for example, two or three marker molecules may be selected, or four to all marker molecules may be selected.

In the case of the Group 1, for example, the marker molecules may include PROS1 and CLU or may be PROS 1 and CLU.

In the case of the Group 2, the marker molecules may include VCAM1 or may be VCAM1, or may be VCAM1 and HA or may include VCAM1 and HA, or may be VCAM1 and CTSD or may include VCAM1 and CTSD, or may be VCAM1 and COL4 or may include VCAM1 and COL4, or may be VCAM1 and COL4-7S or may include VCAM1 and COL4-7S, or may be HA and COL4-7S or may include HA and COL4-7S, or may be HA and CTSD or may include HA and CTSD, or may be HA and COL4 or may include HA and COL4, or may be CTSD and COL4 or may include CTSD and COL4, or may be CTSD and COL4-7S or may include CTSD and COL4-7S.

In the case of the Group 3, the marker molecules may be AST and PSAT or may include AST and PSAT, or may be AST and LEP or may include AST and LEP, or may be AST and ICAM1 or may include AST and ICAM1, or may be AST, PSAT, and LEP or may include AST, PSAT, and LEP, or may be AST, PSAT, and ICAM1 or may include AST, PSAT, and ICAM1, or may be AST, LEP, and CK-18 or may include AST, LEP, and CK-18, or may be AST, ICAM1, and GSTA1 or may include AST, ICAM1, and GSTA1, or may be AST, PSAT, and 6Ckine or may include AST, PSAT, and 6Ckine.

The normalized score is calculated based on the quantity or quantities of the marker molecule or marker molecules by using the above formula 1. Examples of reference values for determining whether a subject is possibly affected with NASH include the values below.

In the case that the Human MAP method is used for measurement of PROS1 and CLU, the reference value can be set as follows.

In the case that the marker molecules are PROS1 and CLU, the reference value is, for example, a value set to 0.256 to 0.634, and is preferably a value set to 0.256 to 0.42 from the viewpoint of improvement of the accuracy for individuals affected with NASH, and is preferably a value set to 0.42 to 0.634 from the viewpoint of improvement of the accuracy for individuals affected with NAFL.

Any method may be used for measurement of the quantity of each marker molecule, and, for example, commercially available methods can be used. In the case that ELISA with a "Hyaluronan Quantikine ELISA Kit" is used for measurement of HA, the Human MAP method is used for measurement of VCAM1, CTSD, and COL4, and a radioimmunoassay with a "Type IV Collagen 7S Kit" is used for measurement of COL4-7S, the reference value can be set as follows.

In the case that the marker molecule or marker molecules is/are VCAM1; VCAM1 and HA; VCAM1 and CTSD; VCAM1 and COL4; VCAM1 and COL4-7S; HA and COL4-7S; HA and CTSD; HA and COL4; CTSD and COL4; or CTSD and COL4-7S, the reference value is, for example, a value set to 0.256 to 0.644, and is preferably a value set to 0.256 to 0.42 from the viewpoint of improvement of the accuracy for individuals affected with NASH, and is preferably 0.389 to 0.644 from the viewpoint of improvement of the accuracy for individuals affected with NAFL.

In the case that the marker molecule is VCAM1, the reference value is, for example, a value set to 0.28 to 0.644, and is preferably a value set to 0.28 to 0.389 from the viewpoint of improvement of the accuracy for individuals affected with NASH, and is preferably a value set to 0.389 to 0.644 from the viewpoint of improvement of the accuracy for individuals affected with NAFL.

In the case that the marker molecules are VCAM1 and HA, the reference value is, for example, a value set to 0.326 to 0.617, and is preferably a value set to 0.326 to 0.398 from the viewpoint of improvement of the accuracy for individuals affected with NASH, and is preferably a value set to 0.398 to 0.617 from the viewpoint of improvement of the accuracy for individuals affected with NAFL.

In the case that the marker molecules are VCAM1 and CTSD, the reference value is, for example, a value set to 0.277 to 0.604, and is preferably a value set to 0.277 to 0.408 from the viewpoint of improvement of the accuracy for individuals affected with NASH, and is preferably a value set to 0.408 to 0.604 from the viewpoint of improvement of the accuracy for individuals affected with NAFL.

In the case that the marker molecules are VCAM1 and COL4, the reference value is, for example, a value set to 0.319 to 0.617, and is preferably a value set to 0.319 to 0.407 from the viewpoint of improvement of the accuracy for individuals affected with NASH, and is preferably a value set to 0.407 to 0.617 from the viewpoint of improvement of the accuracy for individuals affected with NAFL.

In the case that the marker molecules are VCAM1 and COL4-7S, the reference value is, for example, a value set to 0.303 to 0.543, and is preferably a value set to 0.303 to 0.416 from the viewpoint of improvement of the accuracy for individuals affected with NASH, and is preferably a value set to 0.416 to 0.543 from the viewpoint of improvement of the accuracy for individuals affected with NAFL.

In the case that the marker molecules are HA and COL4-7S, the reference value is, for example, a value set to 0.326 to 0.509, and is preferably a value set to 0.326 to 0.398 from the viewpoint of improvement of the accuracy for individuals affected with NASH, and is preferably a value set to 0.398 to 0.509 from the viewpoint of improvement of the accuracy for individuals affected with NAFL.

In the case that the marker molecules are HA and COL4, the reference value is, for example, a value set to 0.343 to 0.487, and is preferably a value set to 0.343 to 0.395 from the viewpoint of improvement of the accuracy for individuals affected with NASH, and is preferably a value set to 0.395 to 0.487 from the viewpoint of improvement of the accuracy for individuals affected with NAFL.

In the case that the marker molecules are HA and CTSD, the reference value is, for example, a value set to 0.305 to 0.545, and is preferably a value set to 0.305 to 0.411 from the viewpoint of improvement of the accuracy for individuals affected with NASH, and is preferably a value set to 0.411 to 0.545 from the viewpoint of improvement of the accuracy for individuals affected with NAFL.

In the case that the marker molecules are CTSD and COL4, the reference value is, for example, a value set to 0.307 to 0.536, and is preferably a value set to 0.307 to 0.415 from the viewpoint of improvement of the accuracy for individuals affected with NASH, and is preferably a value set to 0.415 to 0.536 from the viewpoint of improvement of the accuracy for individuals affected with NAFL.

In the case that the marker molecules are CTSD and COL4-7S, the reference value is, for example, a value set to 0.271 to 0.575, and is preferably a value set to 0.271 to 0.419 from the viewpoint of improvement of the accuracy for individuals affected with NASH, and is preferably a value set to 0.419 to 0.575 from the viewpoint of improvement of the accuracy for individuals affected with NAFL.

In the case that the in vitro diagnostic reagent "Chemilumi hyaluronic acid" in accordance with a latex agglutination method or CLIA is used for measurement of HA, ELISA with a "Human sVCAM-1/CD106 Quantikine ELISA Kit" is used for measurement of VCAM1, and a radioimmunoassay with a "Type IV Collagen 7S Kit" is used for measurement of COL4-7S, the reference value can be set as follows.

In the case that the marker molecules are VCAM1 and HA, the reference value is, for example, a value set to 0.326 to 0.684, and is preferably a value set to 0.326 to 0.39 from the viewpoint of improvement of the accuracy for individuals affected with NASH, and is preferably a value set to 0.39 to 0.684 from the viewpoint of improvement of the accuracy for individuals affected with NAFL.

In the case that the marker molecules are VCAM1 and COL4-7S, the reference value is, for example, a value set to 0.293 to 0.548, and is preferably a value set to 0.293 to 0.394 from the viewpoint of improvement of the accuracy for individuals affected with NASH, and is preferably a value set to 0.394 to 0.548 from the viewpoint of improvement of the accuracy for individuals affected with NAFL.

In the case that the marker molecules are HA and COL4-7S, the reference value is, for example, a value set to 0.308 to 0.662, and is preferably a value set to 0.308 to 0.394 from the viewpoint of improvement of the accuracy for individuals affected with NASH, and is preferably a value set to 0.394 to 0.662 from the viewpoint of improvement of the accuracy for individuals affected with NAFL.

In the case that the marker molecule is VCAM1, the measurement of VCAM1 (the quantity of VCAM1) may be directly used as an index value in place of a normalized score calculated by using the above formula 1. In the case that ELISA with a "Human sVCAM-1/CD106 Quantikine ELISA Kit" is used for measurement of the quantity of VCAM1, the reference value (serum level (ng/mL)) is, for example, a value set to 551.1 to 1280.1, and is preferably a value set to 551.1 to 806.2 from the viewpoint of improvement of the accuracy for individuals affected with NASH, and is preferably a value set to 806.2 to 1280.1 from the viewpoint of improvement of the accuracy for individuals affected with NAFL.

In the case that an enzyme activity measurement method is used for measurement of AST, the Human MAP method is used for measurement of PSAT, LEP, ICAM1, GSTA1, and 6Ckine, and ELISA with an "M30 Apoptosense ELISA" is used for measurement of CK-18, the reference value can be set as follows.

In the case that the marker molecules are AST and PSAT, AST and LEP, AST and ICAM1, AST, PSAT, and LEP, AST, PSAT, and ICAM1, AST, LEP, and CK-18, AST, ICAM1, and GSTA1, or AST, PSAT, and 6Ckine, the reference value is, for example, a value set to 0.284 to 0.629, and is preferably a value set to 0.284 to 0.439 from the viewpoint of improvement of the accuracy for individuals affected with NASH, and is preferably a value set to 0.393 to 0.629 from the viewpoint of improvement of the accuracy for individuals affected with NAFL.

In the case that the marker molecules are AST and PSAT, the reference value is, for example, a value set to 0.284 to 0.544, and is preferably a value set to 0.284 to 0.397 from the viewpoint of improvement of the accuracy for individuals affected with NASH, and is preferably a value set to 0.397 to 0.544 from the viewpoint of improvement of the accuracy for individuals affected with NAFL.

In the case that the marker molecules are AST and LEP, the reference value is, for example, a value set to 0.309 to 0.629, and is preferably a value set to 0.309 to 0.412 from the viewpoint of improvement of the accuracy for individuals affected with NASH, and is preferably a value set to 0.412 to 0.629 from the viewpoint of improvement of the accuracy for individuals affected with NAFL.

In the case that the marker molecules are AST and ICAM1, the reference value is, for example, a value set to 0.302 to 0.546, and is preferably a value set to 0.302 to 0.406 from the viewpoint of improvement of the accuracy for individuals affected with NASH, and is preferably a value set to 0.406 to 0.546 from the viewpoint of improvement of the accuracy for individuals affected with NAFL.

In the case that the marker molecules are AST, PSAT, and LEP, the reference value is, for example, a value set to 0.303 to 0.619, and is preferably a value set to 0.303 to 0.418 from the viewpoint of improvement of the accuracy for individuals affected with NASH, and is preferably a value set to 0.418 to 0.619 from the viewpoint of improvement of the accuracy for individuals affected with NAFL.

In the case that the marker molecules are AST, PSAT, and ICAM1, the reference value is, for example, a value set to 0.301 to 0.551, and is preferably a value set to 0.301 to 0.406 from the viewpoint of improvement of the accuracy for individuals affected with NASH, and is preferably a value set to 0.406 to 0.551 from the viewpoint of improvement of the accuracy for individuals affected with NAFL.

In the case that the marker molecules are AST, LEP, and CK-18, the reference value is, for example, a value set to 0.297 to 0.625, and is preferably a value set to 0.297 to 0.439 from the viewpoint of improvement of the accuracy for individuals affected with NASH, and is preferably a value set to 0.439 to 0.625 from the viewpoint of improvement of the accuracy for individuals affected with NAFL.

In the case that the marker molecules are AST, ICAM1, and GSTA1, the reference value is, for example, a value set to 0.302 to 0.573, and is preferably a value set to 0.302 to 0.393 from the viewpoint of improvement of the accuracy for individuals affected with NASH, and is preferably a value set to 0.393 to 0.573 from the viewpoint of improvement of the accuracy for individuals affected with NAFL.

In the case that the marker molecules are AST, PSAT, and 6Ckine, the reference value is, for example, a value set to 0.291 to 0.535, and is preferably a value set to 0.291 to 0.416 from the viewpoint of improvement of the accuracy for individuals affected with NASH, and is preferably a value set to 0.416 to 0.535 from the viewpoint of improvement of the accuracy for individuals affected with NAFL.

In another aspect of the present embodiment, one marker molecule may be selected as a marker molecule representative of a given group (module). In this case, the measurement of the marker molecule (the quantity of the marker molecule measured) may be directly used as an index value in place of a normalized score calculated by using the above formula 1.

For example, VCAM1 may be employed as the marker molecule representative of the Group 2 to use the measurement of VCAM1 (e.g., serum level) as an index value. In the case that ELISA with a "Human sVCAM-1/CD106 Quantikine ELISA Kit" is used for measurement of the quantity of VCAM1, the reference value (serum level (ng/mL)) is, for example, a value set to 551.1 to 1280.1, and is preferably a value set to 693.5 to 925.3 from the viewpoint of improvement of the accuracy for individuals affected with NASH.

An index value may be calculated from a normalized score calculated based on the quantities of PROS1 and CLU as the first normalized score, and a normalized score calculated based on the quantity of at least one marker molecule selected from the Group 3, the quantities of AST and PSAT, the quantities of AST and LEP, the quantities of AST and ICAM1, the quantities of AST, PSAT, and LEP, the quantities of AST, PSAT, and ICAM1, the quantities of AST, LEP, and CK-18, the quantities of AST, ICAM1, and GSTA1, or the quantities of AST, PSAT, and 6Ckine as the second normalized score, by using the above formula 2. In this case, examples of the values of $a_1$ and $a_2$ in the formula 2 include values corresponding to "M1 + M3" in Table 9 below. Examples of the reference value for determining whether a subject is possibly affected with NASH include the following values.

In the case that the Human MAP method is used for measurement of PROS1, CLU, PSAT, LEP, ICAM1, GSTA1, and 6Ckine, an enzyme activity measurement method is used for measurement of AST, and ELISA with an "M30 Apoptosense ELISA" is used for measurement of CK-18, the reference value can be set as follows.

In the case that the marker molecules are PROS 1 and CLU, and, AST and PSAT, AST and LEP, AST and ICAM1, AST, PSAT, and LEP, AST, PSAT, and ICAM1, AST, LEP, and CK-18, AST, ICAM1, and GSTA1, or AST, PSAT, and 6Ckine, the reference value is, for example, a value set to 13.493 to 21.205, and is preferably a value set to 13.493 to 18.331 from the viewpoint of improvement of the accuracy for individuals affected with NASH, and is preferably a value set to 16.789 to 21.205 from the viewpoint of improvement of the accuracy for individuals affected with NAFL.

In the case that the marker molecules are PROS 1 and CLU, and, AST and PSAT, the reference value is, for example, a value set to 13.493 to 21.02, and is preferably a value set to 13.493 to 17.141 from the viewpoint of improvement of the accuracy for individuals affected with NASH, and is preferably a value set to 17.141 to 21.02 from the viewpoint of improvement of the accuracy for individuals affected with NAFL.

In the case that the marker molecules are PROS1 and CLU, and, AST and LEP, the reference value is, for example, a value set to 14.228 to 20.755, and is preferably a value set to 14.228 to 18.145 from the viewpoint of improvement of the accuracy for individuals affected with NASH, and is preferably a value set to 18.145 to 20.755 from the viewpoint of improvement of the accuracy for individuals affected with NAFL.

In the case that the marker molecules are PROS 1 and CLU, and, AST and ICAM1, the reference value is, for example, a value set to 13.663 to 20.831, and is preferably a value set to 13.663 to 17.406 from the viewpoint of improvement of the accuracy for individuals affected with NASH, and is preferably a value set to 17.406 to 20.831 from the viewpoint of improvement of the accuracy for individuals affected with NAFL.

In the case that the marker molecules are PROS 1 and CLU, and, AST, PSAT, and LEP, the reference value is, for example, a value set to 14.229 to 20.843, and is preferably a value set to 14.229 to 18.331 from the viewpoint of improvement of the accuracy for individuals affected with NASH, and is preferably a value set to 18.331 to 20.843 from the viewpoint of improvement of the accuracy for individuals affected with NAFL.

In the case that the marker molecules are PROS 1 and CLU, and, AST, PSAT, and ICAM1, the reference value is, for example, a value set to 13.929 to 21.053, and is preferably a value set to 13.929 to 17.749 from the viewpoint of improvement of the accuracy for individuals affected with NASH, and is preferably a value set to 17.749 to 21.053 from the viewpoint of improvement of the accuracy for individuals affected with NAFL.

In the case that the marker molecules are PROS 1 and CLU, and, AST, LEP, and CK-18, the reference value is, for example, a value set to 13.544 to 21.168, and is preferably a value set to 13.544 to 18.319 from the viewpoint of improvement of the accuracy for individuals affected with NASH, and is preferably a value set to 18.319 to 21.168 from the viewpoint of improvement of the accuracy for individuals affected with NAFL.

In the case that the marker molecules are PROS1 and CLU, and, AST, ICAM1, and GSTA1, the reference value is, for example, a value set to 13.788 to 20.882, and is preferably a value set to 13.788 to 17.643 from the viewpoint of improvement of the accuracy for individuals affected with NASH, and is preferably a value set to 17.643 to 20.882 from the viewpoint of improvement of the accuracy for individuals affected with NAFL.

In the case that the marker molecules are PROS 1 and CLU, and, AST, PSAT, and 6Ckine, the reference value is, for example, a value set to 13.844 to 21.205, and is preferably a value set to 13.844 to 16.789 from the viewpoint of improvement of the accuracy for individuals affected with NASH, and is preferably a value set to 16.789 to 21.205 from the viewpoint of improvement of the accuracy for individuals affected with NAFL.

The discrimination method according to the present embodiment can be used as a method for determining the degree of progression of a symptom of NAFLD, NAFL, or NASH. As the index value is larger, it is determined that the degree of progression of a symptom of NAFLD, NAFL, or NASH in the subject is possibly higher. Alternatively, in the case that the index value after application of a therapeutic drug is lower than the index value before the application, it may be determined that the application of the therapeutic drug is possibly effective.

The discrimination method according to the present embodiment can be used as a method for determining the presence of hepatic fibrosis such as a method for discriminating between symptoms of Type-1 to Type-3 and a symptom of Type-4 in the Matteoni classification and a method for discriminating between a symptom of Stage 0, Stage 1, or Stage 2 and a symptom of Stage 4 in the Stages of fibrosis. In the method for determining the presence of hepatic fibrosis, it is determined that the subject possibly has a symptom of hepatic fibrosis in the case that the normalized score is larger than a reference value. While such a symptom has been determined based on pathological findings from a liver biopsy, the discrimination method enables determination through a noninvasive method with a high diagnostic accuracy.

The Matteoni classification is a diagnosis method for NAFL and NASH based on pathological findings from a liver biopsy, and is a method commonly used (Matteoni, C., Younossi, Z. & Gramlich, T.Nonalcoholic fatty liver disease: a spectrum of clinical and pathological severity. Gastroenterology 1413-1419 (1999).). The relation between the Matteoni classification and pathological findings (histological findings) is shown in Table 1. Among symptoms of NASH, hepatic fibrosis has been revealed to be a factor for a poor prognosis in recent years. Accordingly, discrimination of symptoms classified as Type-4 in the Matteoni classification including hepatic fibrosis in an individual affected with NASH from symptoms of NASH is increasingly becoming important.

[Table 1]

| Diagnosis of NAFL and NASH and Matteoni classification | | |
|---|---|---|
| NAFL - NASH Diagnosis | Matteoni classification | Histological findings |
| NAFL | Type 1 | Fatty liver alone |
| | Type 2 | Fat accumulation and lobular inflammation |
| NASH | Type 3 | Fat accumulation and ballooning degeneration |
| | Type 4 | Fat accumulation, ballooning degeneration, and either Mallory hyaline or fibrosis |

The degree of progression of fibrosis is generally evaluated by using "Stage" (Kleiner et.al., Design and validation of a histological scoring system for nonalcoholic fatty liver disease, Hepatology 41: 1313-21 (2005)) shown in the following Table 2 as an indicator.

[Table 2]

| "Stage", Index of "degree of progression of fibrosis" | |
|---|---|
| Fibrosis classification | Histological findings |
| Stage 0 | None |

(continued)

| "Stage", Index of "degree of progression of fibrosis" | |
|---|---|
| Fibrosis classification | Histological findings |
| Stage 1 | Perisinusoidal or periportal fibrosis<br>1A: Mild, central venous region fibrosis<br>1B: Moderate, central venous region fibrosis<br>1C: Portal/periportal region fibrosis |
| Stage 2 | Perisinusoidal and portal/periportal region fibrosis |
| Stage 3 | Bridging fibrosis |
| Stage 4 | Cirrhosis |

The marker molecule or marker molecules to be measured may be all marker molecules belonging to the Group 2, or may include VCAM1 or may be VCAM 1, or may be VCAM1 and HA or may include VCAM1 and HA, or may be VCAM1 and CTSD or may include VCAM1 and CTSD, or may be VCAM1 and COL4 or may include VCAM1 and COL4, or may be VCAM1 and COL4-7S or may include VCAM1 and COL4-7S, or may be HA and COL4-7S or may include HA and COL4-7S, or may be HA and CTSD or may include HA and CTSD, or may be HA and COL4 or may include HA and COL4, or may be CTSD and COL4 or may include CTSD and COL4, or may be CTSD and COL4-7S or may include CTSD and COL4-7S. A normalized score is calculated based on the quantity or quantities of the marker molecule or marker molecules by using the above formula 1.

In the method for discriminating between symptoms of Type-1 to Type-3 and a symptom of Type-4 in the Matteoni classification, it is determined that a subject possibly has a symptom of Type-4 in the Matteoni classification in the case that the normalized score is larger than a reference value. In the case that ELISA with a "Hyaluronan Quantikine ELISA Kit" is used for measurement of HA, the Human MAP method is used for measurement of VCAM1, CTSD, and COL4, and a radioimmunoassay with a "Type IV Collagen 7S Kit" is used for measurement of COL4-7S, setting can be performed as follows.

In the case that the marker molecules are at least two marker molecules belonging to the Group 2, the reference value is, for example, a value set to 0.087 to 0.848.

In the case that the maker molecule or marker molecules is/are all marker molecules belonging to the Group 2, VCAM1, VCAM1 and HA, VCAM1 and CTSD, VCAM1 and COL4, VCAM1 and COL4-7S, HA and COL4-7S, HA and CTSD, HA and COL4, CTSD and COL4, or CTSD and COL4-7S, the reference value is, for example, a value set to 0.303 to 0.617, and is preferably a value set to 0.303 to 0.475 from the viewpoint of improvement of the accuracy for a symptom of Type-4, and is preferably a value set to 0.408 to 0.536 from the viewpoint of improvement of the accuracy for symptoms of Type-1 to Type-3.

In the case that the marker molecules are all marker molecules belonging to the Group 2, the reference value is, for example, a value set to 0.401 to 0.536, and is preferably a value set to 0.401 to 0.45 from the viewpoint of improvement of the accuracy for a symptom of Type-4, and is preferably a value set to 0.45 to 0.536 from the viewpoint of improvement of the accuracy for symptoms of Type-1 to Type-3.

In the case that the maker molecule is VCAM1, the reference value is, for example, a value set to 0.303 to 0.538, and is preferably a value set to 0.303 to 0.421 from the viewpoint of improvement of the accuracy for a symptom of Type-4, and is preferably a value set to 0.421 to 0.538 from the viewpoint of improvement of the accuracy for symptoms of Type-1 to Type-3.

In the case that the maker molecules are VCAM1 and HA, the reference value is, for example, a value set to 0.328 to 0.617, and is preferably a value set to 0.328 to 0.418 from the viewpoint of improvement of the accuracy for a symptom of Type-4, and is preferably a value set to 0.418 to 0.617 from the viewpoint of improvement of the accuracy for symptoms of Type-1 to Type-3.

In the case that the maker molecules are VCAM1 and CTSD, the reference value is, for example, a value set to 0.313 to 0.581, and is preferably a value set to 0.313 to 0.432 from the viewpoint of improvement of the accuracy for a symptom of Type-4, and is preferably a value set to 0.432 to 0.581 from the viewpoint of improvement of the accuracy for symptoms of Type-1 to Type-3.

In the case that the maker molecules are VCAM1 and COL4, the reference value is, for example, a value set to 0.343 to 0.558, and is preferably a value set to 0.343 to 0.457 from the viewpoint of improvement of the accuracy for a symptom of Type-4, and is preferably a value set to 0.457 to 0.558 from the viewpoint of improvement of the accuracy for symptoms of Type-1 to Type-3.

In the case that the maker molecules are VCAM1 and COL4-7S, the reference value is, for example, a value set to 0.358 to 0.602, and is preferably a value set to 0.358 to 0.438 from the viewpoint of improvement of the accuracy for a symptom of Type-4, and is preferably a value set to 0.438 to 0.602 from the viewpoint of improvement of the accuracy for symptoms of Type-1 to Type-3.

In the case that the maker molecules are HA and COL4-7S, the reference value is, for example, a value set to 0.354 to 0.562, and is preferably a value set to 0.354 to 0.451 from the viewpoint of improvement of the accuracy for a symptom of Type-4, and is preferably a value set to 0.451 to 0.562 from the viewpoint of improvement of the accuracy for symptoms of Type-1 to Type-3.

In the case that the maker molecules are HA and COL4, the reference value is, for example, a value set to 0.36 to 0.522, and is preferably a value set to 0.36 to 0.408 from the viewpoint of improvement of the accuracy for a symptom of Type-4, and is preferably a value set to 0.408 to 0.522 from the viewpoint of improvement of the accuracy for symptoms of Type-1 to Type-3.

In the case that the maker molecules are HA and CTSD, the reference value is, for example, a value set to 0.311 to 0.545, and is preferably a value set to 0.311 to 0.432 from the viewpoint of improvement of the accuracy for a symptom of Type-4, and is preferably a value set to 0.432 to 0.545 from the viewpoint of improvement of the accuracy for symptoms of Type-1 to Type-3.

In the case that the maker molecules are CTSD and COL4, the reference value is, for example, a value set to 0.317 to 0.544, and is preferably a value set to 0.317 to 0.436 from the viewpoint of improvement of the accuracy for a symptom of Type-4, and is preferably a value set to 0.436 to 0.544 from the viewpoint of improvement of the accuracy for symptoms of Type-1 to Type-3.

In the case that the maker molecules are CTSD and COL4-7S, the reference value is, for example, a value set to 0.364 to 0.604, and is preferably a value set to 0.364 to 0.475 from the viewpoint of improvement of the accuracy for a symptom of Type-4, and is preferably a value set to 0.475 to 0.604 from the viewpoint of improvement of the accuracy for symptoms of Type-1 to Type-3.

In the case that the in vitro diagnostic reagent "Chemilumi hyaluronic acid" in accordance with a latex agglutination method or CLIA is used for measurement of HA, ELISA with a "Human sVCAM-1/CD106 Quantikine ELISA Kit" is used for measurement of VCAM1, and a radioimmunoassay with a "Type IV Collagen 7S Kit" is used for measurement of COL4-7S, the reference value can be set as follows.

In the case that the maker molecules are VCAM1 and HA, the reference value is, for example, a value set to 0.357 to 0.572, and is preferably a value set to 0.357 to 0.403 from the viewpoint of improvement of the accuracy for a symptom of Type-4, and is preferably a value set to 0.403 to 0.572 from the viewpoint of improvement of the accuracy for symptoms of Type-1 to Type-3.

In the case that the maker molecules are VCAM1 and COL4-7S, the reference value is, for example, a value set to 0.356 to 0.603, and is preferably a value set to 0.356 to 0.426 from the viewpoint of improvement of the accuracy for a symptom of Type-4, and is preferably a value set to 0.426 to 0.603 from the viewpoint of improvement of the accuracy for symptoms of Type-1 to Type-3.

In the case that the maker molecules are HA and COL4-7S, the reference value is, for example, a value set to 0.331 to 0.563, and is preferably a value set to 0.331 to 0.42 from the viewpoint of improvement of the accuracy for a symptom of Type-4, and is preferably a value set to 0.42 to 0.563 from the viewpoint of improvement of the accuracy for symptoms of Type-1 to Type-3.

In the case that the marker molecule is VCAM1, the measurement of VCAM1 (the quantity of VCAM1) may be directly used as an index value in place of a normalized score calculated by using the above formula 1.

In the case that ELISA with a "Human sVCAM-1/CD106 Quantikine ELISA Kit" is used for measurement of the quantity of VCAM1, the reference value (serum level (ng/mL)) is, for example, a value set to 618.1 to 1239.5, and is preferably a value set to 618.1 to 840.0 from the viewpoint of improvement of the accuracy for a symptom of Type-4, and is preferably a value set to 840.0 to 1239.5 from the viewpoint of improvement of the accuracy for symptoms of Type-1 to Type-3.

In the method for discriminating between a symptom of Stage 0, Stage 1, or Stage 2 and a symptom of Stage 3 or Stage 4 in the Stages of fibrosis, it is determined that a subject possibly has a symptom of Stage 3 or Stage 4 in the Stages of fibrosis in the case that the normalized score is larger than a reference value. The reference value to be used in this case can be set as follows in the case that ELISA with a "Hyaluronan Quantikine ELISA Kit" is used for measurement of HA and the Human MAP method is used for measurement of VCAM1.

Examples of the reference value include the following values.

In the case that the marker molecules are at least two marker molecules belonging to the Group 2, the reference value is, for example, a value set to 0.087 to 0.848.

In the case that the marker molecule or marker molecules is/are all marker molecules belonging to the Group 2, VCAM1, VCAM1 and HA, VCAM1 and CTSD, VCAM1 and COL4, VCAM1 and COL4-7S, HA and COL4-7S, HA and CTSD, HA and COL4, CTSD and COL4, or CTSD and COL4-7S, the reference value is, for example, a value set to 0.353 to 0.766, and is preferably a value set to 0.353 to 0.574 from the viewpoint of improvement of the accuracy for a symptom of Stage 3 or Stage 4, and is preferably a value set to 0.453 to 0.766 from the viewpoint of improvement of the accuracy for symptoms of Stage 0, Stage 1, or Stage 2.

In the case that the marker molecules are all marker molecules belonging to the Group 2, the reference value is, for example, a value set to 0.421 to 0.632, and is preferably a value set to 0.421 to 0.544 from the viewpoint of improvement of the accuracy for a symptom of Stage 3 or Stage 4, and is preferably a value set to 0.544 to 0.632 from the viewpoint of improvement of the accuracy for symptoms of Stage 0, Stage 1, or Stage 2.

In the case that the marker molecule is VCAM1, the reference value is, for example, a value set to 0.371 to 0.587, and is preferably a value set to 0.371 to 0.453 from the viewpoint of improvement of the accuracy for a symptom of Stage 3 or Stage 4, and is preferably a value set to 0.453 to 0.587 from the viewpoint of improvement of the accuracy for symptoms of Stage 0, Stage 1, or Stage 2.

In the case that the marker molecules are VCAM1 and HA, the reference value is, for example, a value set to 0.389 to 0.766, and is preferably a value set to 0.389 to 0.519 from the viewpoint of improvement of the accuracy for a symptom of Stage 3 or Stage 4, and is preferably a value set to 0.519 to 0.766 from the viewpoint of improvement of the accuracy for symptoms of Stage 0, Stage 1, or Stage 2.

In the case that the marker molecules are VCAM1 and CTSD, the reference value is, for example, a value set to 0.406 to 0.718, and is preferably a value set to 0.406 to 0.524 from the viewpoint of improvement of the accuracy for a symptom of Stage 3 or Stage 4, and is preferably a value set to 0.524 to 0.718 from the viewpoint of improvement of the accuracy for symptoms of Stage 0, Stage 1, or Stage 2.

In the case that the marker molecules are VCAM1 and COL4, the reference value is, for example, a value set to 0.407 to 0.678, and is preferably a value set to 0.407 to 0.534 from the viewpoint of improvement of the accuracy for a symptom of Stage 3 or Stage 4, and is preferably a value set to 0.534 to 0.678 from the viewpoint of improvement of the accuracy for symptoms of Stage 0, Stage 1, or Stage 2.

In the case that the marker molecules are VCAM1 and COL4-7S, the reference value is, for example, a value set to 0.413 to 0.685, and is preferably a value set to 0.413 to 0.574 from the viewpoint of improvement of the accuracy for a symptom of Stage 3 or Stage 4, and is preferably a value set to 0.574 to 0.685 from the viewpoint of improvement of the accuracy for symptoms of Stage 0, Stage 1, or Stage 2.

In the case that the marker molecules are HA and COL4-7S, the reference value is, for example, a value set to 0.398 to 0.7, and is preferably a value set to 0.398 to 0.539 from the viewpoint of improvement of the accuracy for a symptom of Stage 3 or Stage 4, and is preferably a value set to 0.539 to 0.7 from the viewpoint of improvement of the accuracy for symptoms of Stage 0, Stage 1, or Stage 2.

In the case that the marker molecules are HA and COL4, the reference value is, for example, a value set to 0.392 to 0.683, and is preferably a value set to 0.392 to 0.463 from the viewpoint of improvement of the accuracy for a symptom of Stage 3 or Stage 4, and is preferably a value set to 0.463 to 0.683 from the viewpoint of improvement of the accuracy for symptoms of Stage 0, Stage 1, or Stage 2.

In the case that the marker molecules are HA and CTSD, the reference value is, for example, a value set to 0.373 to 0.69, and is preferably a value set to 0.373 to 0.465 from the viewpoint of improvement of the accuracy for a symptom of Stage 3 or Stage 4, and is preferably a value set to 0.465 to 0.69 from the viewpoint of improvement of the accuracy for symptoms of Stage 0, Stage 1, or Stage 2.

In the case that the marker molecules are CTSD and COL4, the reference value is, for example, a value set to 0.353 to 0.635, and is preferably a value set to 0.353 to 0.469 from the viewpoint of improvement of the accuracy for a symptom of Stage 3 or Stage 4, and is preferably a value set to 0.469 to 0.635 from the viewpoint of improvement of the accuracy for symptoms of Stage 0, Stage 1, or Stage 2.

In the case that the marker molecules are CTSD and COL4-7S, the reference value is, for example, a value set to 0.403 to 0.676, and is preferably a value set to 0.403 to 0.559 from the viewpoint of improvement of the accuracy for a symptom of Stage 3 or Stage 4, and is preferably a value set to 0.559 to 0.676 from the viewpoint of improvement of the accuracy for symptoms of Stage 0, Stage 1, or Stage 2.

In the case that the in vitro diagnostic reagent "Chemilumi hyaluronic acid" in accordance with a latex agglutination method or CLIA is used for measurement of HA, ELISA with a "Human sVCAM-1/CD106 Quantikine ELISA Kit" is used for measurement of VCAM1, and a radioimmunoassay with a "Type IV Collagen 7S Kit" is used for measurement of COL4-7S, the reference value can be set as follows.

In the case that the marker molecules are VCAM1 and HA, the reference value is, for example, a value set to 0.381 to 0.759, and is preferably a value set to 0.381 to 0.526 from the viewpoint of improvement of the accuracy for a symptom of Stage 3 or Stage 4, and is preferably a value set to 0.526 to 0.759 from the viewpoint of improvement of the accuracy for symptoms of Stage 0, Stage 1, or Stage 2.

In the case that the marker molecules are VCAM1 and COL4-7S, the reference value is, for example, a value set to 0.394 to 0.71, and is preferably a value set to 0.394 to 0.565 from the viewpoint of improvement of the accuracy for a symptom of Stage 3 or Stage 4, and is preferably a value set to 0.565 to 0.71 from the viewpoint of improvement of the accuracy for symptoms of Stage 0, Stage 1, or Stage 2.

In the case that the marker molecules are HA and COL4-7S, the reference value is, for example, a value set to 0.373 to 0.698, and is preferably a value set to 0.373 to 0.563 from the viewpoint of improvement of the accuracy for a symptom of Stage 3 or Stage 4, and is preferably a value set to 0.563 to 0.698 from the viewpoint of improvement of the accuracy for symptoms of Stage 0, Stage 1, or Stage 2.

In the case that the marker molecule is VCAM1, the measurement of VCAM1 (the quantity of VCAM1) may be directly used as an index value in place of a normalized score calculated by using the above formula 1.

In the case that ELISA with a "Human sVCAM-1/CD106 Quantikine ELISA Kit" is used for measurement of the quantity of VCAM1, the reference value (serum level) (ng/mL)) is, for example, a value set to 789.8 to 1344.2, and is preferably a value set to 789.8 to 1029.3 from the viewpoint of improvement of the accuracy for a symptom of Stage 3 or Stage 4, and is preferably a value set to 1029.3 to 1344.2 from the viewpoint of improvement of the accuracy for symptoms of Stage 0, Stage 1, or Stage 2.

The discrimination method of the present embodiment can also be used as a method for determining the degree of progression of a symptom of NAFLD, NAFL or NASH. The larger the index value is, it is determined that a subject has a possibly high degree of progression of a symptom of NAFLD, NAFL or NASH. Alternatively, it may also be determined that the application of the therapeutic drug is possibly effective in a case that the index value after the application of the therapeutic drug is lower than the index value before the application.

The discrimination method of the present embodiment can also be used as a method for determining the degree of progression of a symptom of a hepatic disease. The degree of progression of symptoms of hepatic diseases that has been so far determined based on pathological findings by a liver biopsy can be determined by a non-invasive method. Examples of the hepatic disease include NAFLD, alcoholic liver disorders, chronic hepatitis and drug-induced liver disorders. Examples of the degree of progression of a symptom of a hepatic disease include the degree of progression of hepatic fibrosis, the degree of progression of hepatic inflammation, the degree of progression of hepatocellular ballooning degeneration, the degree of progression of NAFLD activity and hepatocellular necrosis. The hepatic fibrosis is a symptom found in, for example, NAFLD, alcoholic liver disorders, chronic hepatitis and drug-induced liver disorders. The hepatic inflammation, particularly liver lobular inflammation, is a symptom found in, for example, NAFLD, alcoholic liver disorders, chronic hepatitis and drug-induced liver disorders. The hepatocellular ballooning degeneration is a symptom found in, for example, NAFLD, alcoholic liver disorders and chronic hepatitis. The hepatocellular necrosis is a symptom found in, for example, NAFLD, alcoholic liver disorders, chronic hepatitis and drug-induced liver disorders.

NAFLD Activity Score (NAS), which is the score for evaluating the NAFLD activity, is now described. NAFLD Activity Score is scored from pathological findings of hepatic steatosis rate, hepatocellular ballooning degeneration and lobular inflammation based respectively on the following Table 3 and determined as the total value thereof. The higher this score is, it is concluded that the severity of a symptom of NAFLD is high.

[Table 3]

| "NAFLD Activity Score", Index for "Activity of NAFLD" | | | |
|---|---|---|---|
| Activity evaluation | Histological findings | | |
| Score | Hepatic steatosis rate | Ballooning degeneration | Lobular inflammation |
| 0 | <5% | None | None |
| 1 | 5 to 33% | Few balloon cells | Up to 1 foci per 200 x field |
| 2 | 33 to 66% | Many cells/prominent ballooning | 2 to 4 foci per 200 x field |
| 3 | >67% | | 5 or more foci per 200 x field |
| * Activity is evaluated using the total score (score 0 to score 8) of 3 items | | | |

The marker molecules measured in the step (1) may include VCAM1, may be VCAM1, may be VCAM1 and HA, may include VCAM1 and HA, may be VCAM1 and COL4, may include VCAM1 and COL4, may be VCAM1 and COL4-7S, may include VCAM1 and COL4-7S, may be HA and COL4-7S, may include HA and COL4-7S, may be HA and COL4, may include HA and COL4. The normalized score is calculated from the above formula 1 based on quantities of the above marker molecules. The normalized score can be used as the index value of the degree of progression of a symptom in a hepatic disease.

When the marker molecule is VCAM1, a measured value of VCAM 1 (a quantity of VCAM1) may directly be the index value in place of the normalized score calculated from the above formula 1.

The higher the index value is, it is determined that a subject has a possibly high degree of progression of hepatic fibrosis, degree of progression of hepatic inflammation, degree of progression of hepatocellular ballooning degeneration, degree of progression of NAFLD activity or degree of progression of hepatocellular necrosis. Alternatively, when an index value is more increased than the index value determined in the last time, it may be determined that a subject has a possibly advanced degree of progression of hepatic fibrosis, degree of progression of hepatic inflammation, degree of progression of hepatocellular ballooning degeneration, degree of progression of NAFLD activity or degree of progression of hepatocellular necrosis. Alternatively, when an index value of the therapeutic drug after the application is lower than the index value before the application, it may also be determined that the application of the therapeutic drug is possibly effective.

Examples of the therapeutic drug include therapeutic drugs for NAFLD, particularly therapeutic drugs for NASH. Examples of the therapeutic drug for NASH include those obvious to a person skilled in the art such as thiazolidin derivatives (rosiglitazone, pioglitazone), HMG-CoA reductase inhibitors (atorvastatin), cholesterol transporter inhibitors (ezetimibe), angiotensin II receptor antagonists (losartan, telmisartan, valsartan) and vitamin E, but not limited thereto. Additionally, the application period of therapeutic drug can be suitably set as necessary and the effect may also be confirmed, for example, 12, 24, 36, 48, 60, 72, 84 and 96 weeks after the application.

Another aspect of the present embodiment provides a kit to be used in the above method of the present embodiment which includes a reagent for detecting the above marker molecules and an instruction for performing the above method.

Further, another aspect of the present embodiment provides a kit to be used in the above method of the present embodiment which includes a reagent for detecting VCAM1. The kit can be considered as a diagnostic reagent for determining whether a subject is affected by nonalcoholic steatohepatitis (NASH), a subject has a symptom of hepatic fibrosis, the degree of progression of hepatic fibrosis is high, the degree of progression of a symptom of hepatocellular ballooning degeneration is high, the degree of progression of hepatic inflammation is high, a subject has a symptom of hepatomegaly, a subject has a symptom of hepatocellular necrosis, a subject has a symptom of hepatocellular apoptosis, a subject has a symptom of hepatocellular degeneration, or a subject has a symptom of hepatic steatosis or a subject has a possibility of having these symptoms.

Examples of the reagent for detecting the above marker molecules include antibodies specifically recognize the above marker molecules, proteins or nucleic acids specifically bind to the above marker molecules and synthetic VCAM1 to be the standard of mass spectrometry. The reagent for detecting the above marker molecules may be those commercially available or produced by a known method.

The instruction for carrying out the above method is not limited as long as it is described in such a way that the method of the present embodiment can be carried out and examples include an instruction which describes the calculation method for the above normalized score or the above index value and the reference value used in the above method.

The above kit may include as necessary, in addition to the above reagent and the instruction, for example, sterilized water, physiological saline, a surfactant, a buffer, a protein stabilizer, a preservative, a blocking solution, a reaction solution, a reaction quenching solution, reagents for a control (for example, tissue samples, biomolecules for a positive control and a negative control), a labeling reagent and a solid phase support.

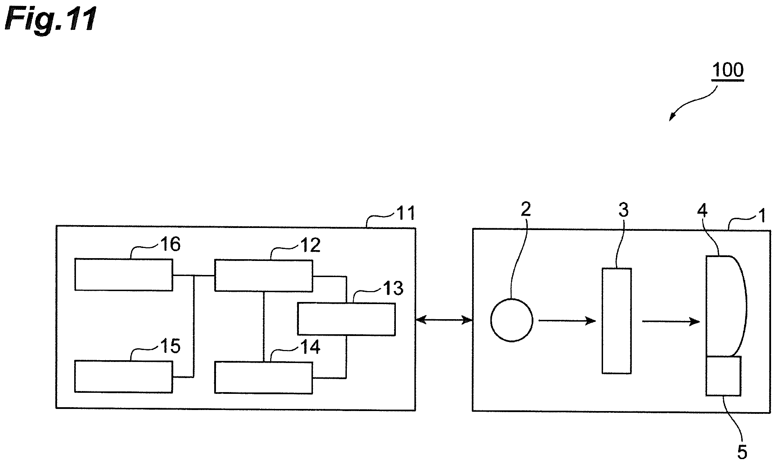

Another aspect of the present embodiment provides a liver disease determination system to be used in the method of the present embodiment. Fig. 11 is a schematic view showing an embodiment of the liver disease determination system. The liver disease determination system 100 comprises, for example, a measurement apparatus 1 configured to measure quantities of the marker molecules contained in blood collected from a subject and a liver disease determination apparatus 11 configured to determine a symptom of a liver disease on a basis of the quantities of the marker molecules measured. Hereinbelow, the details are described.

The measurement apparatus comprises a sample-setting part 3 configured to set a sample therein, a light source part 2 configured to irradiate the sample with light, a detection part 4 configured to detect the light transmitted through the sample and a communication part 5 configured to electrically send the measurement data (measurement data of quantities of the marker molecules) obtained by the detection part 4 to the liver disease determination apparatus 11. The above sample is prepared from, for example, the blood collected from a subject.

The light source part 2 consists of, for example, a single or a plurality of light sources which can irradiate a plurality of wavelengths. More specifically, multi-wavelength light sources such as a halogen lamp, those combining a wavelength selecting filter therewith or an aggregate of LED with different peak wavelengths.

The light source part 2 irradiates a sample set in the sample-setting part 3 with a predetermined wavelength light, and the wavelength can be suitably determined depending on a reagent (fluorescent pigment, etc.) to be detected such as 400 nm to 1000 nm. The light source part 2 herein may be a single light source or a combination light source of a plurality of light sources. Further, when a target to be detected is a light amount of a luminous pigment or radiation emitted by a radioactive isotope, the light source part 2 does not have to be comprised.

The detection part 4 detects the light transmitted through the above sample. The detection part 4 may be those detecting a light amount of a fluorescent pigment or a luminous pigment or may be those detecting radiation emitted by a radioactive isotope.

The communication part 5 electrically sends the measurement data obtained by the detection part 4 to the liver disease determination apparatus 11.

Further, the light source part 2, the detection part 4 and the communication part 5 are electrically connected to the liver disease determination apparatus 11 and the functions thereof may be controlled by a control part 12 provided in the liver disease determination apparatus 11.

A part of Fig. 11 can be considered as a diagrammatic view showing the hardware structure of the liver disease determination apparatus 11. The liver disease determination apparatus 11 comprises the control part 12, a recording part 13 configured to record the information required for analyzing the measurement data sent from the communication part 5, an arithmetic part 14 configured to carry out an arithmetic analysis of the measurement results, a display part 15 configured to display the analysis results and an input part 16 configured to enter the information required for the control. Each function of the liver disease determination apparatus 11 to be described later is executed when a predetermined computer software is run on the hardware such as the recording part 13 and the arithmetic part 14 to allow the input part 16 and the display part 15 to operate under the control of the arithmetic part 14 along with the data readout and writing at the recording part 13 and the arithmetic part 14.

The control part 12 is a control apparatus which can control the working conditions of the liver disease determination system 100 by following a predetermined procedure.

A single computer provided with both functions of the liver disease determination apparatus 11 and the control part 12 may be used or a computer provided with the functions corresponds to the control part 12, the recording part 13, the arithmetic part 14, the display part 15 and the input part 16 may also be used.

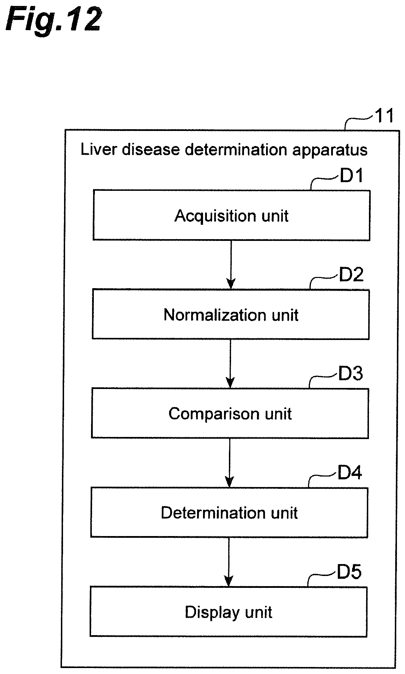

Fig. 12 is a diagrammatic view showing the functional structure of the liver disease determination apparatus 11. The liver disease determination apparatus 11 comprises, as functional components, an acquisition unit D1, a normalization unit D2, a comparison unit D3, a determination unit D4 and a display unit D5.

The acquisition unit D1 is configured to acquire the measurement data (measurement data of quantities of the above marker molecules) from the measurement apparatus 1. The normalization unit D2 is configured to calculate the normalized score from the acquired measurement data based on the above formula 1 and to determine the index value from the calculated normalized score. The comparison unit D3 is configured to compare the index value determined by the normalization unit D2 with a reference value. The determination unit D4 is configured to determine the presence or absence of a hepatic disease based on the compared result. The display unit 5 is configured to display the determined result.

(Determination program for hepatic diseases)

The program to be used in the method of the present embodiment is to allow a computer to function as the acquisition unit D1, the normalization unit D2, the comparison unit D3, the determination unit D4 and the display unit D5 described above. In another aspect, the program allows the computer to execute an acquisition step S1, a normalization step S2, a comparison step S3, a determination step S4 and a display step S5 to be described later. The computer operates as the liver disease determination apparatus 11 by allowing the computer to read the above program. The program is provided, for example, as stored in a recording medium. Note that examples of the recording medium include those such as flexible disks, CD and DVD, those such as ROM and semiconductor memories.

(Method for determining hepatic diseases)

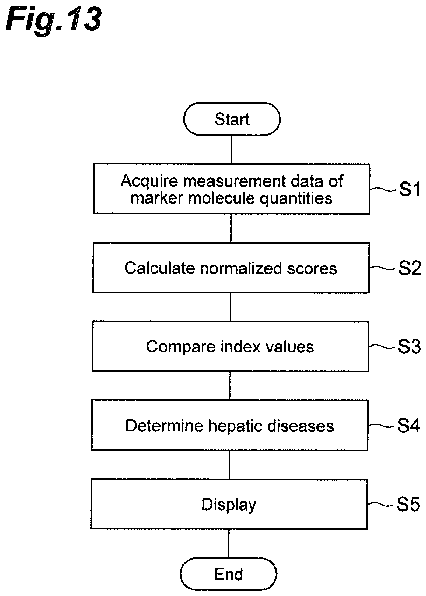

Next, the method for determining a hepatic disease carried out by the liver disease determination apparatus 11 is described. Fig. 13 is a flow chart of the method for determining a hepatic disease. The determination method carried out by the liver disease determination apparatus 11 enables the determination of the presence or absence of a symptom of a hepatic disease quantitatively and automatically with a high accuracy.

[Acquisition step S1]

First, the acquisition unit D1 is configured to acquire the measurement data (measurement data of quantities of the above marker molecules contained in blood collected from a subject) from the measurement apparatus 1.

[Normalization step S2]

Next, the normalization unit D2 is configured to calculate a normalized score from the measurement data acquired by the acquisition unit D1 based on the above formula 1 and determines an index value from the calculated normalized score. For the measurement data, measurement data obtained in advance may be entered in the liver disease determination apparatus 11 instead of acquiring by the acquisition unit D1. Further, the normalized score may directly be used as the index value.

[Comparison step S3]

Next, the comparison unit D3 is configured to compare the index value determined by the normalization unit D2 with a reference value. When the marker molecule to be measured is a single kind, the comparison step S3 may be carried out using the measurement data acquired by the acquisition unit D1 as the index value without carrying out the normalization step S2.

[Determination step S4]

Next, the determination unit D4 is configured to determine the presence or absence of a symptom of a hepatic disease based on the compared result. For example, the determination step S4 determines that a subject is affected by nonalcoholic steatohepatitis (NASH), a subject has a symptom of hepatic fibrosis, the degree of progression of hepatic fibrosis, the degree of progression of a symptom of hepatocellular ballooning degeneration or the degree of progression of a hepatic inflammation is high, a subject has a symptom of hepatomegaly, a subject has a symptom of hepatocellular necrosis, a subject has a symptom of hepatocellular apoptosis, a subject has a symptom of hepatocellular degeneration, a subject has a symptom of hepatic steatosis or a subject has a possibility of having thereof, in a case that an index value is larger than a reference value.

[Display step S5]

Next, the display unit 5 is configured to display the result determined by the determination step S4. For example, the display unit 5 displays whether a target has a symptom of a hepatic disease.

The discrimination method of the present embodiment as described above is non-invasive and enables the discrimination of symptoms of hepatic diseases such as nonalcoholic fatty liver disease with a higher accuracy than the conventional methods. Additionally, the discrimination method can also be used as a method for collecting data to diagnose hepatic diseases such as nonalcoholic fatty liver disease.

In the discrimination method, the index value is determined using the normalized score calculated from the formula 1 but the index value may also be determined using a statistical value other than the normalized score. Examples of the statistical value include maximum values, minimum values, mode values, average values, median values and percentile values. For the statistical value used to determine the index value, one kind of the value may be used singly or a plurality thereof may be used in combination. When a plurality of the values are used in combination, a measured value may be standardized to correct the differences in the unit, scale, concentration, quantity, etc., of the measured value of each marker molecule. Additionally, the distribution of measured values may be converted to a distribution suitable for the discrimination using the Sigmoid function.

**Example**

The present invention is described in detail below with reference to Examples, but is not limited thereto.

Test Example 1: Method for discriminating between NAFL and NASH (1)

132 Patients, classified into the NASH patients and the NAFL patients in advance by Matteoni classification, were measured for quantities of the biomolecules (marker molecules) shown in the following Table 4 in blood. Of the biomolecules, ALB, ALT, AST, COL4-7S and INS$^{IVD}$ were measured using in-vitro diagnostic reagents pharmaceutically approved in Japan. Specifically, ALB was measured by an improved BCP method which is a type of the dye-binding methods. ALT and AST were measured by an enzyme activity measurement method. COL4-7S was measured by the RIA method. INS$^{IVD}$ was measured by the CLIA method. CK-18 was measured by a commercial evaluation kit (M30 Apoptosense (registered trademark) ELISA). HA was measured by a commercial evaluation kit (Hyaluronan Quantikine ELISA Kit). Mac-2bp was measured by a commercial evaluation kit (Human Mac-2 binding protein (Mac-2bp) Assay Kit - IBL). When the commercial evaluation kits were used, a multimode microplate reader "Spectramax M5" (Molecular Devices) was used. P3NP was measured using a commercial evaluation kit (ELISA Kit for Procollagen III N-Terminal Propeptide (PIIINP)). Other biomolecules were measured utilizing a multiplex immunoassay services, Human Discovery MAP (registered trademark) 250+ (v. 2.0) provided by Myriad RBM.

[Table 4]

| | | Biomolecules (Marker molecules) |
|---|---|---|
| | Module 1 (M1, Group 1) | ANG, APOC3, APOD, CFHR1, CLU, Cortisol, EGFR, HPN, IGFBP3, IL1B, IL23A, MET, MMP10, PROS1, Tetranectin, TTR, VDBP, VEGFR-2 |
| | Module 2 (M2, Group 2) | ALB, COL4-7S, AFP, AXL, CCL19, CGB, COL4, CSF1, CTSD, FAS, HA, Mac-2bp, CA19-9, NRCAM, OPG, VCAM1, VWF, YKL-40 |
| | Module 3 (M3, Group 3) | ALT, AST, $INS^{IVD}$, $INS^{MAP}$, 6Ckine, AGT, CK-18, CRP, CXCL10, FABP4, G6PI, GSTA1, HSP-60, ICAM1, LEP, P3NP, PSAT, Testosterone |
| | Module 4 (M4, Group 4) | AREG, BDNF, CD40-L, EREG, FGF2, HBEGF, IGFBP2, MMP9, PAI-1, PDGFB, PLGF, SAP, TGFA, TGFB1, THBS1, VTN |

Next, the normalized scores respectively corresponding to M1 to M4 were determined from quantities of the biomolecules of the same module based on the above formula 1. For the mean ($m_i$), sd ($m_i$), mean ($m_i^{NASH}$) and mean ($m_i^{NAFL}$) in the formula 1, the values shown in the following Table 5 to Table 8 were used.

[Table 5]

| Biomolecule of M1 | mean($m_i$) | sd($m_i$) | mean($m_i^{NASH}$) | mean($m_i^{NAFL}$) |
|---|---|---|---|---|
| ANG | 414.74 | 124.67 | 481.56 | 384.64 |
| APOC3 | 231.59 | 96.87 | 277.80 | 210.77 |
| APOD | 129.81 | 54.44 | 160.27 | 116.09 |
| CFHR1 | 3195.14 | 749.31 | 3447.56 | 3081.41 |
| CLU | 248.88 | 48.24 | 272.24 | 238.35 |
| Cortisol | 352.20 | 510.96 | 370.20 | 344.10 |
| EGFR | 4.12 | 0.79 | 4.45 | 3.97 |
| HPN | 1036.86 | 310.19 | 1167.27 | 978.11 |
| IGFBP3 | 2903.80 | 753.69 | 3203.41 | 2768.81 |
| IL1B | 4.85 | 3.03 | 5.73 | 4.45 |
| IL23A | 1.77 | 0.36 | 1.95 | 1.68 |
| MET | 82.34 | 175.20 | 51.28 | 96.97 |
| MMP10 | 1.34 | 0.57 | 1.51 | 1.26 |
| PROS1 | 15.07 | 2.94 | 17.22 | 14.10 |
| Tetranectin | 13.65 | 2.96 | 15.31 | 12.90 |
| TTR | 27.51 | 7.49 | 32.95 | 25.05 |
| VDBP | 205.15 | 95.64 | 246.32 | 186.60 |
| VEGFR-2 | 7.05 | 1.78 | 8.01 | 6.62 |

[Table 6]

| Biomolecule of M2 | mean($m_i$) | sd($m_i$) | mean($m_i^{NASH}$) | mean($m_i^{NAFL}$) |
|---|---|---|---|---|
| ALB | 4.29 | 0.35 | 4.45 | 4.22 |
| COL4-7S | 5.39 | 1.67 | 4.24 | 5.88 |
| AFP | 2.73 | 4.21 | 1.57 | 3.25 |
| AXL | 15.62 | 5.95 | 12.96 | 16.82 |

(continued)

| Biomolecule of M2 | mean($m_i$) | sd($m_i$) | mean($m_i^{NASH}$) | mean($m_i^{NAFL}$) |
|---|---|---|---|---|
| CCL19 | 767.30 | 937.15 | 535.90 | 871.56 |
| CGB | 1.65 | 0.78 | 1.39 | 1.76 |
| COL4 | 129.32 | 106.79 | 85.10 | 149.24 |
| CSF1 | 0.85 | 0.41 | 0.72 | 0.91 |
| CTSD | 853.89 | 375.08 | 706.02 | 920.52 |
| FAS | 32.28 | 40.69 | 24.83 | 35.64 |
| HA | 193.51 | 227.08 | 72.14 | 248.19 |
| Mac-2bp | 1.84 | 0.86 | 1.52 | 1.99 |
| CA19-9 | 23.91 | 26.75 | 11.76 | 29.39 |
| NRCAM | 1.74 | 1.26 | 1.25 | 1.96 |
| OPG | 7.02 | 2.85 | 5.80 | 7.57 |
| VCAM1 | 632.91 | 242.86 | 521.59 | 683.07 |
| VWF | 91.67 | 39.94 | 74.95 | 99.20 |
| YKL-40 | 85.99 | 84.47 | 43.98 | 104.92 |

[Table 7]

| Biomolecule of M3 | mean($m_i$) | sd($m_i$) | mean($m_i^{NASH}$) | mean($m_i^{NAFL}$) |
|---|---|---|---|---|
| ALT | 89.88 | 65.44 | 61.78 | 102.54 |
| AST | 59.28 | 36.01 | 37.68 | 69.01 |
| INS$^{IVD}$ | 15.25 | 12.51 | 11.14 | 17.21 |
| INS$^{MAP}$ | 2.30 | 3.82 | 2.11 | 2.39 |
| 6Ckine | 627.81 | 238.55 | 497.37 | 686.58 |
| AGT | 229.13 | 602.43 | 72.39 | 299.75 |
| CK-18 | 559.70 | 320.09 | 342.85 | 657.39 |
| CRP | 2.73 | 2.96 | 1.65 | 3.22 |
| CXCL10 | 647.97 | 488.53 | 476.29 | 725.32 |
| FABP4 | 21.01 | 12.74 | 16.30 | 23.13 |
| G6PI | 62.58 | 37.34 | 45.17 | 70.42 |
| GSTA1 | 215.85 | 230.79 | 134.45 | 252.53 |
| HSP-60 | 54.82 | 67.22 | 73.90 | 46.22 |
| ICAM1 | 184.42 | 107.25 | 138.39 | 205.15 |
| LEP | 15.78 | 15.30 | 12.96 | 17.06 |
| P3NP | 13.99 | 6.07 | 11.62 | 15.00 |
| PSAT | 22.64 | 19.68 | 14.23 | 26.44 |
| Testosterone | 3.25 | 1.92 | 3.95 | 2.94 |

[Table 8]

| Biomolecule of M4 | mean($m_i$) | sd($m_i$) | mean($m_i^{NASH}$) | mean($m_i^{NAFL}$) |
|---|---|---|---|---|
| AREG | 344.88 | 142.30 | 459.78 | 293.11 |
| BDNF | 25.45 | 10.51 | 30.32 | 23.26 |
| CD40-L | 2.13 | 1.13 | 2.59 | 1.93 |
| EREG | 66.43 | 40.44 | 94.24 | 53.90 |
| FGF2 | 37.38 | 17.38 | 50.93 | 31.27 |
| HBEGF | 70.89 | 21.00 | 84.22 | 64.88 |
| IGFBP2 | 59.45 | 32.01 | 49.32 | 64.01 |
| MMP9 | 1547.88 | 598.87 | 1816.07 | 1427.04 |
| PAI-1 | 197.98 | 62.65 | 225.41 | 185.62 |
| PDGFB | 15423.86 | 6877.80 | 18728.78 | 13934.84 |
| PLGF | 43.04 | 14.39 | 48.98 | 40.36 |
| SAP | 14.45 | 4.31 | 17.26 | 13.18 |
| TGFA | 17.47 | 7.64 | 20.22 | 16.23 |
| TGFB1 | 11.01 | 3.60 | 12.38 | 10.39 |
| THBS1 | 26881.59 | 12864.55 | 34515.61 | 23442.09 |
| VTN | 2370.23 | 551.50 | 2772.44 | 2189.01 |

Further, when two normalized scores corresponding to the above modules were used, the index value was determined based on the above formula 2. The following values were used herein for $M_{a1}$ (first normalized score), $M_{a2}$ (second normalized score), $a_1$ and $a_2$ in the formula 2.

[Table 9]

| | | $M_{a1}$ | $a_1$ | $M_{a2}$ | $a_2$ |
|---|---|---|---|---|---|
| M1 + M2 | | Normalized score corresponding to M1 | 14.253 | Normalized score corresponding to M2 | 15.104 |
| M1 + M3 | | Normalized score corresponding to M1 | 18.693 | Normalized score corresponding to M3 | 19.524 |
| M1 + M4 | | Normalized score corresponding to M1 | 10.007 | Normalized score corresponding to M4 | 6.045 |
| M2 + M3 | | Normalized score corresponding to M2 | 10.778 | Normalized score corresponding to M3 | 11.578 |
| M2 + M4 | | Normalized score corresponding to M2 | 11.165 | Normalized score corresponding to M4 | 5.698 |
| M3 + M4 | | Normalized score corresponding to M3 | 14.918 | Normalized score corresponding to M4 | 8.189 |

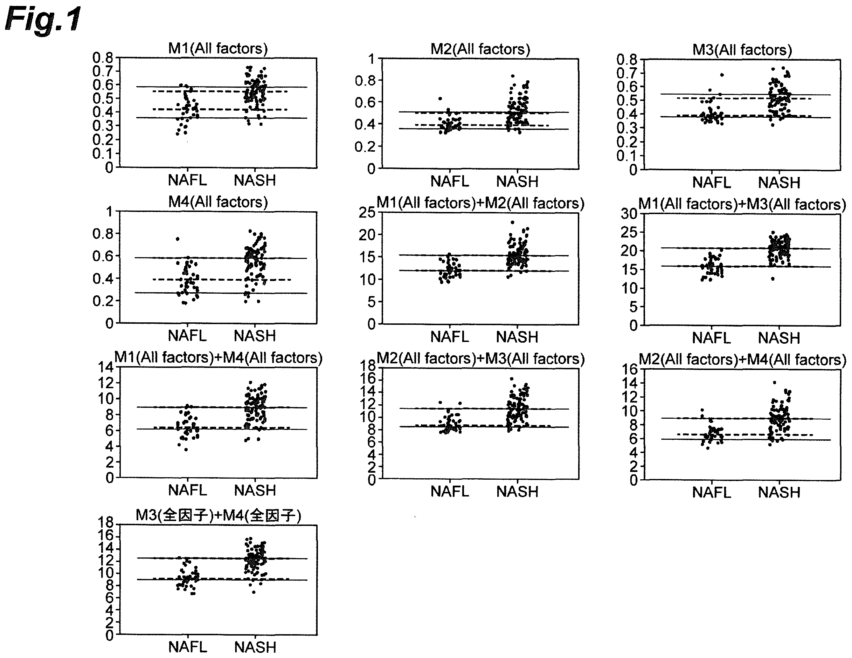

Graphs of the determined normalized scores or index values classified into the NAFL patients group and the NASH patients group and plotted are shown in Fig. 1. In Fig. 1, the solid lines represent both ends of the "reference value range A" in Table 10 and the dotted lines represent both ends of the "reference value range B" in Table 10. In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set within the ranges shown in the following Table 10. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 10]

| NAFL to NASH Discrimination method | AUROC | Reference value range (satisfies at least A or B) | Reference value range A: Sensitivity 95% to Specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M1 (All factors) | 0.832 | 0.359-0.586 | 0.359-0.586 | 0.359 | 95.6 | 26.8 | 0.586 | 39.6 | 95.1 | 0.42-0.553 | 0.420 | 90.1 | 51.2 | 0.553 | 50.5 | 90.2 |
| M2 (All factors) | 0.825 | 0.357-0.509 | 0.357-0.509 | 0.357 | 95.6 | 19.5 | 0.509 | 45.1 | 95.1 | 0.391-0.499 | 0.391 | 90.1 | 51.2 | 0.499 | 50.5 | 92.7 |
| M3 (All factors) | 0.846 | 0.378-0.545 | 0.378-0.545 | 0.378 | 95.6 | 43.9 | 0.545 | 38.5 | 95.1 | 0.388-0.515 | 0.388 | 94.5 | 51.2 | 0.515 | 50.5 | 90.2 |
| M4 (All factors) | 0.818 | 0.272-0.583 | 0.272-0.547 | 0.272 | 95.6 | 22.0 | 0.547 | 58.2 | 95.1 | 0.392-0.583 | 0.392 | 86.8 | 51.2 | 0.583 | 50.5 | 97.6 |
| M1 (All factors) + M2 (All factors) | 0.903 | 12.025-15.396 | 12.259-14.567 | 12.259 | 95.6 | 61.0 | 14.567 | 64.8 | 95.1 | 12.025-15.396 | 12.025 | 95.6 | 51.2 | 15.396 | 50.5 | 97.6 |
| M1 (All factors) + M3 (All factors) | 0.939 | 15.915-20.712 | 16.654-18.766 | 16.654 | 95.6 | 68.3 | 18.766 | 82.4 | 95.1 | 15.915-20.712 | 15.915 | 98.9 | 51.2 | 20.712 | 50.5 | 100.0 |
| M1 (All factors) + M4 (All factors) | 0.859 | 6.219-8.967 | 6.219-8.885 | 6.219 | 95.6 | 46.3 | 8.885 | 52.7 | 95.1 | 6.409-8.967 | 6.409 | 93.4 | 51.2 | 8.967 | 50.5 | 95.1 |
| M2 (All factors) + M3 (All factors) | 0.867 | 8.497-11.466 | 8.497-11.214 | 8.497 | 95.6 | 39.0 | 11.214 | 54.9 | 95.1 | 8.719-11.466 | 8.719 | 94.5 | 51.2 | 11.466 | 50.5 | 95.1 |
| M2 (All factors) + M4 (All factors) | 0.852 | 5.889-8.934 | 5.889-8.651 | 5.889 | 95.6 | 26.8 | 8.651 | 58.2 | 95.1 | 6.599-8.934 | 6.599 | 89.0 | 51.2 | 8.934 | 50.5 | 95.1 |

| NAFL to NASH Discrimination method | AUROC | Reference value range (satisfies at least A or B) | Reference value range A: Sensitivity 95% to Specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M3 (All factors) + M4 (All factors) | 0.899 | 9.023-12.548 | 9.023-12.047 | 9.023 | 95.6 | 46.3 | 12.047 | 61.5 | 95.1 | 9.185-12.548 | 9.185 | 94.5 | 51.2 | 12.548 | 50.5 | 97.6 |

Furthermore, when two normalized scores corresponding to the above modules were used, index values based on the Decision tree (Decision tree 1 to Decision tree 6) described above were also determined and found as follows.

When the marker molecules are selected from the above Group 1 and Group 2,

if $M1_a \geq 0.476$, then I = 2, if $M1_a < 0.476$ and $M2_a \geq 0.466$, then I = 1, and if $M1_a < 0.476$ and $M2_a < 0.466$, then I = 0; (Decision tree 1)

When the marker molecules are selected from the Group 1 and Group 3,

if $M3_a \geq 0.409$ and $M1_a \geq 0.45$, then I = 2, if $M3_a \geq 0.409$ and $M1_a < 0.45$, then I = 1, and if $M3_a < 0.409$, then I = 0; (Decision tree 2)

When the marker molecules are selected from the Group 1 and Group 4,

if $M4_a \geq 0.547$, then I = 2, if $M4_a < 0.547$ and $M1_a \geq 0.468$, then I = 1, and if $M4_a < 0.547$ and $M1_a < 0.468$, then I = 0; (Decision tree 3)

When the marker molecules are selected from the Group 2 and Group 3,

if $M3_a \geq 0.409$ and $M2_a \geq 0.412$, then I = 2, if $M3_a \geq 0.409$ and $M2_a < 0.412$, then I = 1, and if $M3_a < 0.409$, then I = 0; (Decision tree 4)

When the marker molecules are selected from the Group 2 and Group 4,

if $M4_a \geq 0.547$, then I = 2, if $M4_a < 0.547$ and $M2_a \geq 0.413$, then I = 1, and if $M4_a < 0.547$ and $M2_a < 0.413$, then I = 0; (Decision tree 5)

When the marker molecules are selected from the Group 3 and Group 4,

if $M3_a \geq 0.409$, then I = 2, if $M3_a < 0.409$ and $M4_a \geq 0.588$, then I = 1, and if $M3_a < 0.409$ and $M4_a < 0.588$, then I = 0, (Decision tree 6)

wherein I denotes the index value; and $M1_a$, $M2_a$, $M3_a$, and $M4_a$ denote normalized scores for the Group 1, Group 2, Group 3, and Group 4, respectively.

In all the index values, significant differences were found between the NAFL patients group and the NASH patients group, thereby revealing the effectiveness in the discrimination between NAFL and NASH. It is also revealed that the values shown in the following Table 11 are effective as the reference value. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 11]

| NAFL to NASH Discrimination method | Index value based on decision tree | | | AUROC | Examples of Reference value | | |
|---|---|---|---|---|---|---|---|
| | First node | second node | Index value | | Reference value | Sensitivity | Specificity |
| $M1_a + M2_a$ (Decision tree 1) | $M1_a \geq 0.476$ | None | 2 | 0.82 | 0 | 0.945 | 0.683 |
| | $M1_a < 0.476$ | $M2_a \geq 0.466$ | 1 | | | | |
| | | $M2_a \geq 0.466$ | 0 | | | | |
| $M1_a + M3_a$ (Decision tree 2) | $M3_a \geq 0.409$ | $M1_a \geq 0.45$ | 2 | 0.87 | 1 | 0.747 | 0.927 |
| | | $M1_a < 0.45$ | 1 | | | | |
| | $M3_a < 0.409$ | None | 0 | | | | |
| $M1_a + M4_a$ (Decision tree 3) | $M4_a \geq 0.547$ | None | 2 | 0.857 | 0 | 0.890 | 0.683 |
| | $M1_a < 0.547$ | $M2_a \geq 0.468$ | 1 | | | | |
| | | $M2_a \geq 0.468$ | 0 | | | | |

(continued)

| NAFL to NASH Discrimination method | Index value based on decision tree | | | AUROC | Examples of Reference value | | |
| --- | --- | --- | --- | --- | --- | --- | --- |
| | First node | second node | Index value | | Reference value | Sensitivity | Specificity |
| $M2_a + M3_a$ (Decision tree 4) | $M3_a \geq 0.409$ | $M2_a \geq 0.412$ | 2 | 0.872 | 1 | 0.758 | 0.927 |
| | | $M2_a < 0.412$ | 1 | | | | |
| | $M3_a < 0.409$ | None | 0 | | | | |
| $M2_8 + M4_8$ (Decision tree 5) | $M4_a \geq 0.547$ | None | 2 | 0.852 | 0 | 0.879 | 0.683 |
| | $M4_a < 0.547$ | $M2_a \geq 0.413$ | 1 | | | | |
| | | $M2_a \geq 0.413$ | 0 | | | | |
| $M3_a + M4_a$ (Decision tree 6) | $M3_a \geq 0.409$ | None | 2 | 0.815 | 0 | 0.945 | 0.683 |
| | $M3_a < 0.409$ | $M4_a \geq 0.588$ | 1 | | | | |
| | | $M2_a \geq 0.588$ | 0 | | | | |

Further, when two normalized scores corresponding to the above modules were used, index values based on the arithmetic averaging described above were also determined and found as follows.

For the preferable index value, the value calculated from the following formula 3 is used. In the formula 3, I denotes the index value, $M_{a1}$ and $M_{a2}$ respectively denote the normalized score of one of the above two groups and the normalized score of the other ("first normalized score" and "second normalized score").

$$I = (M_{a1} + M_{a2})/2 \qquad \text{(formula 3)}$$

The values shown in the following Table 12 were used herein for $M_{a1}$ (first normalized score) and $M_{a2}$ (second normalized score) in the formula 3.

[Table 12]

| | $M_{a1}$ | $M_{a2}$ |
| --- | --- | --- |
| M1 + M2 | Normalized score corresponding to M1 | Normalized score corresponding to M2 |
| M1 + M3 | Normalized score corresponding to M1 | Normalized score corresponding to M3 |
| M1 + M4 | Normalized score corresponding to M1 | Normalized score corresponding to M4 |
| M2 + M3 | Normalized score corresponding to M2 | Normalized score corresponding to M3 |
| M2 + M4 | Normalized score corresponding to M2 | Normalized score corresponding to M4 |
| M3 + M4 | Normalized score corresponding to M3 | Normalized score corresponding to M4 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 13. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 13]

| NAFL to NASH Discrimination method | AUROC | Reference value | Sensitivity | Specificity |
|---|---|---|---|---|
| M1 (All factors) + M2 (All factors) | 0.904 | 0.450 | 0.890 | 0.780 |
| M1 (All factors) + M3 (All factors) | 0.939 | 0.490 | 0.824 | 0.951 |
| M1 (All factors) + M4 (All factors) | 0.859 | 0.498 | 0.725 | 0.854 |
| M2 (All factors) + M3 (All factors) | 0.866 | 0.453 | 0.791 | 0.829 |
| M2 (All factors) + M4 (All factors) | 0.848 | 0.465 | 0.791 | 0.878 |
| M3 (All factors) + M4 (All factors) | 0.891 | 0.494 | 0.769 | 0.902 |

Further, when two normalized scores corresponding to the above modules were used, index values based on the linear regression equation described above were also determined and found as follows.

For the preferable index value, the value calculated from the following formula 4 is used. In the formula 4, I denotes the index value, $M_{a1}$ and $M_{a2}$ respectively denote the normalized score of one of the above two groups and the normalized score of the other ("first normalized score" and "second normalized score"), $b_1$ and $b_2$ denote the constants determined by the linear regression analysis.

$$I = b_1 \times M_{a1} + b_2 \times M_{a2} \qquad \text{(formula 4)}$$

The values shown in the following Table 14 were used herein for $M_{a1}$ (first normalized score), $M_{a2}$ (second normalized score), $b_1$ and $b_2$ in the formula 4.

[Table 14]

| | $M_{a1}$ | $M_{a2}$ | b1 | b2 |
|---|---|---|---|---|
| M1 + M2 | Normalized score corresponding to M1 | Normalized score corresponding to M2 | 1.885 | 1.729 |
| M1 + M3 | Normalized score corresponding to M1 | Normalized score corresponding to M3 | 2.113 | 2.405 |
| M1 + M4 | Normalized score corresponding to M1 | Normalized score corresponding to M4 | 1.69 | 0.97 |
| M2 + M3 | Normalized score corresponding to M2 | Normalized score corresponding to M3 | 1.473 | 1.952 |
| M2 + M4 | Normalized score corresponding to M2 | Normalized score corresponding to M4 | 1.519 | 1.081 |
| M3 + M4 | Normalized score corresponding to M3 | Normalized score corresponding to M4 | 2.242 | 1.311 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 15. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 15]

| NAFL to NASH Discrimination method | AUROC | Reference value | Sensitivity | Specificity |
|---|---|---|---|---|
| M1 (All factors) + M2 (All factors) | 0.904 | 1.629 | 0.901 | 0.780 |
| M1 (All factors) + M3 (All factors) | 0.941 | 2.148 | 0.857 | 0.902 |

(continued)

| NAFL to NASH Discrimination method | AUROC | Reference value | Sensitivity | Specificity |
|---|---|---|---|---|
| M1 (All factors) + M4 (All factors) | 0.860 | 1.196 | 0.846 | 0.732 |
| M2 (All factors) + M3 (All factors) | 0.869 | 1.474 | 0.868 | 0.780 |
| M2 (All factors) + M4 (All factors) | 0.850 | 1.171 | 0.802 | 0.854 |
| M3 (All factors) + M4 (All factors) | 0.899 | 1.692 | 0.802 | 0.854 |

Further, when two normalized scores corresponding to the above modules were used, index values based on the linear discriminant analysis described above were also determined and found as follows.

For the preferable index value, the value calculated from the following formula 5 is used. In the formula 5, I denotes the index value, $M_{a1}$ and $M_{a2}$ respectively denote the normalized score of one of the above two groups and the normalized score of the other ("first normalized score" and "second normalized score"), $C_1$ and $C_2$ denote the constants determined by the linear discriminant analysis.

$$I = c_1 \times M_{a1} + c_2 \times M_{a2} \qquad \text{(formula 5)}$$

The values shown in the following Table 16 were used herein for $M_{a1}$ (first normalized score), $M_{a2}$ (second normalized score), $c_1$ and $c_2$ in the formula 5.

[Table 16]

| | $M_{a1}$ | $M_{a2}$ | c1 | c2 |
|---|---|---|---|---|
| M1 + M2 | Normalized score corresponding to M1 | Normalized score corresponding to M2 | 7.755 | 5.78 |
| M1 + M3 | Normalized score corresponding to M1 | Normalized score corresponding to M3 | 8.543 | 9.254 |
| M1 + M4 | Normalized score corresponding to M1 | Normalized score corresponding to M4 | 6.947 | 3.574 |
| M2 + M3 | Normalized score corresponding to M2 | Normalized score corresponding to M3 | 4.997 | 8.024 |
| M2 + M4 | Normalized score corresponding to M2 | Normalized score corresponding to M4 | 5.081 | 4.771 |
| M3 + M4 | Normalized score corresponding to M3 | Normalized score corresponding to M4 | 8.564 | 5.16 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 17. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 17]

| NAFL to NASH Discrimination method | AUROC | Reference value | Sensitivity | Specificity |
|---|---|---|---|---|
| M1 (All factors) + M2 (All factors) | 0.902 | 6.155 | 0.879 | 0.780 |
| M1 (All factors) + M3 (All factors) | 0.940 | 8.756 | 0.802 | 0.951 |
| M1 (All factors) + M4 (All factors) | 0.859 | 4.912 | 0.802 | 0.780 |
| M2 (All factors) + M3 (All factors) | 0.871 | 5.512 | 0.890 | 0.780 |
| M2 (All factors) + M4 (All factors) | 0.848 | 4.553 | 0.791 | 0.878 |
| M3 (All factors) + M4 (All factors) | 0.899 | 6.536 | 0.813 | 0.854 |

Further, when two normalized scores corresponding to the above modules were used, index values based on the geometric averaging described above were also determined and found as follows.

For the preferable index value, the value calculated from the following formula 6 is used. In the formula 6, I denotes the index value, $M_{a1}$ and $M_{a2}$ respectively denote the normalized score of one of the above two groups and the normalized score of the other ("first normalized score" and "second normalized score").

$$I = \sqrt{M_{a1} \times M_{a2}} \quad \text{(formula 6)}$$

The values shown in the following Table 18 were used herein for $M_{a1}$ (first normalized score) and $M_{a1}$ (second normalized score) in the formula 6.

[Table 18]

|  | $M_{a1}$ | $M_{a2}$ |
|---|---|---|
| M1 + M2 | Normalized score corresponding to M1 | Normalized score corresponding to M2 |
| M1 + M3 | Normalized score corresponding to M1 | Normalized score corresponding to M3 |
| M1 + M4 | Normalized score corresponding to M1 | Normalized score corresponding to M4 |
| M2 + M3 | Normalized score corresponding to M2 | Normalized score corresponding to M3 |
| M2 + M4 | Normalized score corresponding to M2 | Normalized score corresponding to M4 |
| M3 + M4 | Normalized score corresponding to M3 | Normalized score corresponding to M4 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 19. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 19]

| NAFL to NASH Discrimination method | AUROC | Reference value | Sensitivity | Specificity |
|---|---|---|---|---|
| M1 (All factors) + M2 (All factors) | 0.903 | 0.450 | 0.879 | 0.780 |
| M1 (All factors) + M3 (All factors) | 0.939 | 0.473 | 0.846 | 0.902 |
| M1 (All factors) + M4 (All factors) | 0.858 | 0.447 | 0.824 | 0.756 |
| M2 (All factors) + M3 (All factors) | 0.868 | 0.433 | 0.857 | 0.780 |
| M2 (All factors) + M4 (All factors) | 0.848 | 0.458 | 0.791 | 0.854 |
| M3 (All factors) + M4 (All factors) | 0.888 | 0.481 | 0.802 | 0.878 |

Further, when two normalized scores corresponding to the above modules were used, index values based on the linear equation multiplication described above were also determined and found as follows.

For the preferable index value, the value calculated from the following formula 7 is used. In the formula 7, I denotes the index value, $M_{a1}$ and $M_{a2}$ respectively denote the normalized score of one of the above two groups and the normalized score of the other ("first normalized score" and "second normalized score"), $d_1$, $d_2$ and $d_3$ denote the constants determined by the non-linear regression analysis.

$$I = d_1 \times M_{a1} + d_2 \times M_{a2} + d_3 \times M_{a1} \times M_{a2} \quad \text{(formula 7)}$$

The values shown in the following Table 20 were used herein for $M_{a1}$ (first normalized score), $M_{a2}$ (second

normalized score), $d_1$, $d_2$ and $d_3$ in the formula 7.

[Table 20]

| | $M_{a1}$ | $M_{a2}$ | d1 | d2 | d3 |
|---|---|---|---|---|---|
| M 1 + M2 | Normalized score corresponding to M1 | Normalized score corresponding to M2 | 4.606 | 4.711 | -5.738 |
| M1 + M3 | Normalized score corresponding to M1 | Normalized score corresponding to M3 | 0.863 | 1.17 | 2.682 |
| M 1 + M4 | Normalized score corresponding to M1 | Normalized score corresponding to M4 | 1 | 0.366 | 1.305 |
| M2 + M3 | Normalized score corresponding to M2 | Normalized score corresponding to M3 | 3.56 | 3.964 | -4.246 |
| M2 + M4 | Normalized score corresponding to M2 | Normalized score corresponding to M4 | 3.09 | 2.239 | -2.683 |
| M3 + M4 | Normalized score corresponding to M3 | Normalized score corresponding to M4 | 1.584 | 0.658 | 1.416 |

In all the normalized scores and index values, significant differences were found between the NAFL patients group and the NASH patients group, thereby revealing the effectiveness in the discrimination between NAFL and NASH. Moreover, the reference value (cutoff value) to discriminate the NASH affected is effective when set to the values shown in the following Table 21. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 21]

| NAFL to NASH Discrimination method | AUROC | Reference value | Sensitivity | Specificity |
|---|---|---|---|---|
| M1 (All factors) + M2 (All factors) | 0.902 | 3.031 | 0.901 | 0.780. |
| M1(All factors) + M3 (All factors) | 0.940 | 1.563 | 0.846 | 0.902 |
| M1 (All factors) + M4 (All factors) | 0.860 | 0.871 | 0.846 | 0.732 |
| M2 (All factors) + M3 (All factors) | 0.866 | 2.461 | 0.868 | 0.756 |
| M2 (All factors) + M4 (All factors) | 0.851 | 1.850 | 0.802 | 0.854 |
| M3 (All factors) + M4 (All factors) | 0.900 | 1.331 | 0.835 | 0.829 |

Further, when two normalized scores corresponding to the above modules were used, index values based on the neural network described above were also determined and found as follows.

For the preferable index value, the values calculated from the following formula 8 and formula 9 are used. In the formula 8, $A_1$, $B_1$, $X_1$ and $X_2$ respectively denote the constant determined by the neural network. In the formula 9, I denotes the index value, $A_2$, $B_2$ and $X_2$ denote the constants determined by the neural network.

$$X_2 = f(A_1 \cdot X_1 + B_1) \qquad (\text{formula } 8)$$

$$I = f(A_2 \cdot X_2 + B_2) \qquad (\text{formula } 9)$$

wherein,

$$f(x) = \frac{1}{1 + e^{-x}},$$

the "·" denotes the dot product of matrix, $X_1$ is the matrix of 2 x 1, $A_1$ is the matrix of 2 x 2, $B_1$ is the matrix of 2 x 1, $X_2$ is the matrix of 2 x 1, $A_2$ is the matrix of 1 x 2, and $B_2$ is a real number.

The values shown in the following Table 22 were used for $A_1$, $B_1$, $X_1$, $A_2$ and $B_2$.

[Table 22]

|  | $x_1$ | $A_1$ | $B_1$ | $B_2$ |
|---|---|---|---|---|
| M1+M2 | [[$M_{a1}$],[$M_{a2}$]] | [[46.992,38.658],[-12.907,-12.553]] | [[-39.335],[3.592]] | [-26.046] |
| M1+M3 | [[$M_{a1}$],[$M_{a2}$]] | [[-2.459,-3.359],[-7.585,-8.645]] | [[-2.971],[8.653]] | [9.002] |
| M1+M4 | [[$M_{a1}$],[$M_{a2}$]] | [[4.894,12.162],[14.443,0.505]] | [[-11.443],[-3.38]] | [-11.274] |
| M2+M3 | [[$M_{a1}$],[$M_{a2}$]] | [[-4.143,-6.075],[3.085,3.442]] | [[3.564],[1.64]] | [0.677] |
| M2+M4 | [[$M_{a1}$],[$M_{a2}$]] | [[-14.87,2.227],[4.188,-21.96]] | [[3.196],[11.856]] | [13.664] |
| M3+M4 | [[$M_{a1}$],[$M_{a2}$]] | [[8.414,11.694],[-4.648,-2.674]] | [[0.772],[3.909]] | [4.232] |

$M_{a1}$ and $M_{a2}$ in the above Table 22 respectively denote the normalized score of one of the above two groups and the normalized score of the other ("first normalized score" and "second normalized score"). The values shown in the following Table 23 were used for $M_{a1}$ and $M_{a2}$.

[Table 23]

|  | $M_{a1}$ | $M_{a2}$ |
|---|---|---|
| M1 + M2 | Normalized score corresponding to M1 | Normalized score corresponding to M2 |
| M1 + M3 | Normalized score corresponding to M1 | Normalized score corresponding to M3 |
| M1 + M4 | Normalized score corresponding to M1 | Normalized score corresponding to M4 |
| M2 + M3 | Normalized score corresponding to M2 | Normalized score corresponding to M3 |
| M2 + M4 | Normalized score corresponding to M2 | Normalized score corresponding to M4 |
| M3 + M4 | Normalized score corresponding to M3 | Normalized score corresponding to M4 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 24. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 24]

| NAFL to NASH Discrimination method | AUROC | Reference value | Sensitivity | Specificity |
|---|---|---|---|---|
| M1 (All factors) + M2 (All factors) | 0.871 | 0.573 | 0.901 | 0.780 |
| M1 (All factors) + M3 (All factors) | 0.941 | 0.454 | 0.857 | 0.902 |
| M1 (All factors) + M4 (All factors) | 0.867 | 0.475 | 0.736 | 0.854 |
| M2 (All factors) + M3 (All factors) | 0.872 | 0.357 | 0.890 | 0.780 |
| M2 (All factors) + M4 (All factors) | 0.867 | 0.399 | 0.813 | 0.829 |

(continued)

| NAFL to NASH Discrimination method | AUROC | Reference value | Sensitivity | Specificity |
|---|---|---|---|---|
| M3 (All factors) + M4 (All factors) | 0.899 | 0.441 | 0.835 | 0.829 |

Further, when two normalized scores corresponding to the above modules were used, index values based on the Support Vector Machine described above were also determined and found as follows.

For the preferable index value, the value calculated from the following formula 10 is used. In the formula 10, I denotes the index value, N, $\alpha$, $\chi$ and v denote the constants determined by the Support Vector Machine.

$$I = \sum_{i=1}^{M} \alpha_i \times K(x, v_i)$$

(formula 10)

wherein,

$K(x,v) = (x^T \cdot v)^2$ the "·" denotes the dot product of matrix, $\chi$ is the matrix of 2 x 1, $\chi^T$ is the transposed matrix of $\chi$, $\alpha$ is the matrix of 1 x N, $\alpha_i$ is a real number at the i row of $\alpha$, v is the matrix of 2 x N, $v_i$ is the matrix of 2 x 1 at the i row of v, and the values shown in the following Table 25 were used for N, $\alpha$, $\chi$ and v.

[Table 25]

| | χ | N | α | v |
|---|---|---|---|---|
| M1+M2 | [[M$_{a1}$]-[M$_{a2}$]] | 32 | [-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311] | [[0.55,0.249,0.387,0.378,0.597,0.291,0.305,0.293,0.496,0.567,0.585,0.243,0.591,0.36,0.446,0.288,0.473,0.518,0.348,0.336,0.455,0.419,0.532,0.413,0.351,0.406,0.398,0.504,0.425,0.27,0.376,0.424,0.432,0.363,0.485,0.447,0.495,0.356,0.435,0.457,0.373,0.587,0.424,0.338,0.437,0.515,0.606,0.36,0.538.0.376,0.509,0.409,0.578,0.515,0.63,0.481,0.622,0.562,0.668,0.503,0.614,0.506,0.592,0.52,0.598.0.584,0.315,0.611,0.533,0.591,0.672,0.502,0.313,0.536,0.627,0.564,0.541,0.518,0.364,0.685,0.605,0.459,0.44,0.544,0.501,0.574,0.508,0.711,0.504,0.662,0.483,0.557,0.678,0.491,0.619,0.723,0.566,0.626.0.404,0.499,0.48,0.577,0.732,0.613,0.641,0.454,0.49,0.421,0.357,0.603,0.487,0.555,0.58,0.479,0.672,0.53,0.544,0.538,0.668,0.701,0.603,0.635,0.501,0.637,0.48,0.653,0.625,0.489,0.668,0.73,0.669,0.622],[0.445,0.4,0.323,0.375,0.364,0.531,0.417,0.353,0.397,0.361,0.484,0.446,0.393,0.406,0.408,0.37,0.346,0.509,0.632,0.382,0.326,0.382,0.341,0.375,0.412,0.352,0.385,0.438,0.336,0.373,0.37,0.437,0.406,0.361,0.494,0.412,0.344,0.368,0.389,0.442,0.45,0.441,0.489,0.408,0.482,0.548,0.532,0.361,0.484,0.421,0.413,0.403,0.42,0.444,0.459,0.358,0.364,0.391,0.528,0.387,0.385,0.499,0.355,0.344,0.399,0.569,0.727,0.42,0.539,0.626,0.634,0.542,0.632,0.427,0.431,0.504,0.413,0.564,0.736,0.562,0.414,0.68,0.635,0.506,0.6,0.499,0.416,0.842,0.509,0.752,0.491,0.49,0.688,0.414,0.679,0.639,0.694,0.514,0.445,0.41,0.601,0.353,0.462,0.572,0.327,0.647,0.409,0.587,0.506,0.638,0.598,0.554,0.762,0.454,0.523,0.758,0.412,0.437,0.788,0.736,0.45,0.589,0.547,0.46,0.509,0.587,0.478,0.484,0.599,0.459,0.502,0.466]] |

45

| | $\chi$ | N | $\alpha$ | $\nu$ |
|---|---|---|---|---|
| M1+M3 | [[M_{a1}], [M_{a2}]] | 132 | [-0.689,-0.689,-0.689,-0.689,-0.689,-0.689,-0.689,-0.689.-0.689,-0.689,-0.689,-0.689,-0.689,-689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689-0.689,-0.689,-0.689,-0.689,-0.689,-0.689,-0.689,-0.689,-0.689,0.311,0.311,0.311,0.311,0.311,0.311,0.311,0.311,0.311, 0.311,0.311,0.311,0.311,0.311,0.311,0.311,0.311,0.311,0.311. 0.311,0.311,0.311,0.311,0.311,0.311,0.311,0.311,0.311,0.311, 0.311,0.311,0.311,0.311,0.311,0.311,0.311,0.311,0.3i 1, 0.311,0.311,0.311,0.311,0.311,0.311,0.311,0.311,0.311,0.311. 0.311,0.311,0.311,0.311,0.311,0.311,0.311,0.311,0.311,0.311, 0.311,0.311,0.311,0.311,0.311,0.311,0.311,0.311,0.311,0.311, 0.311,0.311,0.311,0.311,0.311,0.311,0.311.0.311,0.311,0.311, 0.311,0.311,0.311,0.311,0.311,0.311,0.311,0.311,0.311,0.311,0.311, 0.311,0.311] | [ [0.55,0.249,0.387,0.378.0.597,0.291,0.305,0.293.0,496,0.567,0.585,0.243,0.591,0.36,0.446,0.288,0. 473,0.518,0.348,0.336,0.455,0.419,0.532,0.413,0.351,0.406,0.398,0.504,0.425,0.27,0.376,0.424,0.432,0.363,0.485,0.447,0.495,0.356,0.435,0.457,0.373,0.587,0.424,0.338,0.437,0.515,0.606,0.36,0.538.0 . 376,0.509,0.409,0.578,0.515,0.63,0.481,0.622,0.562,0.668,0.503,0.614,0.506,0.592,0.52,0.598,0.584 , 0.315,0.611,0.533,0.591,0.672,0.502,0.313,0.536,0.627,0.564,0.541,0.518,0.364,0.685,0.605,0.459,0 . 44,0.544,0.501,0.574,0.508,0.711,0.504,0.662,0.483,0.557,0.678,0.491,0.619,0.723,0.566,0.626,0.404,0.499,0.48,0.577,0.732,0.613,0.641,0.454,0.49,0.421,0.357,0.603,0.487,0.555,0.58,0.479,0.672,0.530.544,0.538,0.668,0.701,0.603,0.635,0.501,0.637,0.48,0.653,0.625,0.489,0.668,0.73,0.669,0.622], [ 0.403,0.575,0.35,0.335,0.336,0.349,0.366,0.349,0.343,0.363,0.379,0.393,0.372,0.687,0.497,0.441,0.389,0.488,0.488,0.396,0.355,0.347,0.342,0.37,0.489,0.405,0.386,0.373,0.386,0.402,0.365,0.377,0.432. 0.328,0.447,0.519,0.418,0.371,0.544,0.399,0.404,0.652,0.558,0.32,0.412,0.634,0.559,0.555,0.534,0.509,0.506,0.507,0.391,0.395,0.609,0.379,0.474,0.442,0.371,0.418,0.423,0.417,0.361,0.357,0.391,0.52, 0.731,0.522,0.634,0.681,0.635,0.656,0.679,0.534,0.574,0.526,0.55,0.5,0.493,0.562,0.482,0.618,0.504 , 0.529,0.679,0.512,0.454,0.395,0.587,0.471,0.502,0.404,0.53,0.551,0.554,0.424,0.686,0.421,0.584,0. 498,0.567,0.494,0.465,0.504,0.43,0.71,0.508,0.58,0.738,0.471,0.615,0.431,0.531,0.622,0.519,0.605,0 . 556,0.546,0.547,0.519,0.489,0.48,0.698,0.469,0.512,0.572,0.4,0.509,0.521,0.457,0.587.0.493]] |

(continued)

| | χ | N | α | ν |
|---|---|---|---|---|
| M1+M4 | [[M$_{a1}$], [M$_{a2}$]] | 131 | [-0.689,-0.291,-0.689,-0.689,-0.689,-0.689,-0.689,-0.689,-0.689,-0.689,-0.689,-0.689,-0.689.-0.689,-0.689,-0.689,-0.689,-0.689,-0.689,-0.689,-0.689,-0.689,-0.689.-0.689,-0.689,-0.689,-0.68,-0.689,-0.68,-0.689,-0.689,-0.689,-0.689,-0.689,-0.689,-0.689,-0.689,-0.689,0.311,0.311,0.311,0.311,0.311,0.311,0.311,0.311,0.311, 0.311,0.311,0.311,0.311,0.311,0.311,0.311,0.311,0.311, 0.311,0.311,0.311,0.311,0.311,0.311,0.311,0.311,0.311. 0.311,0.311,0.311,0.311,0.311,0.311,0.311,0.311,0.311, 0.311,0.311,0.311,0.311,0.311,0.311,0.311,0.311,0.311. 0.311,0.311,0.311,0.311,0.311,0.223,0.311,0.311,0.311,0.311, 0.311,0.311,0.311,0.311,0.311,0.311,0.311,0.311,0.311, 0.311,0.311,0.311,0.311,0.311,0.311,0.311,0.311,0.311, 0.311, 0.311]] | [ [0.55,0.249,0.387,0.378,0.597,0.291,0.305,0.293,0.496,0.567,0.585,0.243,0.591,0.36,0.446,0.288,0. 473,0.518,0.348,0.336,0.455,0.419,0.532,0.413,0.351,0.406,0.398,0.504,0.425,0.27,0.376,0.424,0.43 2,0.363,0.485,0.447,0.495,0.356,0.435,0.457,0.373,0.587,0.424,0.338.0,437,0.515.0.606, 0.36,0.538,0 . 376,0.509,0.409,0.578,0.515,0.63,0.481,0.622,0.562,0.668,0.503,0.614,0.506,0.592,0.52, 0.598,0.584 , 0.315,0.611,0.533,0.591,0.672,0.502,0.313,0.536,0.627,0.564,0.541,0.518,0.364,0.685,0.605,0.459,0 . 44,0.544,0.501,0.574,0.508,0.504,0.662,0.483,0.557,0.678,0.491,0.619,0.723,0.566,0.626,0.404,0.49 9,0.48,0.577,0.732,0.613,0.641,0.454,0.49,0.421,0.357,0.603,0.487,0.555,0.58,0.479,0.672,0.53,0.54 4,0.538,0.668,0.701,0.603,0.635,0.501,0.637,0.48,0.653,0.625,0.489,0.668,0.73,0.669,0.622],[0.361, 0.177,0.181,0.208,0.391,0.475,0.334,0.328,0.528,0.545,0.54,0.752,0.489,0.292,0.44,0.473,0.582,0.49 9,0.537,0.387,0.27,0.433,0,295,0.323,0.271,0.316,0.264,0.486,0.444,0.252,0.233,0.358,0.462,0.398,0 . 319,0.506,0.357,0.395,0.525,0.247,0.4199,0.591,0.356,0.272,0.502,0.526,0.527,0.196,0.666,0.198,0.5 71,0.358,0.527,0.389,0.622,0.336,0.392,0.534,0.766,0.24,0.556,0.604,0.593,0.58,0.716,0.541,0.604,0 . 54,0.694,0.611,0.63,0.587,0.542,0.67,0.798,0.67,0.498,0.671,0.641,0.595,0.587,0.56,0.632,0.573,0.3 71,0.639,0.261,0.49,0.801,0.514,0.654,0.626,0.437,0.653,0.763,0.667,0.665,0.471,0.405,0.42,0.3,0.6 17,0.654,0.422,0.418,0.499,0.646,0.43,0.738,0.441,0.714,0.688,0.275,0.666,0.517,0.559,0.495,0.695, 0.777,0.639,0.625,0.516,0.621,0.484,0.584,0.629,0.519,0.622,0.672,0.757.0.548]] |

47

| | χ | N | α | ν |
|---|---|---|---|---|
| M2+M3 | [[M_{a1}]. [M_{a2}]] | 132 | [-0.689,-0.689;0.689,-0.689,-0.689,-0.689,-0.689,-0.689,-0.689,-0.689,-0.689,-0,689,-0.689.-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689.0.311,0.311,0.311,0.311,0.311,0.311,0.311,0.311,0.311, 0.311,0.311,0.311,0.311,0.311,0.311,0.311,0.311,0.311 ,0.311, 0.311,0.311,0.311,0.311,0.311,0.311,0.311,0.311,0.311 ,0.311, 0.311,0.311,0.311,0.311,0.311,0.311,0.311,0.311,0.311 ,0.311, 0.311,0.311,0.311,0.311,0.311,0.311,0.311,0.311,0.311 ,0.311, 0.311,0.311,0.311,0.311,0.311,0.311,0.311,0.311,0.311 ,0.311, 0.311,0.311,0.311,0.311,0.311,0.311,0.311,0.311,0.311 ,0.311, 0.311,0.311,0.311.0.311,0.311,0.311,0.311,0.311,0.311 ,0.311, 0.311,0.311]  | [ [0.445,0.4,0.323,0.375,0.364,0.531,0.417,0.353,0.397,0.361,0.484,0.446,0.393,0.406,0.408,0.37,0.346,0.509,0.632,0.382,0.326,0.382,0.341,0.375,0.412,0.352,0.385,0.438,0.336,0.373,0.37,0.437,0.406,0.361,0.494,0.412,0.344,0.368,0.389,0.442,0.45,0.441,0.489,0.408,0.482,0.548,0.532,0.361,0.484,0.421,0.413,0.403,0.42,0.444,0.459,0.358,0.364,0.397,0.528,0.387,0.385,0.499,0.355,0.344,0.399,0.569,0.727,0.42,0.539,0.626,0.634,0.542,0.632,0.427,0.431,0.504,0.413,0.564,0.736,0.562,0.414,0.68,0.635,0.506,0.6,0.499,0.416,0.842,0.509,0.752,0.491,0.49,0.688,0.414,0.679,0.639,0.694,0.514,0.445,0.41,0.601,0.353,0.462,0.572,0.327,0.647,0.409,0.587,0.506,0.638,0.598,0.554,0.762,0.454,0.523,0.758,0.412,0.437,0.788,0.736,0.45,0.589,0.547,0.46,0.509,0.587,0.478,0.484,0.599,0.459,0.502,0.466],[0.403,0.575,0.35,0.335,0.336,0.349,0.366,0.349,0.343,0.363,0.379,0.393,0.372,0.687,0.497,0.441,0,389,0.488,0.488,0.396,0.355,0.347,0.342,0.37,0.489,0.405,0.386,0.373,0.386,0.402,0.365,0.377,0.432,0.328,0.447,0.519,0.418,0.371,0.544,0.399,0.404,0.652,0.558,0.32,0.412,0.634,0.559,0.555,0.534,0.509,0.506,0.507,0.391,0.395,0.609,0.379,0.474,0.442.0.371,0.418,0.423,0.417,0.361,0.357,0.391,0.52,0.731,0.522,0.634,0.681,0.635,0.656,0.679,0.534,0.574,0.526,0.55,0.5,0.493,0.562,0.482,0.618,0.504,0,529,0.679,0.512,0.454,0.395,0.587,0.471,0.502,0.404,0.53,0.551,0.554,0.424,0.686,0.421,0.584,0.498,0.567,0.494,0.465,0.504,0.43,0.71,0.508,0.58,0.738,0.471,0.615,0,431,0.531,0.622,0.519,0.605,0.556,0.546,0.547,0.519,0.489,0.48,0.698,0.469,0.512,0.572,0.4,0.509,0.521,0.457,0.587,0.493]] |

(continued)

| | $\chi$ | N | $\alpha$ | $\nu$ |
|---|---|---|---|---|
| M2+M4 | [[M_{a1}], [M_{a2}]] | 131 | [-0.689.-0.689,-0.333,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,-0.689,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.265,0.311,0.311,0.311,0.311,0.311,0.311,0.311,0.311,0.311,0.311,0.311,0.311,0.311,0.311,0.311,0.311,0.311,0.311.0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311,0.311, 0.311] | [[0.445,0.4,0.323,0.375,0.364,0.531,0.417,0.353,0.397,0.361,0.484,0.446,0.393,0.406.0.408,0.37,0.346,0.509,0.632,0.382,0.326,0.382,0.341,0.375,0.412,0.352,0.385,0.438,0.336,0.373,0.37,0.437,0.406,0.361,0.494,0.412,0.344,0.368,0.389,0.442,0.45,0.441,0.489,0.408,0.482,0.548,0.532,0.361,0.484,0.421,0.413,0.403,0.42,0.444,0.459,0.358,0.364,0.397,0.528,0.387,0.385,0.499,0.355,0.344,0.399,0.569,0.727,0.42,0.539,0.626,0.634,0.542,0.632,0.427,0.431,0.504,0.413,0.564,0.736,0.562,0.414,0.68,0.635,0.506,0.6,0.499,0.416,0.509,0.752,0.491,0.49,0.688,0.414,0.679,0.639,0.694,0.514,0.445,0.41,0.601,0.353,0.462,0.572,0.327,0.647,0.409,0.587,0.506,0.638,0.598,0.554,0.762,0.454,0.523,0.758,0.412,0.437,0.788,0.736,0.45,0.589,0.547,0.46,0.509,0.587,0.478,0.484,0.599,0.459,0.502,0.466],[0.361,0.177,0.181,0.208,0.391,0.475,0.334,0.328,0.528,0.545,0.54,0.752,0.489,0.292,0.44,0.473,0.582,0.499,0.537,0.367,0.27,0.433,0.295,0.323,0.271,0.316,0.264,0.486,0.444,0.252,0.233,0.358,0.462,0.398,0.319,0.506,0.357,0.395,0.525,0.247,0.419,0.591,0.356,0.272,0.502,0.526,0.527,0.196,0.666,0.198,0.571,0.358,0.527,0.389,0.622,0.336,0.392,0.534,0.766,0.24,0.556,0.604,0.593,0.58,0.716,0.541,0.604,0.54,0.694,0.611,0.63,0.587,0.542,0.67,0.798,0.67,0.498,0.671,0.641,0.595,0.587,0.56,0.632,0.573,0.371,0.639,0.261,0.49,0.801,0.514,0.654,0.626,0.437,0.653,0.763,0.667,0.665,0.471,0.405,0.42,0.3,0.617,0.654,0.422,0.418,0.499,0.646,0.43,0.738,0.441,0.714,0.688,0.275,0.666,0.517,0.559,0.495,0.695,0.717,0.639,0.625,0.516,0.621,0.484,0.584,0.629,0.519,0.622,0.672,0.757,0.548]] |

49

(continued)

| | $\chi$ | N | $\alpha$ | $\nu$ |
|---|---|---|---|---|
| M3+M4 | [[M_{a1}], [M_{a2}]] | 132 | [-0,689,-0.689,-0,689.-0.689.-0.689,-0.689,-0.689,-0.689.-0.689,-0.689,-0.689,-0.689,-0.689,-0.689,-0.689,-0.68 9,-0.689,-0.689,-0.689,-0.689,-0.689,-0.689,-0.689,-0.689,-0.689,-0.689,-0.689,--0.689,-0.689,-0.689,-0.689,-0.689,-0.689,-689,-0.689,-0.689,0.311,0.311,0.311,0.311,0.311,0.311,0.311,0.311,0.311, 0.311,0.311,0.311,0.311,0.311,0.311,0.311,0.311,0.311,0.311, 0.311,0.311,0.311,0.311,0.311,0.311,0.311,0.311,0.311, 0.311, 0.311,0.311,0.311,0.311,0.311,0.311,0.311, 0.311,0.311,0.311, 0.311,0.311,0.311,0.311,0.311,0.311,0.311,0.311,0.311, 0.311, 0.311,0.311,0.311,0.311,0.311,0.311,0.311,0.311,0.311, 0.311, 0.311,0.311,0.311,0.311,0.311,0.311,0.311,0.311,0.311, 0.311, 0.311,0.311,0.311,0.311,0.311,0.311,0.311,0.311,0.311, 0.311] 0.311,0.311] | [ [0.403,0.575,0.35,0.335,0.336,0.349,0.366,0.349,0.343,0.363,0.379,0.393,0.372,0.687,0.4 97,0,441,0 . 389,0.488,0.488,0.396,0.355,0.347,0.342,0.37,0.489,0.405,0.386,0.373,0.386,0.402,0.365 ,0.377,0.43 2,0.328,0.447,0.519,0.418,0.371,0.544,0.399,0.404,0.652,0.558,0.32,0.412,0.634,0.559,0. 555,0.534,0 . 509,0.506,0.507,0.391,0.395,0.609,0.379,0.474,0.442,0.371,0.418,0.423,0.417,0.361,0.35 7,0.391,0.5 2,0.731,0.522,0.634,0.681,0.635,0.656,0.679,0.534,0.574,0.526,0.55,0.5,0.493,0.562,0.48 2,0.618,0.5 04,0.529,0.679,0.512,0.454,0.395,0.587,0.471,0.502,0.404,0.53,0.551,0.554,0.424,0.686, 0.421,0.584, 0.498,0.567,0.494,0.465,0.504,0.43,0.71,0.508,0.58,0.738,0.471,0.615,0.431,0.531,0.622, 0.519,0.605 , 0.556,0.546,0.547,0.519,0.489,0.48,0.698,0.469,0.512,0.572,0.4.0.509,0.521,0.457,0.587, 0.493],[0.3 61,0.177,0.181,0.208,0.391,0.475,0.334,0.328,0.528,0.545,0.54,0.752,0.489,0.292,0.44,0. 473,0.582,0 . 499,0.537,0.367,0.27,0.433,0.295,0.323,0.271,0.316,0.264,0.486,0.444,0.252,0.233,0.358 ,0.462,0.39 8,0.319,0.506,0.357,0.395,0.525,0.247,0.419,0.591,0.356,0.272,0.502,0.526,0.527,0.196, 0.666,0.198, 0.571,0.358,0.527,0.389,0.622,0.336,0.392,0.534,0.766,0.24,0.556,0.604,0.593,0.58,0.71 6,0.541,0.60 4,0.54,0.694,0.611,0.63,0.587,0.542,0.67,0.798,0.67,0.498,0.671,0.641,0.595,0.587,0.56, 0.632,0.573, 0.371,0.639,0.261,0.825,0.49,0.801,0.514,0.654,0.626,0.437,0.653,0.763,0.667,0.665,0.4 71,0.405,0.4 2,0.3,0.617,0.654,0.422,0.418.0.499,0646,0.43.0.738.0.441,0.714,0.688,0.275,0.666,0.51 7,0.559,0.4 95,0.695,0.777,0.639,0.625,0.516,0.621,0.484,0.584,0.629,0.519,0.622,0.672,0.757,0.548 ]] |

$M_{a1}$ and $M_{a2}$ in the above Table 25 respectively denote the normalized score of one of the above two groups and the normalized score of the other ("first normalized score" and "second normalized score"). The values shown in the following Table 26 were used for $M_{a1}$ and $M_{a2}$.

[Table 26]

|  | $M_{a1}$ | $M_{a2}$ |
|---|---|---|
| M1 + M2 | Normalized score corresponding to M1 | Normalized score corresponding to M2 |
| M1 + M3 | Normalized score corresponding to M1 | Normalized score corresponding to M3 |
| M1 + M4 | Normalized score corresponding to M1 | Normalized score corresponding to M4 |
| M2 + M3 | Normalized score corresponding to M2 | Normalized score corresponding to M3 |
| M2 + M4 | Normalized score corresponding to M2 | Normalized score corresponding to M4 |
| M3 + M4 | Normalized score corresponding to M3 | Normalized score corresponding to M4 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 27. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 27]

| NAFL to NASH Discrimination method | AUROC | Reference value | Sensitivity | Specificity |
|---|---|---|---|---|
| M1 (All factors) + M2 (All factors) | 0.904 | 2.773 | 0.901 | 0.780 |
| M1 (All factors) + M3 (All factors) | 0.940 | 3.153 | 0.835 | 0.951 |
| M1 (All factors) + M4 (All factors) | 0.857 | 3.857 | 0.747 | 0.829 |
| M2 (All factors) + M3 (All factors) | 0.866 | 2.570 | 0.791 | 0.829 |
| M2 (All factors) + M4 (All factors) | 0.847 | 3.228 | 0.791 | 0.854 |
| M3 (All factors) + M4 (All factors) | 0.884 | 3.389 | 0.824 | 0.854 |

Further, when two normalized scores corresponding to the above modules were used, index values based on the nonlinear logistic regression equation described above were also determined and found as follows.

For the preferable index value, the value calculated from the following formula 11 is used. In the formula 11, I denotes the index value, $M_{a1}$ and $M_{a2}$ respectively denote the normalized score of one of the above two groups and the normalized score of the other ("first normalized score" and "second normalized score"), $e_1$, $e_2$ and $e_3$ denote the constants determined by the non-linear regression analysis.

$$I = e_1 \times M_{a1} + e_2 \times M_{a2} + e_3 \times M_{a1} \times M_{a2} \qquad (\text{formula } 11)$$

The values shown in the following Table 28 were used herein for $M_{a1}$ (first normalized score), $M_{a2}$ (second normalized score), $e_1$, $e_2$ and $e_3$ in the formula 11.

[Table 28]

|  | $M_{a1}$ | $M_{a2}$ | e1 | e2 | e3 |
|---|---|---|---|---|---|
| M1 + M2 | Normalized score corresponding to M1 | Normalized score corresponding to M2 | 10.998 | 11.845 | 7.327 |
| M1 + M3 | Normalized score corresponding to M1 | Normalized score corresponding to M3 | -8.17 | -6.533 | 59.8 |

(continued)

|  | M$_{a1}$ | M$_{a2}$ | e1 | e2 | e3 |
|---|---|---|---|---|---|
| M1 + M4 | Normalized score corresponding to M1 | Normalized score corresponding to M4 | -1.668 | -4.391 | 23.207 |
| M2 + M3 | Normalized score corresponding to M2 | Normalized score corresponding to M3 | -10.043 | -8.342 | 46.243 |
| M2 + M4 | Normalized score corresponding to M2 | Normalized score corresponding to M4 | 1.54 | -2.617 | 19.485 |
| M3 + M4 | Normalized score corresponding to M3 | Normalized score corresponding to M4 | 1.965 | -5.435 | 30.026 |

In all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 29. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 29]

| NAFL to NASH Discrimination method | AUROC | Reference value | Sensitivity | Specificity |
|---|---|---|---|---|
| M1 (All factors) + M2 (All factors) | 0.904 | 11.762 | 0.890 | 0.780 |
| M1 (All factors) + M3 (All factors) | 0.939 | 6.371 | 0.835 | 0.902 |
| M1 (All factors) + M4 (All factors) | 0.859 | 2.721 | 0.714 | 0.854 |
| M2 (All factors) + M3 (All factors) | 0.872 | 0.581 | 0.857 | 0.780 |
| M2 (All factors) + M4 (All factors) | 0.850 | 3.563 | 0.758 | 0.878 |
| M3 (All factors) + M4 (All factors) | 0.902 | 4.698 | 0.835 | 0.829 |

Test Example 2: Method for discriminating between NAFL and NASH (2)

Measurement method 1

Normalized scores were determined by the same method as in the above Test Example 1 except that 1 to 3 kinds of the biomolecules shown in the following Table 30 were used for determining the normalized scores.

[Table 30]

|  | Biomolecule |
|---|---|
| M1 (PROS1, CLU) | PROS1, CLU |
| M2 (VCAM1) | VCAM1 |
| M2 (VCAM1, HA) | VCAM1, HA |
| M2 (VCAM1, CTSD) | VCAM1, CTSD |
| M2 (VCAM1, COL4) | VCAM1, COL4 |
| M2 (VCAM1, COL4-7S) | VCAM1, COL4-7S |
| M2 (HA, COL4-7S) | HA, COL4-7S |
| M2 (HA, COL4) | HA, COL4 |
| M2 (HA, CTSD) | HA, CTSD |

(continued)

| | Biomolecule |
|---|---|
| M2 (CTSD, COL4) | CTSD, COL4 |
| M2 (CTSD, COL4-7S) | CTSD, COL4-7S |
| M3 (AST, PSAT) | AST, PSAT |
| M3 (AST, LEP) | AST, LEP |
| M3 (AST, ICAM1) | AST, ICAM1 |
| M3 (AST, PSAT, LEP) | AST, PSAT, LEP |
| M3 (AST, PSAT, ICAM1) | AST, PSAT, ICAM1 |
| M3 (AST, LEP, CK-18) | AST, LEP, CK-18 |
| M3 (AST, ICAM1, GSTA1) | AST, ICAM1, GSTA1 |
| M3 (AST, PSAT, 6Ckine) | AST, PSAT, 6Ckine |

With the normalized score of the module 1 (M1) and the normalized score of the module 3 (M3) being respectively defined as the "first normalized score" and "second normalized score", the index values were determined for all the combinations of M1 and M3 shown in above Table 30 based on the above formula 2 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 31. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 31]

| NAFL to NASH Discrimination method | AUROC | Reference value | Sensitivity | Specificity |
|---|---|---|---|---|
| M1(PROS1,CLU)+M3(AST,PSAT) | 0.900 | 18.17 | 0.747 | 0.927 |
| M1(PROS1,CLU)+M3(AST,LEP) | 0.889 | 18.635 | 0.758 | 0.902 |
| M1(PROS1,CLU)+M3(AST,ICAM1) | 0.904 | 18.434 | 0.769 | 0.951 |
| M1(PROS1,CLU)+M3(AST,PSAT,LEP) | 0.904 | 18.528 | 0.769 | 0.927 |
| M1(PROS1,CLU)+M3(AST,PSAT,ICAM1) | 0.906 | 17.905 | 0.791 | 0.902 |
| M1(PROS1,CLU)+M3(AST,LEP,CK18) | 0.906 | 18.319 | 0.802 | 0.902 |
| M1(PROS1,CLU)+M3(AST,ICAM1,GSTA1) | 0.902 | 17.643 | 0.813 | 0.878 |
| M1(PROS1,CLU)+M3(AST,PSAT,6Ckine) | 0.915 | 15.867 | 0.934 | 0.756 |

With the normalized score of the module 1 (M1) and the normalized score of the module 3 (M3) being respectively defined as the "first normalized score" and "second normalized score", the index values based on the above Decision tree 2 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 32. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 32]

| NAFL to NASH Discrimination method | AUROC | Reference value | Sensitivity | Specificity |
|---|---|---|---|---|
| M1(PROS1,CLU)+M3(AST,PSAT) | 0.787 | 0 | 0.714 | 0.756 |
| M1(PROS1,CLU)+M3(AST,LEP) | 0.812 | 1 | 0.527 | 0.976 |
| M1(PROS1,CLU)+M3(AST,ICAM1) | 0.799 | 0 | 0.736 | 0.756 |
| M1(PROS1,CLU)+M3(AST,PSAT,LEP) | 0.789 | 1 | 0.516 | 0.951 |
| M1(PROS1,CLU)+M3(AST,PSAT,ICAM1) | 0.757 | 0 | 0.692 | 0.732 |
| M1(PROS1,CLU)+M3(AST,LEP,CK18) | 0.816 | 0 | 0.791 | 0.732 |
| M1(PROS1,CLU)+M3(AST,ICAM1,GSTA1) | 0.757 | 1 | 0.473 | 0.951 |
| M1(PROS1,CLU)+M3(AST,PSAT,6Ckine) | 0.819 | 0 | 0.802 | 0.756 |

With the normalized score of the module 1 (M1) and the normalized score of the module 3 (M3) being respectively defined as the "first normalized score" and "second normalized score", the index values based on the above formula 3 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 33. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 33]

| NAFL to NASH Discrimination method | AUROC | Reference value | Sensitivity | Specificity |
|---|---|---|---|---|
| M1(PROS1,CLU)+M3(AST,PSAT) | 0.901 | 0.479 | 0.736 | 0.927 |
| M1(PROS1,CLU)+M3(AST,LEP) | 0.889 | 0.49 | 0.758 | 0.902 |
| M1(PROS1,CLU)+M3(AST,ICAM1) | 0.903 | 0.482 | 0.769 | 0.951 |
| M1(PROS1,CLU)+M3(AST,PSAT,LEP) | 0.905 | 0.486 | 0.780 | 0.927 |
| M1(PROS1,CLU)+M3(AST,PSAT,ICAM1) | 0.906 | 0.478 | 0.769 | 0.927 |
| M1(PROS1,CLU)+M3(AST,LEP,CK18) | 0.906 | 0.48 | 0.791 | 0.902 |
| M1(PROS1,CLU)+M3(AST,ICAM1,GSTA1) | 0.901 | 0.469 | 0.802 | 0.878 |
| M1(PROS1,CLU)+M3(AST,PSAT,6Ckine) | 0.914 | 0.416 | 0.934 | 0.756 |

With the normalized score of the module 1 (M1) and the normalized score of the module 3 (M3) being respectively defined as the "first normalized score" and "second normalized score", the index values based on the above formula 4 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 34. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 34]

| NAFL to NASH Discrimination method | AUROC | Reference value | Sensitivity | Specificity |
|---|---|---|---|---|
| M1(PROS1,CLU)+M3(AST,PSAT) | 0.899 | 2.131 | 0.747 | 0.927 |

(continued)

| NAFL to NASH Discrimination method | AUROC | Reference value | Sensitivity | Specificity |
|---|---|---|---|---|
| M1(PROS1,CLU)+M3(AST,LEP) | 0.890 | 2.189 | 0.758 | 0.902 |
| M1(PROS1,CLU)+M3(AST,ICAM1) | 0.905 | 2.158 | 0.791 | 0.927 |
| M1(PROS1,CLU)+M3(AST,PSAT,LEP) | 0.902 | 2.191 | 0.758 | 0.927 |
| M1(PROS1,CLU)+M3(AST,PSAT,ICAM1) | 0.904 | 2.119 | 0.791 | 0.902 |
| M1(PROS1,CLU)+M3(AST,LEP,CK18) | 0.906 | 2.184 | 0.780 | 0.927 |
| M1(PROS1,CLU)+M3(AST,ICAM1,GSTA1) | 0.901 | 2.076 | 0.813 | 0.878 |
| M1(PROS1,CLU)+M3(AST,PSAT,6Ckine) | 0.917 | 1.934 | 0.901 | 0.805 |

With the normalized score of the module 1 (M1) and the normalized score of the module 3 (M3) being respectively defined as the "first normalized score" and "second normalized score", the index values based on the above formula 5 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 35. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 35]

| NAFL to NASH Discrimination method | AUROC | Reference value | Sensitivity | Specificity |
|---|---|---|---|---|
| M1(PROS1,CLU)+M3(AST,PSAT) | 0.900 | 8.236 | 0.769 | 0.902 |
| M1(PROS1,CLU)+M3(AST,LEP) | 0.889 | 8.618 | 0.769 | 0.902 |
| M1(PROS1,CLU)+M3(AST,ICAM1) | 0.905 | 8.605 | 0.758 | 0.951 |
| M1(PROS1,CLU)+M3(AST,PSAT,LEP) | 0.904 | 8.609 | 0.769 | 0.927 |
| M1(PROS1,CLU)+M3(AST,PSAT,ICAM1) | 0.907 | 8.342 | 0.802 | 0.902 |
| M1(PROS1,CLU)+M3(AST,LEP,CK18) | 0.906 | 8.546 | 0.802 | 0.902 |
| M1(PROS1,CLU)+M3(AST,ICAM1,GSTA1) | 0.903 | 8.209 | 0.813 | 0.878 |
| M1(PROS1,CLU)+M3(AST,PSAT,6Ckine) | 0.916 | 7.814 | 0.857 | 0.829 |

With the normalized score of the module 1 (M1) and the normalized score of the module 3 (M3) being respectively defined as the "first normalized score" and "second normalized score", the index values based on the above formula 6 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 36. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 36]

| NAFL to NASH Discrimination method | AUROC | Reference value | Sensitivity | Specificity |
|---|---|---|---|---|
| M1(PROS1,CLU)+M3(AST,PSAT) | 0.910 | 0.436 | 0.791 | 0,878 |
| M1(PROS1,CLU/+M3(AST,LEP) | 0.895 | 0.457 | 0.758 | 0.902 |

(continued)

| NAFL to NASH Discrimination method | AUROC | Reference value | Sensitivity | Specificity |
|---|---|---|---|---|
| M1(PROS1,CLU)+M3(AST,ICAM1) | 0.907 | 0.428 | 0.868 | 0.805 |
| M1(PROS1,CLU)+M3(AST,PSAT,LEP) | 0.907 | 0.486 | 0.681 | 0.976 |
| M1(PROS1,CLU)+M3(AST,PSAT,ICAM1) | 0.912 | 0.437 | 0.835 | 0.829 |
| M1(PROS1,CLU)+M3(AST,LEP,CK18) | 0.904 | 0.467 | 0.747 | 0,951 |
| M1(PROS1,CLU)+M3(AST,ICAM1,GSTA1) | 0.906 | 0.479 | 0.692 | 0.976 |
| M1(PROS1,CLU)+M3(AST,PSAT,6Ckine) | 0.913 | 0.443 | 0.813 | 0.878 |

With the normalized score of the module 1 (M1) and the normalized score of the module 3 (M3) being respectively defined as the "first normalized score" and "second normalized score", the index values based on the above formula 7 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 37. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 37]

| NAFL to NASH Discrimination method | AUROC | Reference value | Sensitivity | Specificity |
|---|---|---|---|---|
| M1(PROS1,CLU)+M3(AST,PSAT) | 0.911 | 1.52 | 0.747 | 0.951 |
| M1(PROS1,CLU)+M3(AST,LEP) | 0.895 | 1.567 | 0.758 | 0.927 |
| M1(PROS1,CLU)+M3(AST,ICAM1) | 0.907 | 1.487 | 0.802 | 0.878 |
| M1(PROS1,CLU)+M3(AST,PSAT,LEP) | 0.908 | 1.604 | 0.736 | 0.951 |
| M1(PROS1,CLU)+M3(AST,PSAT,ICAM1) | 0.914 | 1.459 | 0.813 | 0.878 |
| M1(PROS1,CLU)+M3(AST,LEP,CK18) | 0.910 | 1.676 | 0.714 | 0.976 |
| M1(PROS1,CLU)+M3(AST,ICAM1,GSTA1) | 0.909 | 1.467 | 0.813 | 0.878 |
| M1(PROS1,CLU)+M3(AST,PSAT,6Ckine) | 0.921 | 1.308 | 0.923 | 0.829 |

With the normalized score of the module 1 (M1) and the normalized score of the module 3 (M3) being respectively defined as the "first normalized score" and "second normalized score", the index values based on the above formula 8 and formula 9 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 38. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 38]

| NAFL to NASH Discrimination method | AUROC | Reference value | Sensitivity | Specificity |
|---|---|---|---|---|
| M1(PROS1,CLU)+M3(AST,PSAT) | 0.869 | 0.416 | 0.747 | 0.927 |
| M1(PROS1,CLU)+M3(AST,LEP) | 0.890 | 0.552 | 0.758 | 0.902 |
| M1(PROS1,CLU)+M3(AST,ICAM1) | 0.906 | 0.476 | 0.791 | 0.927 |

(continued)

| NAFL to NASH Discrimination method | AUROC | Reference value | Sensitivity | Specificity |
|---|---|---|---|---|
| M1(PROS1,CLU)+M3(AST,PSAT,LEP) | 0.902 | 0.556 | 0.758 | 0.927 |
| M1(PROS1,CLU)+M3(AST,PSAT,ICAM1) | 0.904 | 0.391 | 0.791 | 0.902 |
| M1(PROS1,CLU)+M3(AST,LEP,CK18) | 0.907 | 0.528 | 0.791 | 0.927 |
| M1(PROS1,CLU)+M3(AST,ICAM1,GSTA1) | 0.901 | 0.307 | 0.813 | 0.878 |
| M1(PROS1,CLU)+M3(AST,PSAT,6Ckine) | 0.917 | 0.139 | 0.901 | 0.805 |

With the normalized score of the module 1 (M1) and the normalized score of the module 3 (M3) being respectively defined as the "first normalized score" and "second normalized score", the index values based on the above formula 10 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 39. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 39]

| NAFL to NASH Discrimination method | AUROC | Reference value | Sensitivity | Specificity |
|---|---|---|---|---|
| M1(PROS1,CLU)+M3(AST,PSAT) | 0.901 | 3.082 | 0.736 | 0.927 |
| M1(PROS1,CLU)+M3(AST,LEP) | 0.890 | 3.295 | 0.736 | 0.902 |
| M1(PROS1,CLU)+M3(AST,ICAM1) | 0.903 | 3.125 | 0.769 | 0.951 |
| M1(PROS1,CLU)+M3(AST,PSAT,LEP) | 0.903 | 3.347 | 0.725 | 0.951 |
| M1(PROS1,CLU)+M3(AST,PSAT,ICAM1) | 0.904 | 2.994 | 0.780 | 0.927 |
| M1(PROS1,CLU)+M3(AST,LEP,CK18) | 0.906 | 3.216 | 0.769 | 0.927 |
| M1(PROS1,CLU)+M3(AST,ICAM1,GSTA1) | 0.898 | 3.132 | 0.747 | 0.927 |
| M1(PROS1,CLU)+M3(AST,PSAT,6Ckine) | 0.911 | 2.306 | 0.934 | 0.756 |

With the normalized score of the module 1 (M1) and the normalized score of the module 3 (M3) being respectively defined as the "first normalized score" and "second normalized score", the index values based on the above formula 11 were determined for all the combinations of M1 and M3 shown in the above Table 12 and it was found that in all the normalized scores and index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set to the values shown in the following Table 40. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 40]

| NAFL to NASH Discrimination method | AUROC | Reference value | Sensitivity | Specificity |
|---|---|---|---|---|
| M1(PROS1,CLU)+M3(AST,LEP) | 0.915 | 5.778 | 0.714 | 0.976 |
| M1(PROS1,CLU)+M3(AST,LEP) | 0.895 | 6.219 | 0.714 | 0.951 |
| M1(PROS1,CLU)+M3(AST,ICAM1) | 0.906 | 6.682 | 0.670 | 1.000 |
| M1(PROS1,CLU)+M3(AST,PSAT, LEP) | 0.908 | 6.343 | 0.714 | 0,951 |

(continued)

| NAFL to NASH Discrimination method | AUROC | Reference value | Sensitivity | Specificity |
|---|---|---|---|---|
| M1(PROS1,CLU)+M3(AST,PSAT,LEP) | 0.911 | 3.789 | 0.912 | 0.756 |
| M1(PROS1,CLU)+M3(AST,LEP,CK18) | 0.908 | 6.528 | 0.736 | 0.976 |
| M1(PROS1,CLU)+M3(AST,ICAM1,GSTA1) | 0.906 | 6.675 | 0.681 | 0.976 |
| M1(PROS1,CLU)+M3(AST,PSAT,6Ckine) | 0.915 | 4.424 | 0.846 | 0.902 |

Measurement method 2

Quantities of HA, VCAM1 and COL4-7S were respectively measured by the method described below to determine the normalized scores. The quantity of HA was measured by latex agglutination method using an in-vitro diagnostic reagent "LPIA ACE HA" (LSI Medience Corporation). The quantity of VCAM1 was measured by the ELISA method using "Human sVCAM-1/CD106 Quantikine ELISA Kit" (R&D Systems). Further, the quantity of COL4-7S was measured by the radioimmunoassay method using an in-vitro diagnostic reagent "Type IV Collagen·7S kit" (LSI Medience Corporation). From the measured quantity of each biomolecule, each normalized score was determined based on the above formula 1. For the mean ($m_i$), sd ($m_i$), mean ($m_i^{NASH}$) and mean ($m_i^{NAFL}$) in the formula 1, the values shown in the following Table 41 were used. Note that the values appeared in Table 41 are the values also used even in the case where a measurement method having the performance equal to the method using the above diagnostic reagent is used. For HA, serum samples obtained from 44 NAFL affected patients and 95 NASH affected patients were measured. For VCAM1, serum samples obtained from 68 NAFL affected patients and 127 NASH affected patients were measured. For COL4-7S, serum samples obtained from 62 NAFL affected patients and 130 NASH affected patients were measured. For each of the molecules, a simple average of the average value of the NAFL affected patients and the average value of the NASH affected patients is denoted as mean ($m_i$), and a simple average of the standard deviation of the NAFL affected patients and the standard deviation of the NASH affected patients is denoted as sd ($m_i$). Further, the mean ($m_i$), sd ($m_i$) in the case where HA was measured by the CLIA method using an in-vitro diagnostic reagent "Chemilumi hyaluronic acid" (SIEMENS Healthcare Diagnostics) were set using the values obtained by inserting the mean ($m_i$), sd ($m_i$) used in the latex agglutination method into the correlation equation ($y = 1.36 \, x - 10.3$) described in the attached document. y herein denotes the value of mean ($m_i$) or sd ($m_i$) in the case where "Chemilumi hyaluronic acid" is used and x denotes the value of mean ($m_i$) or sd ($m_i$) in the case where "LPIA ACE HA" is used.

[Table 41]

| Biomolecule of M2 | mean(mi) | sd(mi) | mean(mi$^{NASH}$) | mean(mi$^{NAFL}$) |
|---|---|---|---|---|
| VCAM1 | 843.8 | 331.9 | 710.9 | 976.6 |
| COL4-7S | 4.75 | 1.6 | 3.97 | 5.54 |
| HA (Latex agglutination method) | 56.37 | 62.52 | 29.91 | 82.82 |
| HA (Chemilumi hyaluronic acid) | 66.36 | 74.73 | 30.38 | 102.34 |

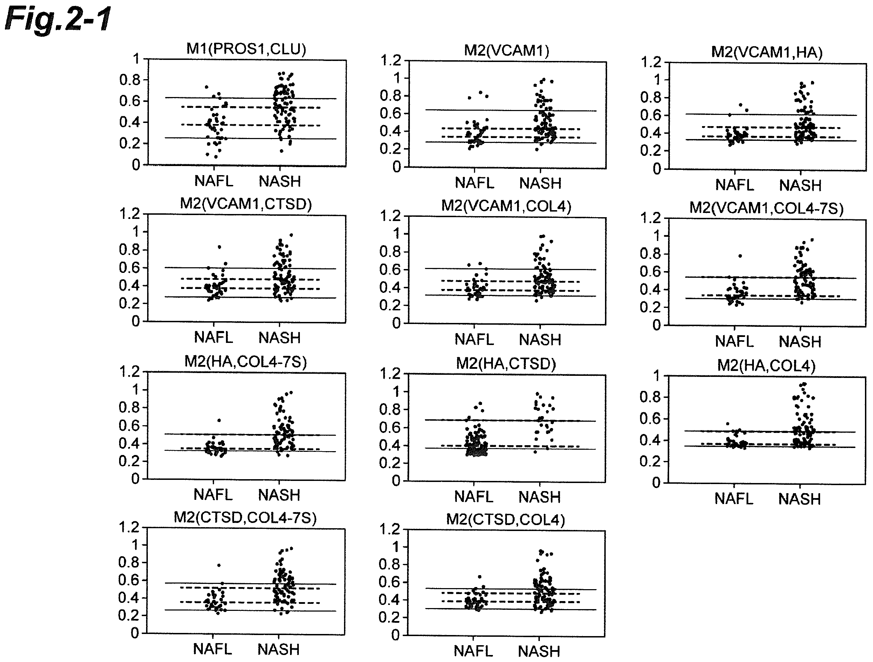

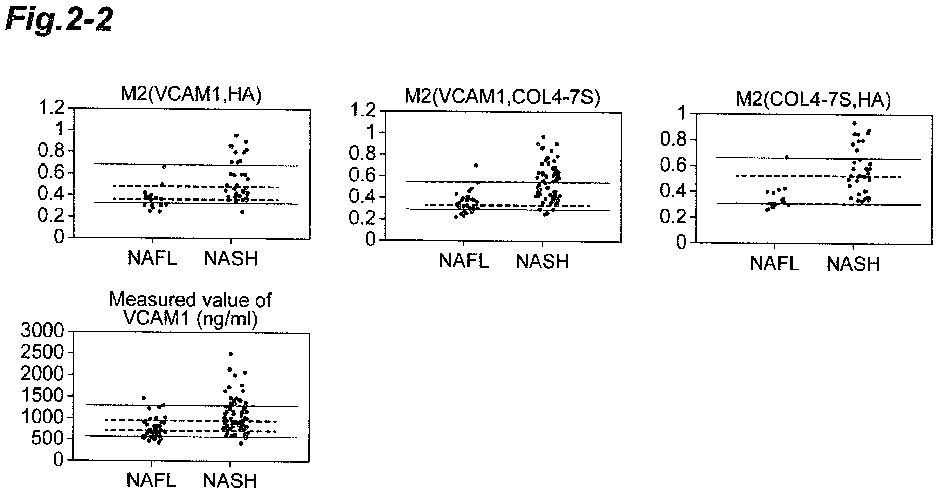

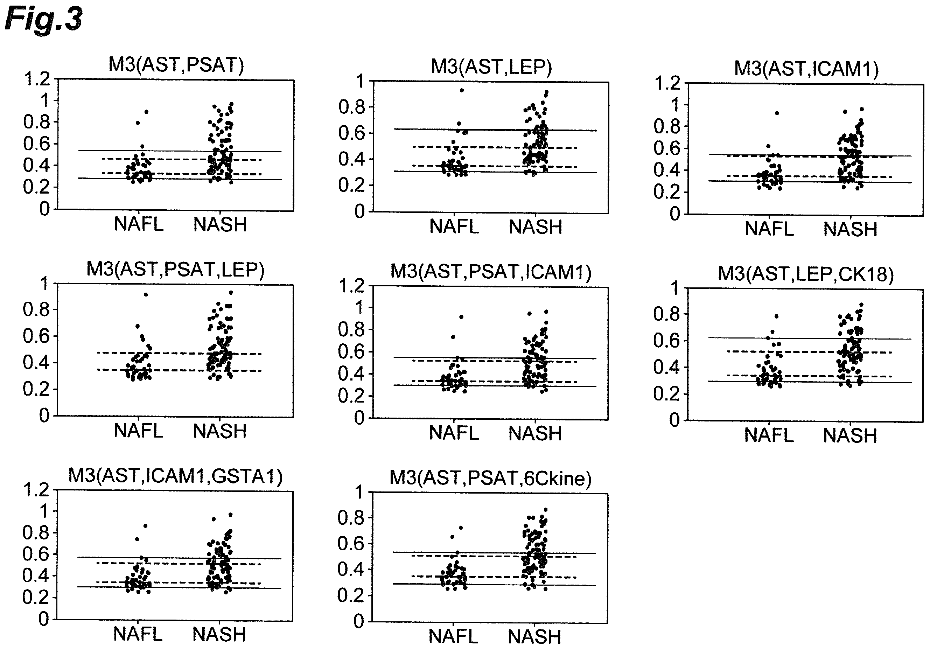

Graphs of the determined normalized scores classified into the NAFL patients group and the NASH patients group and plotted are shown in Fig. 2-1, Fig. 2-2 and Fig. 3. In Figs., the solid lines represent both ends of the "reference value range A" in Tables 42 and 43 and the dotted lines represent both ends of the "reference value range B" in Tables 42 and 43. In all the normalized scores, significant differences were found between the NAFL patients group and the NASH patients group, thereby revealing the effectiveness in the discrimination between NAFL and NASH. Further, it is revealed that the reference value (cutoff value) to discriminate the NASH affected is effective when set within the ranges shown in the following Tables 42 and 43. Additionally, when AUROC determined from these results was compared with AUROC (0.834) of the NAFIC score which is the conventional art, it was suggested that the diagnostic accuracy is equivalent to or more than the conventional art. Note that AUROC of the NAFIC score is the value calculated using the specimen groups of the present Example.

[Table 42]

| NAFL TO NASH DISCRIMINATION METHOD (Measurement method 1) | AUROC | Reference value range (satisfies at least A or B) | Reference value range A: Sensitivity 95% to Specificity 95% | Reference value: Sensitivity 95% | | | Reference value: Specificity 95% | | | Reference value range B: Specificity 50% or more and sensitivity 50% or more | Reference value; Specificity 50% | | | Reference value: Sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M1 (PROS1,CLU) | 0.789 | 0.256-0.634 | 0.256-0.634 | 0.256 | 95.6 | 29.3 | 0.634 | 36.3 | 95.1 | 0.378-0.549 | 0.378 | 84.6 | 51.2 | 0.549 | 50.5 | 85.4 |
| M2 (HA, COL4-7S) | 0.734 | 0.326-0.509 | 0.326-0.476 | 0.326 | 96.2 | 39.4 | 0.476 | 60.3 | 97.0 | 0.348-0.509 | 0.348 | 91.0 | 51.5 | 0.509 | 50.0 | 97.0 |
| M2 (VCAM1) | 0.791 | 0.28-0.644 | 0.28-0.644 | 0.280 | 95.6 | 24.4 | 0.644 | 11.0 | 95.1 | 0.339-0.433 | 0.339 | 81.3 | 51.2 | 0.433 | 50.5 | 82.9 |
| M2 (VCAM1, CTSD) | 0.731 | 0.277-0.604 | 0.277-0.604 | 0.277 | 95.6 | 12.2 | 0.604 | 31.9 | 95.1 | 0.377-0.481 | 0.377 | 78.0 | 51.2 | 0.481 | 50.5 | 82.9 |
| M2 (VCAM1, COL4) | 0.757 | 0.319-0.617 | 0.319-0.617 | 0.319 | 95.6 | 22.0 | 0.617 | 23.1 | 95.1 | 0.375-0.48 | 0.375 | 79.1 | 51.2 | 0.480 | 50.5 | 82.9 |
| M2 (VCAM1, COL4-7S) | 0.821 | 0.303-0.543 | 0.303-0.543 | 0.303 | 96.2 | 27.3 | 0.543 | 50.0 | 97.0 | 0.339-0.543 | 0.339 | 89.7 | 51.5 | 0.543 | 50.0 | 97.0 |
| M2 (VCAM1, HA) | 0.740 | 0.326-0.617 | 0.326-0.617 | 0.326 | 95.6 | 26.8 | 0.617 | 28.6 | 95.1 | 0.367-0.471 | 0.367 | 81.3 | 51.2 | 0.471 | 50.5 | 92.7 |
| M2 (HA, COL4) | 0.796 | 0.343-0.487 | 0.343-0.487 | 0.343 | 95.6 | 17.1 | 0.487 | 47.3 | 95.1 | 0.367-0.485 | 0.367 | 86.8 | 51.2 | 0.485 | 50.5 | 92.7 |
| M2 (HA, CTSD) | 0.874 | 0.305-0.545 | 0.305-0.545 | 0.305 | 95.6 | 9.8 | 0.545 | 38.5 | 95.1 | 0.363-0.492 | 0.363 | 79.1 | 51.2 | 0.492 | 50.5 | 87.8 |
| M2 (CTSD, COL4) | 0.708 | 0.307-0.536 | 0.307-0.536 | 0.307 | 95.6 | 4.9 | 0.536 | 40.7 | 95.1 | 0.392-0.487 | 0.392 | 72.5 | 51.2 | 0.487 | 50.5 | 87.8 |

| NAFL TO NASH DIS-CRIMINA-TION METH-OD (Measurement method 1) | AU-ROC | Refer-ence val-ue range (satisfies at least A or B) | Refer-ence val-ue range A: Sensi-tivity 95% to Specif-icity 95% | Reference value: Sensitivity 95% | | | Reference value: Specificity 95% | | | Refer-ence val-ue range B: Spe-cificity 50% or more and sensitivity 50% or more | Reference value; Specificity 50% | | | Reference value: Sensitivity 50% | | |
|---|---|---|---|---|---|---|---|---|---|---|---|---|---|---|---|---|
| | | | | Refer-ence value | Sensi-tivity (%) | Specific-ity (%) | Refer-ence value | Sensi-tivity (%) | Specific-ity (%) | | Refer-ence value | Sensi-tivity (%) | Specific-ity (%) | Refer-ence value | Sensi-tivity (%) | Specific-ity (%) |
| M2 (CTSD, COL4-7S) | 0.809 | 0.271-0.575 | 0.271-0.575 | 0.271 | 96.2 | 9.1 | 0.575 | 42.3 | 97.0 | 0.361-0.523 | 0.361 | 89.7 | 51.5 | 0.523 | 50.0 | 93.9 |

| NAFL TO NASH DIS-CRIMINA-TION METH-OD (Measure-ment method 2) | AU-ROC | Refer-ence val-ue range (satisfies at least A or B) | Refer-ence val-ue range A: Sensi-tivity 95% to Specif-icity 95% | Reference value: Sensitivity 95% | | | Reference value: Specificity 95% | | | Refer-ence val-ue range B: Spe-cificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Refer-ence value | Sensi-tivity (%) | Speci-ticity (%) | Refer-ence value | Sensi-tivity (%) | Specifi-city (%) | | Refer-ence value | Sensi-tivity | Specifi-city | Refer-ence value | Sensi-tivity (%) | Specifi-city (%) |
| M2 (VCAM1, HA) | 0.813 | 0.326-0.684 | 0.326-0.684 | 0.326 | 97.2 | 43.8 | 0.684 | 30.6 | 100.0 | 0.36-0.482 | 0.360 | 86.1 | 50.0 | 0.482 | 50.0 | 87.5 |
| M2 (VCAM1, COL4-7S) | 0.844 | 0.293-0.548 | 0.293-0.539 | 0.293 | 95.5 | 32.3 | 0.539 | 52.2 | 96.8 | 0.333-0.548 | 0.333 | 91.0 | 51.6 | 0.548 | 50.7 | 96.8 |
| M2 (HA, COL4-7S) | 0.884 | 0.308-0.662 | 0.331-0.662 | 0.331 | 97.2 | 62.5 | 0.662 | 25.0 | 100.0 | 0.308-0.526 | 0.308 | 100.0 | 50.0 | 0.526 | 50.0 | 93.8 |
| VCAM1 measured value (ng/ml) | 0.737 | 551.1-1280.1 | 551.1-1280.1 | 551.1 | 96.0 | 25.6 | 1280.1 | 22.7 | 97.4 | 693.5-925.3 | 693.5 | 82.7 | 51.3 | 925.3 | 50.7 | 82.1 |

[Table 43]

| NAFL TO NASH DIS-CRIMINA-TION METH-OD | AU-ROC | Refer-ence val-ue range (satis-fies at least A or B) | Refer-ence val-ue range A: Sensi-tivity 95% to Specifi-city 95% | Reference value: Sensitivi-ty 95% | | | Preference value: Specifi-city 95% | | | Refer-ence val-ue range B: Spe-cificity 50% or more and sensitivity 50% or more | Reference value: Specifici-ty 50% | | | Reference value: Sensitivi-ty 50% | | |
|---|---|---|---|---|---|---|---|---|---|---|---|---|---|---|---|---|
| | | | | Refer-ence value | Sensi-tivity (%) | Specifi-city (%) | Refer-ence value | Sensi-tivity (%) | Specifi-city (%) | | Refer-ence value | Sensi-tivity (%) | Specifi-city (%) | Refer-ence value | Sensi-tivity (%) | Specifi-city (%) |
| M3 (AST, PSAT) | 0.791 | 0.284-0.544 | 0.284-0.544 | 0.284 | 95.6 | 19.5 | 0.544 | 33.0 | 95.1 | 0.332-0.465 | 0.332 | 87.9 | 51.2 | 0.465 | 50.5 | 87.8 |
| M3 (AST, LEP) | 0.778 | 0.309-0.629 | 0.309-0.629 | 0.309 | 95.6 | 17.1 | 0.629 | 23.1 | 95.1 | 0.352-0.498 | 0.352 | 89.0 | 51.2 | 0.498 | 50.5 | 85.4 |
| M3 (AST, ICAM1) | 0.788 | 0.302-0.546 | 0.302-0.546 | 0.302 | 95.6 | 24.4 | 0.546 | 47.3 | 95.1 | 0.352-0.533 | 0.352 | 83.5 | 51.2 | 0.533 | 50.5 | 90.2 |
| M3 (AST, PSAT, LEP) | 0.778 | 0.303-0.619 | 0.303-0.619 | 0.303 | 95.6 | 19.5 | 0.619 | 26.4 | 95.1 | 0.349-0.476 | 0.349 | 91.2 | 51.2 | 0.476 | 50.5 | 85.4 |
| M3 (AST, PSAT, ICAM1) | 0.793 | 0.301-0.551 | 0.301-0.551 | 0.301 | 95.6 | 22.0 | 0.551 | 42.9 | 95.1 | 0.34-0.522 | 0.340 | 89.0 | 51.2 | 0.522 | 50.5 | 90.2 |
| M3 (AST, LEP, CK18) | 0.802 | 0.297-0.625 | 0.297-0.625 | 0.297 | 95.6 | 29.3 | 0.625 | 27.5 | 95.1 | 0.341-0.521 | 0.341 | 87.9 | 51.2 | 0.521 | 50.5 | 87.8 |
| M3 (AST, ICAM1, GSTA1) | 0.790 | 0.302-0.573 | 0.302-0.573 | 0.302 | 95.6 | 14.6 | 0.573 | 39.6 | 95.1 | 0.343-0.521 | 0.343 | 87.9 | 51.2 | 0.521 | 50.5 | 90.2 |
| M3 (AST, PSAT, 6Ckine) | 0.836 | 0.291-0.535 | 0.291-0.535 | 0.291 | 95.6 | 12.2 | 0.535 | 44.0 | 95.1 | 0.348-0.508 | 0.348 | 92.3 | 51.2 | 0.508 | 50.5 | 92.7 |

Test Example 3: Method for discriminating between NAFL and NASH (3)

Index values were determined by the same calculation method as the "M1 + M3" of Test Example 1 except that the normalized score corresponding to M1 (PROS1, CLU) of Test Example 2 was used as the first normalized score and the normalized score corresponding to M3 (AST, PSAT), M3 (AST, LEP), M3 (AST, ICAM1), M3 (AST, PSAT, LEP), M3(AST, PSAT, ICAM1), M3 (AST, LEP, CK-18), M3 (AST, ICAM1, GSTA1) or M3 (AST, PSAT, 6Ckine) of Test Example 2 was used as the second normalized score.

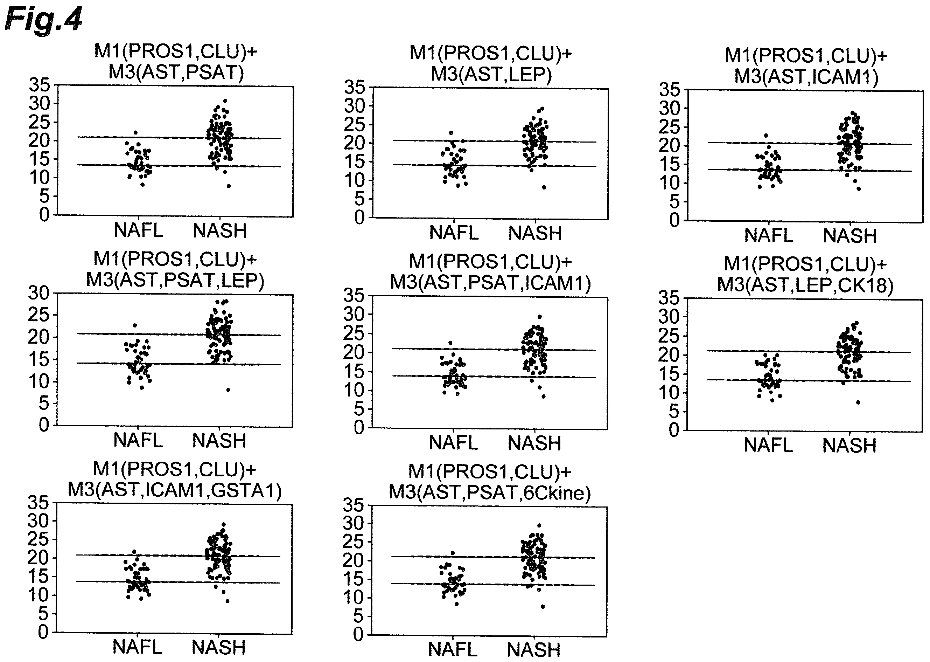

Graphs of the determined index values classified into the NAFL patients group and the NASH patients group and plotted are shown in Fig. 4. In Fig. 4, the solid lines represent both ends of the "reference value range A" in Table 43 and the dotted lines represent both ends of the "reference value range B" in Table 44. In all the index values, significant differences were found between the NAFL patients group and the NASH patients group, thereby revealing the effectiveness in the discrimination between NAFL and NASH. Further, it is revealed that the reference value (cutoff value) to discriminate the NASH affected is effective when set within the ranges shown in the following Table. Additionally, when AUROC determined from these results was compared with AUROC (0.834) of the NAFIC score which is the conventional art, it was suggested that the diagnostic accuracy excels the conventional art. Note that AUROC of the NAFIC score is the value calculated using the specimen groups of the present Example.

[Table 44]

| NAFL TO NASH DISCRIMINATION METHOD | AUROC | Reference value range (satisfies at least A or B) | Reference value range A: Sensitivity 95% to specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference : value | Sensitivity % | Specificity (%) | Reference value | Sensitivity | Specificity (%) | | Reference value | Sensitivity (%) | Specificity (%) | Reference value | Sensitivity (%) | Specificity (%) |
| M1(PROS1, CLU)+ M3 (AST,PSAT) | 0.900 | 13.493-21.02 | 14.641-19.125 | 14.64 1 | 95.6 | 58.5 | 19.12 5 | 69.2 | 95.1 | 13.493-21.02 | 13.49 3 | 96.7 | 51.2 | 21.02 0 | 50.5 | 97.6 |
| M1(PROS1, CLU)+ M3 (AST, LEP) | 0.889 | 14.228-20.755 | 14.814-20.289 | 14.81 4 | 95.6 | 56.1 | 20.28 9 | 56.0 | 95.1 | 14.228-20.755 | 14.22 8 | 96.7 | 51.2 | 20.75 5 | 50.5 | 97.6 |
| M1(PROS1, CLU) + M3 (AST, ICAM1) | 0.904 | 13.663-20.831 | 14.314-18.434 | 14.31 4 | 95.6 | 58.5 | 18.43 4 | 76.9 | 95.1 | 13.663-20.831 | 13.66 3 | 96.7 | 51.2 | 20.83 1 | 50.5 | 97.6 |
| M1(PROS1, CLU)+ + M3 (AST,PSAT, LEP) | 0.904 | 14.229-20.843 | 15.095-19.186 | 15.09 5 | 95.6 | 56.1 | 19.18 6 | 71.4 | 95.1 | 14.229-20.843 | 14.22 9 | 98.9 | 51.2 | 20.84 3 | 50.5 | 97.6 |
| M1(PROS1, CLU)+ M3 (AST, PSAT, ICAM1) | 0.906 | 13.929-21.053 | 14.945-18.959 | 14.94 5 | 95.6 | 61.0 | 18.95 9 | 70.3 | 95.1 | 13.929-21.053 | 13.92 9 | 96.7 | 51.2 | 21.05 3 | 50.5 | 97.6 |
| M1 (PROS1,CLU )+ M3(AST, LEP, CK18) | 0.906 | 13.544-21.168 | 14.694-19.163 | 14.69 4 | 95.6 | 58.5 | 19.16 3 | 73.6 | 95.1 | 13.544-21.168 | 13.54 4 | 97.8 | 51.2 | 21.16 8 | 50.5 | 100.0 |
| M1(PROS1, CLU)+ M3 (AST,ICAM1, GSTA1) | 0.902 | 13.788-20.882 | 14.903-19.109 | 14.90 3 | 95.6 | 61.0 | 19.10 9 | 71.4 | 95.1 | 13.788-20.882 | 13.78 8 | 96.7 | 51.2 | 20.88 2 | 50.5 | 97.6 |

| NAFL TO NASH DISCRIMINATION METHOD | AUROC | Reference value range (satisfies at least A or B) | Reference value range A: Sensitivity 95% to specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Referenc e : value | Sensitivity %) | Specificity (%) | Reference value | Sensitivity | Specificity (%) | | Reference value | Sensitivity (%) | Specificity (%) | Reference value | Sensitivity (%) | Specificity (%) |
| M1 (PROS1,CLU) + M3(AST, PSAT, 6Ckine) | 0.915 | 13.844-21.205 | 15.113-18.982 | 15.11 3 | 95.6 | 65.9 | 18.98 2 | 68.1 | 95.1 | 13.844-21.205 | 13.84 4 | 95.6 | 51.2 | 21.20 5 | 50.5 | 97.6 |

Test Example 4: Method for discriminating between symptoms of Type-1 to Type 3 and a symptom of Type-4 in the Matteoni classification

Normalized scores were determined by the same methods as Measurement method 1 or Measurement method 2 of the above Test Example 2 except that the biomolecules shown in the following Table 45 were used to determine the normalized scores.

[Table 45]

|  | Biomolecules |
|---|---|
| M2 (M2 All factors) | ALB, COL4-7S, AFP, AXL, CCL19, CGB, COL4, CSF1, CTSD, FAS, HA, Mac-2bp, CA19-9, NRCAM, OPG, VCAM1, VWF, YKL-40 |
| M2 (VCAM1) | VCAM1 |
| M2 (VCAM1, HA) | VCAM1, HA |
| M2 (VCAM1, CTSD) | VCAM1,CTSD |
| M2 (VCAM1, COL4) | VCAM1, COL4 |
| M2 (VCAM1, COL4-7S) | VCAM1, COL4-7S |
| M2 (HA, COL4-7S) | HA, COL4-7S |
| M2 (HA, COL4) | HA, COL4 |
| M2 (HA, CTSD) | HA, CTSD |
| M2 (CTSD, COL4) | CTSD, COL4 |
| M2 (CTSD, COL4-7S) | CTSD, COL4-7S |

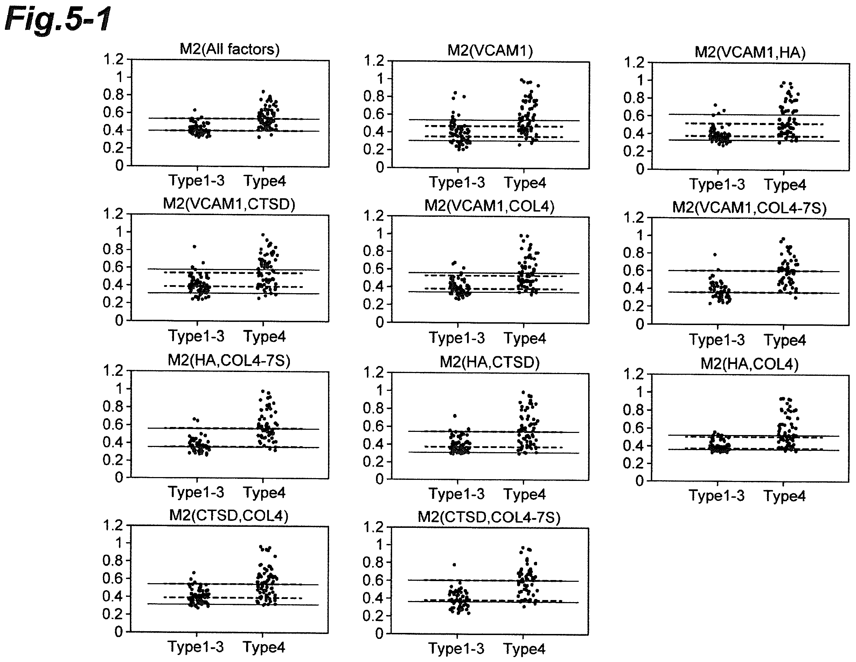

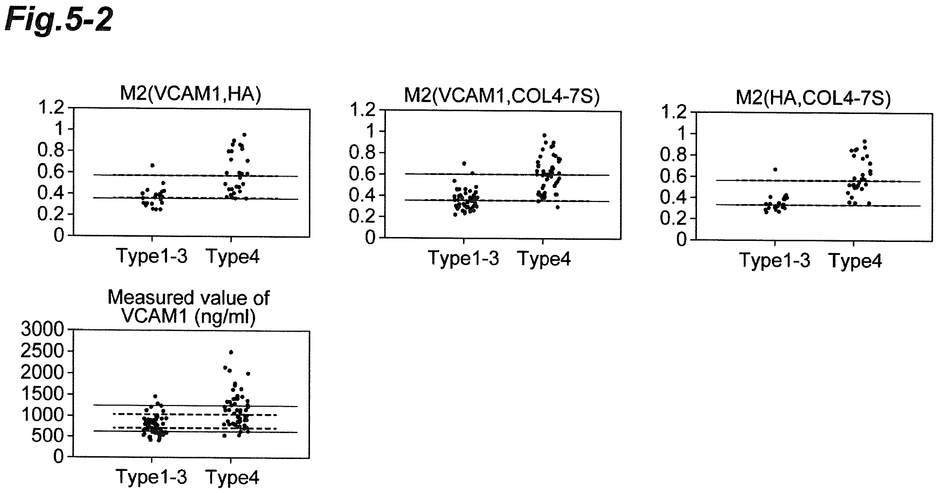

Graphs of the determined normalized scores classified into the patients group applicable to the symptoms of Type-1 to Type-3 (Type 1-3) and the patients group applicable to the symptom of Type-4 in the Matteoni classification and plotted are shown in Fig. 5-1 and Fig. 5-2. In Figs., the solid lines represent both ends of the "reference value range A" in Table 46 and the dotted lines represent both ends of the "reference value range B" in Table 46. In all the normalized scores, significant differences were found between the Type 1-3 patients group and the Type-4 patients group, thereby revealing the effectiveness in the discrimination between the Type 1-3 symptoms and the Type-4 symptom. Further, it is revealed that the reference value (cutoff value) to discriminate the applicability to the Type-4 symptom is effective when set within the ranges shown in the following Table 46. Additionally, when AUROC determined from these results was compared with AUROC (0.790) of the FIB4 index which is the conventional art, it was suggested that the diagnostic accuracy is equivalent to or more than the conventional art.

[Table 46]

| Matteoni classification Types 1-3 vs Type 4 Discrimination method (Measurement method 1) | AUROC | Reference value range (satisfies at least A or B) least A or B) | Reference value range A: Sensitivity 95% to Specificity 95% | Reference value: Sensitivity 95% | | | Reference value: Specificity 95% | | | Reference value range B: Specificity 50% or more and sensitivity 50% or more | Reference value: Specificity | | | Reference value: sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M2 (All factors) | 0.867 | 0.401-0.536 | 0.41-0.532 | 0.410 | 95.5 | 58.5 | 0.532 | 50.7 | 95.4 | 0.401-0.536 | 0.401 | 97.0 | 50.8 | 0.536 | 50.7 | 96.9 |
| M2 (VCAM1) | 0.793 | 0.303-0.538 | 0.303-0.538 | 0.303 | 95.5 | 32.3 | 0.538 | 34.3 | 95.4 | 0.351-0.47 | 0.351 | 88.1 | 50.8 | 0.470 | 50.7 | 89.2 |
| M2 (VCAM1, HA) | 0.838 | 0.328-0.617 | 0.328-0.617 | 0.328 | 95.5 | 20.0 | 0.617 | 37.3 | 95.4 | 0.373-0.514 | 0.373 | 88.1 | 50.8 | 0.514 | 50.7 | 93.8 |
| M2 (VCAM1, CTSD) | 0.785 | 0.313-0.581 | 0.313-0.581 | 0.313 | 95.5 | 16.9 | 0.581 | 46.3 | 95.4 | 0.387-0.542 | 0.387 | 83.6 | 50.8 | 0.542 | 50.7 | 93.8 |
| M2 (VCAM1, COL4) | 0.820 | 0.343-0.558 | 0.343-0.558 | 0.343 | 95.5 | 32.3 | 0.558 | 43.3 | 95.4 | 0.38-0.528 | 0.380 | 88.1 | 50.8 | 0.528 | 50.7 | 92.3 |
| M2 (VCAM1, COL4-7S) | 0.893 | 0.358-0.602 | 0.367-0.554 | 0.367 | 96.6 | 56.6 | 0.554 | 63.8 | 96.2 | 0.358-0.602 | 0.358 | 96.6 | 50.9 | 0.602 | 50.0 | 96.2 |
| M2 (HA, COL4-7S) | 0.785 | 0.354-0.562 | 0.354-0.501 | 0.354 | 96.6 | 47.2 | 0.501 | 70.7 | 96.2 | 0.358-0.562 | 0.358 | 94.8 | 50.9 | 0.562 | 50.0 | 96.2 |
| M2 (HA, COL4) | 0.840 | 0.36-0.522 | 0.36-0.522 | 0.360 | 95.5 | 40.0 | 0.522 | 46.3 | 95.4 | 0.372-0.505 | 0.372 | 91.0 | 50.8 | 0.505 | 50.7 | 93.8 |
| M2 (HA, CTSD) | 0.915 | 0.311-0.545 | 0.311-0.545 | 0.311 | 95.5 | 15.4 | 0.545 | 50.7 | 95.4 | 0.373-0.545 | 0.373 | 82.1 | 50.8 | 0.545 | 50.7 | 95.4 |

(continued)

| Matteoni classification Types 1-3 vs Type 4 Discrimination method (Measurement method 1) | AUROC | Reference value range (satisfies at least A or B) | Reference value range A: Sensitivity 95% to Specificity 95% | Reference value: Sensitivity 95% | | | Reference value: Specificity 95% | | | Reference value range B: Specificity 50% or more and sensitivity 50% or more | Reference value: Specificity | | | Reference value: sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M2 (CTSD, COL4) | 0.779 | 0.317-0.544 | 0.317-0.538 | 0.317 | 95.5 | 10.8 | 0.538 | 52.2 | 95.4 | 0.392-0.544 | 0.392 | 80.6 | 50.8 | 0.544 | 50.7 | 95.4 |
| M2 (CTSD, COL4-7S) | 0.862 | 0.364-0.604 | 0.364-0.535 | 0.364 | 96.6 | 45.3 | 0.535 | 65.5 | 96.2 | 0.379-0.604 | 0.379 | 89.7 | 50.9 | 0.604 | 50.0 | 98.1 |

| Matteoni classification Types 1-3 vs Type 4 Discrimination method (Measurement method 2) | AUROC | Reference value range (satisfies at least A or B) | Reference value to Specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| M2 (VCAM1, HA) | 0.870 | 0.357-0.572 | 0.357-0.526 | 0.357 | 96.7 | 45.5 | 0.526 | 53.3 | 95.5 | 0.36-0.572 | 0.360 | 93.3 | 50.0 | 0.572 | 50.0 | 95.5 |
| M2 (VCAM1, COL4-7S) | 0.904 | 0.356-0.603 | 0.377-0.539 | 0.377 | 96.1 | 59.6 | 0.539 | 66.7 | 95.7 | 0.356-0.603 | 0.356 | 96.1 | 51.1 | 0.603 | 51.0 | 95.7 |
| M2 (HA, COL4-7S) | 0.936 | 0.331-0.563 | 0.35-0.42 | 0.350 | 96.7 | 68.2 | 0.420 | 86.7 | 95.5 | 0.331-0.563 | 0.331 | 100.0 | 50.0 | 0.563 | 50.0 | 95.5 |

(continued)

| Matteoni classification Types 1-3 vs Type 4 Discrimination method (Measurement method 2) | AUROC | Reference value range (satisfies at least A or B) | Reference value to Specificity 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference value | Sensitivity (%) | Specificity (%) | Reference value | Sensitivity (%) | Specificity (%) | | Reference value | Sensitivity (%) | Specificity (%) | Reference value | Sensitivity (%) | Specificity (%) |
| VCAM1 measured value (ng/ml) | 0.796 | 618.1-1239.5 | 618.1-1239.5 | 618.1 | 96.4 | 37.9 | 1239.5 | 33.9 | 96.6 | 701.1-1029.3 | 701.1 | 91.1 | 50.0 | 1029.3 | 50.0 | 89.7 |

Test Example 5: Method for discriminating between symptoms of Stage 0 to Stage 2 and a symptom of Stage 3 or Stage 4 in the Stage of fibrosis

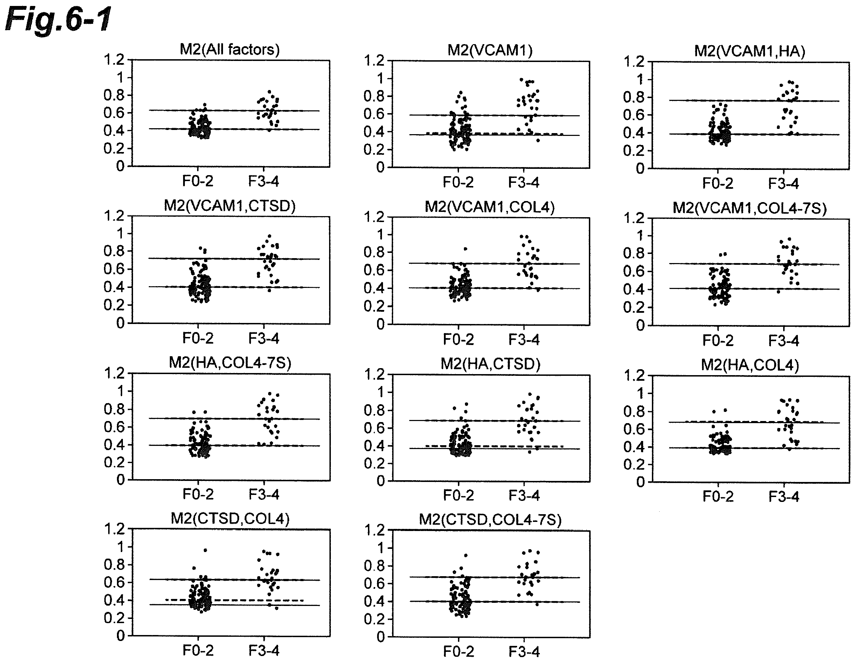

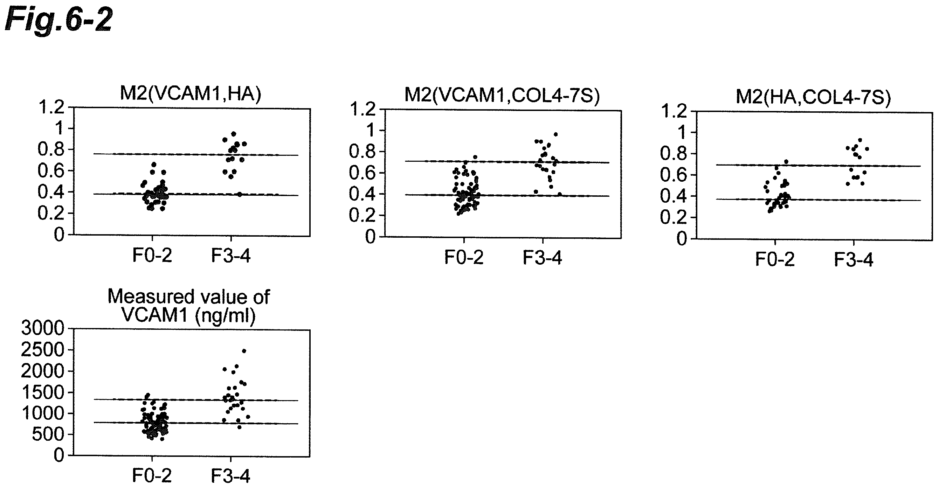

Normalized scores were determined by the same biomolecules and the method as Test Example 4. Graphs of the determined normalized scores classified into the patients group applicable to the symptoms of Stage 0 to Stage 2 (F 0-2) and the patients group applicable to the symptom of Stage 3 or Stage 4 (F 3-4) in the Stage of fibrosis and plotted are shown in Fig. 6-1 and Fig. 6-2. In Figs., the solid lines represent both ends of the "reference value range A" in Table 47 and the dotted lines represent both ends of the "reference value range B" in Table 47. The symptom of Stage 0 herein means the symptom of Type-1 or Type-2 in the Matteoni classification. In all the normalized scores, significant differences were found between the F 0-2 patients group and the F 3-4 patients group, thereby revealing the effectiveness in the discrimination between the Stage 0 to Stage 2 symptoms and the Stage 3 or Stage 4 symptom. Further, it is revealed that the reference value (cutoff value) to discriminate the applicability to the F 3-4 symptom is effective when set within the ranges shown in the following Table 47. Additionally, when AUROC determined from these results was compared with AUROC (0.858) of the FIB4 index which is the conventional art, it was suggested that the diagnostic accuracy excels the conventional art.

[Table 47]

| Fibrosis Stages F0-2 vs F3-4 Discrimination method (Measurement method 1) | AUROC | Refercnce value range (satisfies at least A or B) | Reference value range A: Sensitivity 95% to Specificity 95% | Reference value: Sensitivity 95% | | | Reference value: Specificity 95% Reference value range | | | Refercnce value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Reference value | Sensitivity ; (%) | Specificity (%) | Reference value | Sensitivity (%) | Specificity (%) | | Reference value | Sensitivity (%) | Specificity (%) | Reference value | Sensitivity (%) | Specificity (%) |
| M2 (All factors) | 0.911 | 0.421-0.632 | 0.461-0.629 | 0.461 | 96.4 | 67.3 | 0.629 | 50.0 | 95.2 | 0.421-0.632 | 0.421 | 96.4 | 50.0 | 0.632 | 50.0 | 96.2 |
| M2 (VCAM1) | 0.873 | 0.371-0.587 | 0.371-0.571 | 0.371 | 96.4 | 46.2 | 0.571 | 53.6 | 95.2 | 0.383-0.587 | 0.383 | 92.9 | 50.0 | 0.587 | 50.0 | 96.2 |
| M2 (VCAM1, HA) | 0.911 | 0.389-0.766 | 0.392-0.661 | 0.392 | 96.4 | 51.0 | 0.661 | 60.7 | 95.2 | 0.389-0.766 | 0.389 | 100.0 | 50.0 | 0.766 | 50.0 | 100.0 |
| M2 (VCAM1, CTSD) | 0.900 | 0.406-0.718 | 0.457-0.667 | 0.457 | 96.4 | 67.3 | 0.667 | 57.1 | 95.2 | 0.406-0.718 | 0.406 | 96.4 | 50.0 | 0.718 | 50.0 | 97.1 |
| M2 (VCAM1, COL4) | 0.905 | 0.407-0.678 | 0.407-0.635 | 0.407 | 96.4 | 50.0 | 0.635 | 53.6 | 95.2 | 0.407-0.678 | 0.407 | 96.4 | 50.0 | 0.678 | 50.0 | 99.0 |
| M2 (VCAM1, COL4-7S) | 0.913 | 0.413-0.685 | 0.47-0.632 | 0.470 | 96.0 | 68.6 | 0.632 . | 68.0 | 95.3 | 0.413-0.685 | 0.413 | 96.0 | 50.0 | 0.685 | 52.0 | 97.7 |
| M2 (HA, COL4-7S) | 0.899 | 0.398-0.7 | 0.417-0.655 | 0.417 | 96.0 | 55.8 | 0.655 | 60.0 | 95.3 | 0.398-0.7 | 0.398 | 100.0 | 50.0 | 0.700 | 52.0 | 97.7 |
| M2 (HA, COL4) | 0.901 | 0.392-0.683 | 0.42-0.563 | 0.420 | 96.4 | 64.4 | 0.563 | 75.0 | 95.2 | 0.392-0.683 | 0.392 | 96.4 | 50.0 | 0.683 | 50.0 | 98.1 |
| M2 (HA, CTSD) | 0.906 | 0.373-0.69 | 0.373-0.633 | 0.373 | 96.4 | 42.3 | 0.633 | 64.3 | 95.2 | 0.401-0.69 | 0.401 | 92.9 | 50.0 | 0.690 | 50.0 | 96.2 |
| M2 (CTSD, COL4) | 0.882 | 0.353-0.635 | 0.353-0.633 | 0.353 | 96.4 | 24.0 | 0.633 | 50.0 | 95.2 | 0.405-0.635 | 0.405 | 92.9 | 50.0 | 0.635 | 50.0 | 96.2 |
| M2 (CTSD, COL4-7S) | 0.889 | 0.403-0.676 | 0.475-0.671 | 0.475 | 96.0 | 67.4 | 0.671 | 56.0 | 95.3 | 0.403-0.676 | 0.403 | 96.0 | 50.0 | 0.676 | 52.0 | 95.3 |

| Fibrosis Stages F0-2 vs F3-4 Dis-crimination method (Measure-ment method 2) | | Refercnce value range (satisfies at least A or B) | Referc-nce value range A: Sensitivi-ty 95% to Specifici-ty 95% | Reference value: Sensitivity 95% | | | Reference value: Specificity 95% | | | Reference value range B: Specificity 50% or more and sensitivity 50% or more | Reference value: Specificity 50% | | | Reference value: Sensitivity 50% | | |
|---|---|---|---|---|---|---|---|---|---|---|---|---|---|---|---|---|
| | | | | Refer-ence val-ue | Sensi_tiv-ity (%) | Specifi-city (%) | Refer-ence val-ue | Sensitiv-ity (%) | Specifi-city (%) | | Refer-ence val-ue | Sen-si-t ivi-ty (%) | Specifi-city (%) | Refer-ence val-ue | Sensitiv-ity (%) | Specifi-city (%) |
| M2 (VCAM1, HA) | 0.957 | 0.381-0.759 | 0.381-0.595 | 0.381 | 100.0 | 48.6 | 0.595 | 86.7 | 97.3 | 0.388-0.759 | 0.388 | 93.3 | 51.4 | 0.759 | 53.3 | 100.0 |
| M2 (VCAM1, COL4-7S) | 0.925 | 0.394-0.71 | 0.426-0.637 | 0.426 | 95.8 | 62.2 | 0.637 | 70.8 | 95.9 | 0.394-0.71 | 0.394 | 100.0 | 50.0 | 0.710 | 50.0 | 98.6 |
| M2 (HA, COL4-7S) | 0.955 | 0.373-0.698 | 0.521-0.662 | 0.521 | 100.0 | 83.8 | 0.662 | 53.3 | 97.3 | 0.373-0.698 | 0.373 | 100.0 | 51.4 | 0.698 | 53.3 | 100.0 |
| VCAM1meas ured value (ng/ml) | 0.910 | 789.8-1344.2 | 840-1253.9 | 840.0 | 96.0 | 61.8 | 1253.9 | 64.0 | 95.5 | 789.8-1344.2 | 789.8 | 96.0 | 50.6 | 1344. 2 | 52.0 | 97.8 |

Test Example 6: Method for determining the degree of progression of hepatic fibrosis

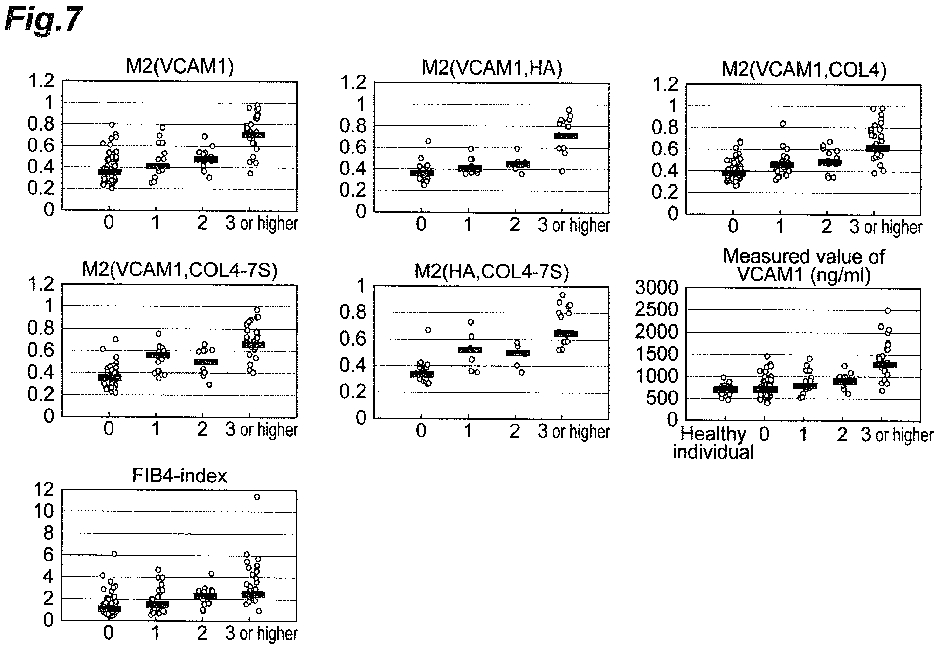

The feasibility to determine the degree of progression of hepatic fibrosis based on each of the normalized scores of Group 2 biomolecules "VCAM1", "VCAM1, HA", "VCAM1, COL4", "VCAM1, COL4-7S" and "HA, COL4-7S" and measured value of VCAM1 (blood concentration) was investigated. The pathological diagnosis score of hepatic fibrosis based on a liver biopsy, Fibrosis stage (scores 0 to 4), was used as the liver pathology index (Kleiner et. al., Design and validation of a histological scoring system for nonalcoholic fatty liver disease, Hepatology 41:1313-21 (2005)). The normalized score was determined using the same method as Test Example 1. The measured value of VCAM1 was quantitatively determined using a reagent "Human sVCAM-1/CD106 Quantikine ELISA Kit" as in the ELISA method. Further, FIB4 index (Hepatology. 2006 Jun; 43(6): 1317-25.) known as the index for evaluating the hepatic fibrosis was determined from a subject's age, AST, ALT and the platelet count. 132 Serum specimens collected from individuals whose pathological diagnosis score was recorded were investigated for the relevance of the Fibrosis stage with the normalized score, measured value of VCAM1 and FIB4 index. For the measured value of VCAM1, the values of Healthy individual measured separately were studied along therewith. The results are shown in Fig. 7. In Fig. 7, the abscissa shows the Fibrosis Stage, the ordinate shows the normalized score, measured value of VCAM1 or FIB4 index and the horizontal line shows the median value of each group. The above normalized score and measured value of VCAM1 were found to be larger as the Fibrosis stage score became larger. Additionally, VCAM1 in Healthy individual was found to have a tendency of showing smaller values than the NAFLD patients.

AUROC (Stage 0 vs Stage 1-4) when the discrimination was carried out between the specimens with score 0 and the specimens with scores 1 or higher on Fibrosis stage, AUROC (Stage 0-1 vs Stage 2-4) when the discrimination was carried out between the specimens with scores 1 or lower and the specimens with scores 2 or higher on Fibrosis stage and AUROC (Stage 0-2 vs Stage 3-4) when the discrimination was carried out between the specimens with scores 2 or lower and the specimens with scores 3 or higher on Fibrosis stage were examined and the accuracy of each index value was compared. The results are shown in Table 48. In the normalized score of each "VCAM1", "VCAM1, HA", "VCAM1, COL4", "VCAM1, COL4-7S" and "HA, COL4-7S" and measured value of VCAM1, AUROC of all the above discriminations exceeded AUROC of FIB4 index under the same conditions. From the above, it is suggested that the normalized score and measured value of VCAM1 are useful to determine the degree of progression of hepatic fibrosis.

[Table 48]

|  | AUROC | | |
|  | Stage 0 vs Stage 1-4 | Stage 0-1 vs Stage 2-4 | Stage 0-2 vs Stage 3-4 |
|---|---|---|---|
| M2 (VCAM1) | 0.796 | 0.842 | 0.910 |
| M2 (VCAM1, HA) | 0.870 | 0.900 | 0.957 |
| M2 (VCAM1, COL4) | 0.820 | 0.858 | 0.905 |
| M2 (VCAM1, COL4-7S) | 0.904 | 0.870 | 0.925 |
| M2 (HA, COL4-7S) | 0.936 | 0.895 | 0.955 |
| VCAM1 measured value | 0.796 | 0.842 | 0.910 |
| FIB4 index (Comparative Example) | 0.790 | 0.840 | 0.858 |

Test Example 7: Method for determining the degree of progression of hepatic inflammation

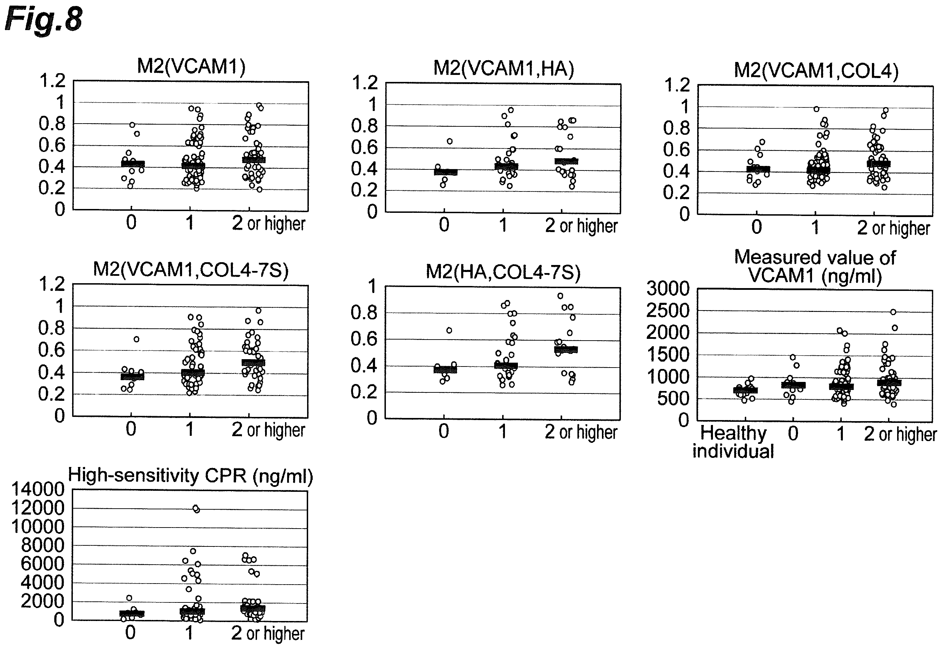

The feasibility to evaluate the degree of progression of hepatic inflammation based on each of the normalized scores of Group 2 biomolecules "VCAM1", "VCAM1, HA", "VCAM1, COL4", "VCAM1, COL4-7S" and "HA, COL4-7S" and measured value of VCAM1 (blood concentration) was investigated. The pathological diagnosis score (scores 0 to 3) of lobular inflammation based on a liver biopsy was used as the hepatic inflammation index (Kleiner et. al., Design and validation of a histological scoring system for nonalcoholic fatty liver disease, Hepatology 41:1313-21 (2005)). The normalized score was determined using the same method as Test Example 1. The measured value of VCAM1 was quantitatively determined using a reagent "Human sVCAM-1/CD106 Quantikine ELISA Kit" as in the ELISA method. Further, high-sensitivity CRP (Hepatogastroenterology. 2014 Mar-Apr; 61(130): 422-5) known as the index for evaluating hepatic inflammation was determined by the latex agglutination method. 132 Serum specimens collected from individuals whose pathological diagnosis score was recorded were investigated for the relevance of the liver lobular inflammation score with the normalized score, measured value of VCAM1 and high-sensitivity CRP. For the measured value of VCAM1, the values of Healthy individual measured separately were studied along therewith. The results are shown in Fig. 8. In Fig. 8, the abscissa shows the liver lobular inflammation score, the ordinate shows the normalized score, measured value of VCAM1 or high-sensitivity CRP measured value and the horizontal line shows the median value of each group. The above normalized score and measured value of VCAM1 were found to be larger as the liver lobular inflammation score became larger. Additionally, VCAM1 in Healthy individual was found to have a tendency of showing smaller values than the NAFLD patients.

AUROC (inflammation 0 vs inflammation 1-3) when the discrimination was carried out between the specimens with score 0 and the specimens with scores 1 or higher on the lobular inflammation score and AUROC (inflammation 0-1 vs inflammation 2-3) when the discrimination was carried out between the specimens with scores 1 or lower and the specimens with scores 2 or higher on the lobular inflammation score were examined and the accuracy of each index value was compared. The results are shown in Table 49. In the normalized score of each "VCAM1, HA", "VCAM1, COL4-7S" and "HA, COL4-7S", AUROC exceeded the high-sensitivity CRP in both of the discriminations between the specimens with score 0 and the specimens with scores 1 or higher and the discrimination between the specimens with scores 1 or lower and the specimens with scores 2 or higher. In the normalized score of each "VCAM1" and "VCAM1, COL4" and the measured value of VCAM1, AUROC exceeded the high-sensitivity CRP in the discrimination between the specimens with scores 1 or lower and the specimen with score 2 or higher. From the above, it is suggested that the normalized score and measured value of VCAM1 are useful to determine the degree of progression of hepatic inflammation.

[Table 49]

| | AUROC | |
| --- | --- | --- |
| | Inflammation 0 vs Inflammation 1-3 | Inflammation0-1 vs Inflammation 2-3 |
| M2 (VCAM1) | 0.552 | 0.689 |
| M2 (VCAM1, HA) | 0.837 | 0.582 |
| M2 (VCAM1, COL4) | 0.647 | 0.725 |
| M2 (VCAM1, COL4-7S) | 0.763 | 0.713 |
| M2 (HA, COL4-7S) | 0.776 | 0.575 |
| VCAM1 measured value | 0.552 | 0.689 |
| High-sensitivity CRP (Comparative Example) | 0.681 | 0.556 |

Test Example 8: Method for determining the degree of progression of hepatocellular ballooning degeneration

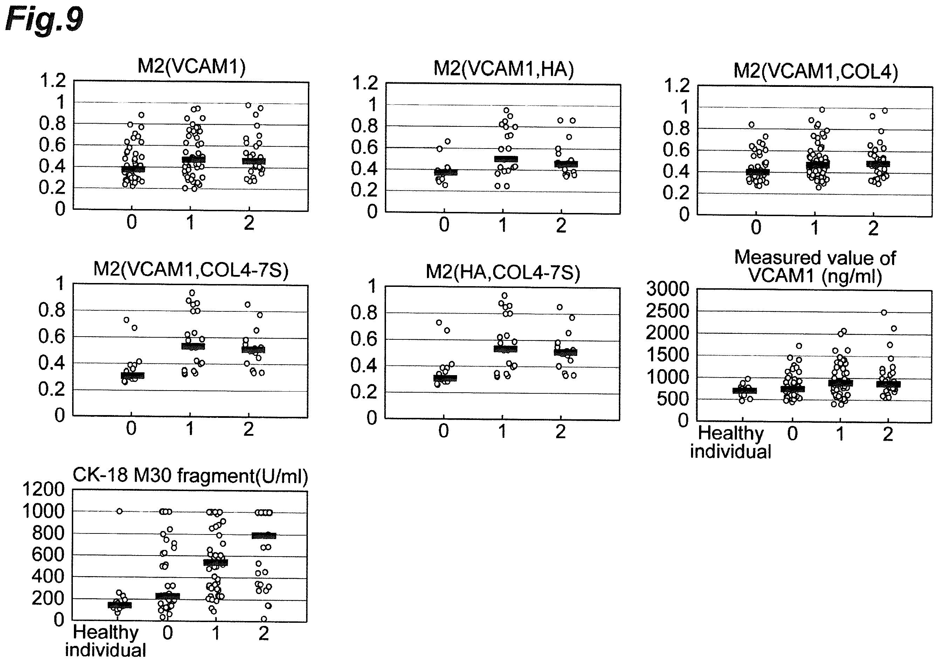

The feasibility to determine the degree of progression of hepatocellular ballooning degeneration based on each of the normalized scores of Group 2 biomolecules "VCAM11", "VCAM1, HA", "VCAM1, COL4", "VCAM1, COL4-7S" and "HA, COL4-7S" and measured value of VCAM1 (blood concentration) was investigated. The pathological diagnosis score (scores 0 to 2) of ballooning degeneration based on a liver biopsy was used as the hepatocellular ballooning degeneration index (Kleiner et. al., Design and validation of a histological scoring system for nonalcoholic fatty liver disease, Hepatology 41:1313-21 (2005)). The normalized score was determined using the same method as Test Example 1. The measured value of VCAM1 was quantitatively determined using a reagent "Human sVCAM-1/CD106 Quantikine ELISA Kit" as in the ELISA method. Further, CK18 M30 fragment (J Clin Gastroenterol. 2010 Jul; 44(6): 440-7) known as the index for evaluating the hepatocellular ballooning degeneration was determined by the ELISA method "M30 Apoptosense ELISA". 132 Serum specimens collected from individuals whose pathological diagnosis score was recorded were investigated for the relevance of the hepatocellular ballooning degeneration score with the normalized score, measured value of VCAM1 and CK18 M30 fragment. For the measured value of VCAM1 and CK18 M30 fragment, the values of Healthy individual measured separately were studied along therewith. The results are shown in Fig. 9. In Fig. 9, the abscissa shows the hepatocellular ballooning degeneration score, the ordinate shows the normalized score, measured value of VCAM1 or CK18 M30 fragment measured value and the horizontal line shows the median value of each group. The above normalized score and measured value of VCAM1 were found to be larger as the hepatocellular ballooning degeneration score became larger. Additionally, VCAM1 in Healthy individual was found to have a tendency of showing smaller values than the NAFLD patients.

AUROC (ballooning degeneration 0 vs ballooning degeneration 1-2) when the discrimination was carried out between the specimens with score 0 and the specimens with scores 1 or higher on the hepatocellular ballooning degeneration score and AUROC (ballooning degeneration 0-1 vs ballooning degeneration 2) when the discrimination was carried out between the specimens with scores 1 or lower and the specimens with score 2 on the hepatocellular ballooning degeneration score were examined and the accuracy of each index value was compared. The results are shown in Table 50. In the normalized score of each "VCAM1", "VCAM1, HA", "VCAM1, COL4", "VCAM1, COL4-7S" and "HA, COL4-7S" and measured value of VCAM1, AUROC in both discriminations exceeded CK18 M30 fragment. From the above, it is suggested that the normalized score and measured value of VCAM1 are useful to determine the degree of progression of hepatocellular ballooning degeneration.

[Table 50]

| | AUROC | |
|---|---|---|
| | Ballooning degeneration 0 vs Ballooning degeneration 1-2 | Ballooning degeneration 0-1 vs Ballooning degeneration 2 |
| M2 (VCAM1) | 0.746 | 0.768 |
| M2 (VCAM1, HA) | 0.807 | 0.759 |
| M2 (VCAM1, COL4) | 0.751 | 0.784 |
| M2 (VCAM1, COL4-7S) | 0.796 | 0.801 |
| M2 (HA, COL4-7S) | 0.837 | 0.801 |
| VCAM1 measured value | 0.746 | 0.768 |
| CK18 M30 fragment (Comparative Example) | 0.676 | 0.677 |

Test Example 9: Method for determining nonalcoholic fatty liver disease (NAFLD) activity

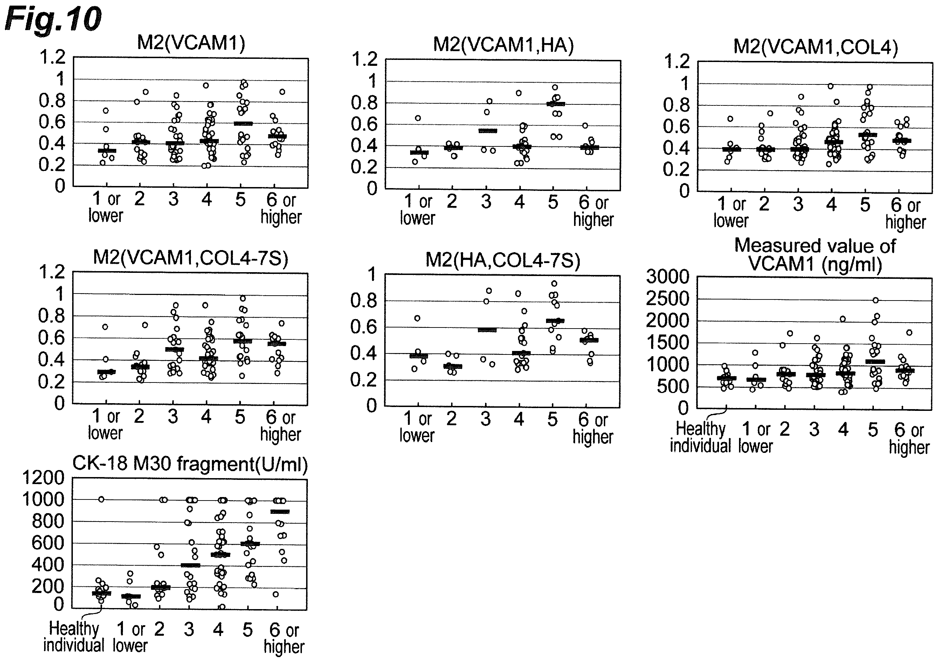

The feasibility to evaluate the activity of NAFLD based on each of the normalized scores of Group 2 biomolecules "VCAM1", "VCAM1, HA", "VCAM1, COL4", "VCAM1, COL4-7S" and "HA, COL4-7S" and measured value of VCAM1 (blood concentration) was investigated. The pathological diagnosis score (scores 0 to 8) of NAFLD activity based on a liver biopsy was used as the NAFLD activity index (Kleiner et. al., Design and validation of a histological scoring system for nonalcoholic fatty liver disease, Hepatology 41:1313-21 (2005)). The normalized score was determined using the same method as Test Example 1. The measured value of VCAM1 was quantitatively determined using a reagent "Human sVCAM-1/CD106 Quantikine ELISA Kit" as in the ELISA method. Further, CK18 M30 fragment (J Clin Gastroenterol. 2010 Jul; 44(6): 440-7) known as the index for evaluating the NAFLD activity was determined by the ELISA method "M30 Apoptosense ELISA". 132 Serum specimens collected from individuals whose pathological diagnosis score was recorded were investigated for the relevance of the NAFLD activity score with the normalized score, measured value of VCAM1 and CK18 M30 fragment. For the measured value of VCAM1 and CK18 M30 fragment, the values of Healthy individual measured separately were studied along therewith. The results are shown in Fig. 10. In Fig. 10, the abscissa shows the NAFLD activity score, the ordinate shows the normalized score, measured value of VCAM1 or CK18 M30 fragment measured value and the horizontal line shows the median of each group. The normalized scores of Group 2 including VCAM1 and measured value of VCAM1 were found to be larger as the NAFLD activity score became larger. Additionally, VCAM1 in Healthy individual was found to have a tendency of showing smaller values than the NAFLD patients.

AUROC (NAS 1 vs NAS 2-8) when the discrimination was carried out between the specimens with score 1 and the specimens with scores 2 or higher on the NAFLD activity score, AUROC (NAS 1-2 vs NAS 3-8) when the discrimination was carried out between the specimens with scores 2 or lower and the specimens with scores 3 or higher on the NAFLD activity score, AUROC (NAS 1-3 vs NAS 4-8) when the discrimination was carried out between the specimens with scores 3 or lower and the specimens with scores 4 or higher on the NAFLD activity score, AUROC (NAS 1-4 vs NAS 5-8) when the discrimination was carried out between the specimens with scores 4 or lower and the specimens with scores 5 or higher on the NAFLD activity score and AUROC (NAS 1-5 vs NAS 6-8) when the discrimination was carried out between the specimens with scores 5 or lower and the specimens with scores 6 or higher on the NAFLD activity score were examined and the accuracy of each index value was compared. The results are shown in Table 51. In the normalized score of "VCAM1, HA", AUROC exceeded the CK18 M30 fragment in the discrimination between the specimens with scores 5 or lower and the specimens with scores 6 or higher. In the normalized score of each "VCAM1", "VCAM1, COL4" and "VCAM1, COL4-7S" and the measured value of VCAM1, AUROC exceeded the CK18 M30 fragment in the discrimination between the specimens with scores 4 or lower and the specimens with scores 5 or higher on the NAFLD activity score and the discrimination between the specimens with scores 5 or lower and the specimens with scores 6 or higher on the NAFLD activity score.

In the normalized score of "HA, COL4-7S", AUROC exceeded the CK18 M30 fragment in the discrimination between the specimens with scores 2 or lower and the specimens with scores 3 or higher on the NAFLD activity score, the discrimination between the specimens with scores 3 or lower and the specimens with scores 4 or higher on the NAFLD activity score, the discrimination between the specimens with scores 4 or lower and the specimens with scores 5 or higher on the NAFLD activity score and the discrimination between the specimens with scores 5 or lower and the specimens with scores 6 or higher on the NAFLD activity score. From the above, it is suggested that the normalized score and measured value of VCAM1 are useful to determine the activity of NAFLD.

[Table 51]

| | AUROC | | | | |
|---|---|---|---|---|---|
| | NAS1 vs NAS 2-8 | NAS1-2 vs NAS 3-8 | NAS1-3 vs NAS 4-8 | NAS1-4 vs NAS 5-8 | NAS1-5 vs NAS 6-8 |
| M2 (VCAM1) | 0.593 | 0.653 | 0.629 | 0.700 | 0.745 |
| M2 (VCAM1, HA) | 0.800 | 0.741 | 0.657 | 0.640 | 0.749 |
| M2 (VCAM1, COL4) | 0.701 | 0.714 | 0.671 | 0.696 | 0.748 |
| M2 (VCAM1, COL4-7S) | 0.790 | 0.800 | 0.690 | 0.727 | 0.785 |
| M2 (HA, COL4-7S) | 0.650 | 0.763 | 0.702 | 0.688 | 0.787 |
| VCAM1 measured value | 0.593 | 0.653 | 0.629 | 0.700 | 0.745 |
| CK18 M30 fragment (Comparative Example) | 0.851 | 0.760 | 0.698 | 0.672 | 0.704 |

Test Example 10: Method for determining the degree of progression of hepatic fibrosis, the degree of progression of hepatic inflammation, the degree of progression of hepatocellular ballooning degeneration and the NAFLD activity, based on the normalized score of "HA, COL4"

The feasibility to determine the degree of progression of hepatic fibrosis, the degree of progression of hepatic inflammation, the degree of progression of hepatocellular ballooning degeneration and the NAFLD activity based on the normalized score of Group 2 biomolecules "HA, COL4" was investigated. The methods used were the same as the above Test Example 6, Test Example 7, Test Example 8 and Test Example 9, respectively.

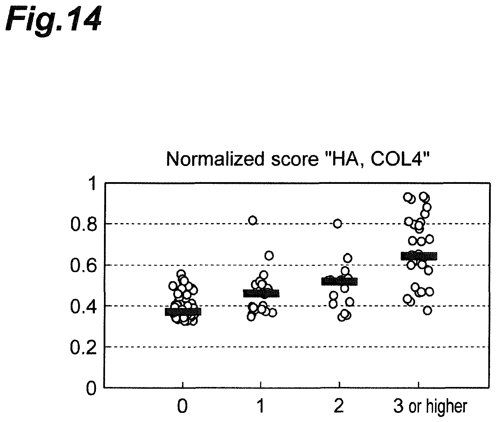

Fig. 14 shows the relation of the normalized score of "HA, COL4" with the degree of progression of hepatic fibrosis. In Fig. 14, the abscissa shows the Fibrosis Stage, the ordinate shows the normalized score and the horizontal line shows the median value of each group. The above normalized score was found to be larger as the Fibrosis stage score became larger.

The results on determination of the degree of progression of hepatic fibrosis are shown in Table 52. In all the discriminations, AUROC exceeded AUROC of FIB4 index under the same conditions. From the above, it is suggested that the normalized score of "HA, COL4-7S" is useful to determine the degree of progression of hepatic fibrosis.

[Table 52]

| Diagnostic method | Stage:0 vs. Stage:1-4 | Stage:0-1 vs. Stage:2-4 | Stage:0-2 vs. Stage:3-4 |
|---|---|---|---|
| Normalized score "HA, COL4" | 0.840 | 0.858 | 0.901 |
| FIB4-index (Comparative Example) | 0.790 | 0.840 | 0.858 |

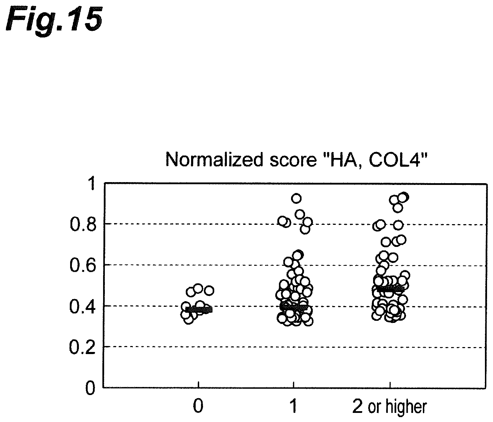

Fig. 15 shows the relation of the normalized score of "HA, COL4" with the degree of progression of hepatic inflammation. In Fig. 15, the abscissa shows the liver lobular inflammation score, the ordinate shows the normalized score and the horizontal line shows the median value of each group. The above normalized score was found to be larger as the liver lobular inflammation score became larger.

The results on determination of the degree of progression of hepatic inflammation are shown in Table 53. In the discrimination between the specimens with scores 1 or lower and the specimens with scores 2 or higher on the liver lobular inflammation score, AUROC of the above normalized score exceeded AUROC of high-sensitivity CRP under the same conditions. From the above, it is suggested that the normalized score of "HA, COL4" is useful to determine the degree of progression of hepatic inflammation.

[Table 53]

| Diagnostic method Diagnostic | Inflammation: 0 vs Inflammation: 1-3 | Inflammation: 0-1 vs Inflammation: 2-3 |
|---|---|---|
| Normalized score "HA, COL4" | 0.650 | 0.693 |
| High-sensitivity CRP (ng/ml) (Comparative Example) | 0.681. | 0.556 |

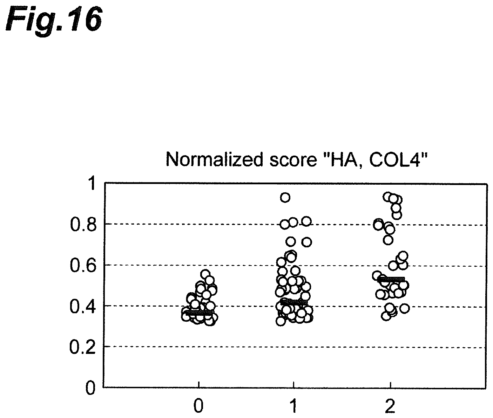

Fig. 16 shows the relation of the normalized score of "HA, COL4" with the hepatocellular ballooning degeneration. In Fig. 16, the abscissa shows the hepatocellular ballooning degeneration score, the ordinate shows the normalized score and the horizontal line shows the median value of each group. The above normalized score was found to be larger as the hepatocellular ballooning degeneration score became larger.

The results on determination of the degree of progression of hepatocellular ballooning degeneration are shown in Table 54. In all the discriminations, AUROC of the above normalized score exceeded AUROC of CK18 M30 fragment under the same conditions. From the above, it is suggested that the normalized score of "HA, COL4" is useful to determine the degree of progression of hepatocellular ballooning degeneration.

[Table 54]

| Diagnostic method | Ballooning degeneration: 0 vs. Ballooning degeneration: 1-2 | Ballooning degeneration: 0-1 vs. Ballooning degeneration: 2 |
|---|---|---|
| Normalized score "HA, COL4" | 0.783 | 0.792 |
| CK18 M30 fragment (U/ml) (Comparative Example) | 0.676 | 0.677 |

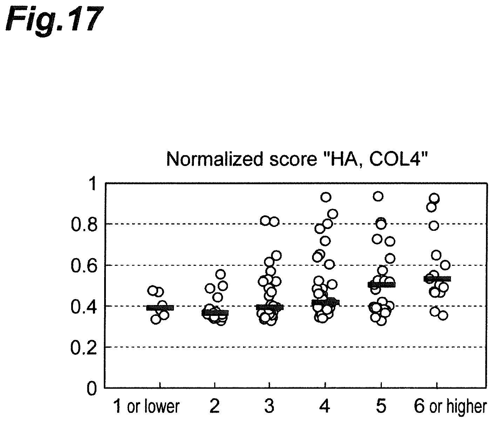

Fig. 17 shows the relation of the normalized score of "HA, COL4" with the NAFLD activity. In Fig. 17, the abscissa shows the NAFLD activity score, the ordinate shows the normalized score and the horizontal line shows the median value of each group. The above normalized score was found to be larger as the NAFLD activity score became larger.

The results on determination of the NAFLD activity are shown in Table 55. In the discrimination carried out between the specimens with scores 4 or lower and the specimens with scores 5 or higher on the NAFLD activity score and the discrimination carried out between the specimens with scores 5 or lower and the specimens with scores 6 or higher on the NAFLD activity score, AUROC of the above normalized score exceeded AUROC of CK18 M30 fragment under the same conditions. From the above, it is suggested that the normalized score of "HA, COL4" is useful to determine the NAFLD activity.

[Table 55]

| Diagnostic method | NAS:1 vs. NAS: 2-8 | NAS:1-2 vs. NAS:3-8 | NAS:1-3 vs. NAS:4-8 | NAS:1-4 vs. NAS:5-8 | NAS:1-5 vs. NAS:6-8 |
|---|---|---|---|---|---|
| Normalized score "HA, COL4" | 0.636 | 0.721 | 0.678 | 0.682 | 0.741 |
| CK18 M30 fragment (U/ml) (Comparative Example) | 0.851 | 0.760 | 0.698 | 0.672 | 0.704 |

**Reference Sings List**

1 ... Measurement apparatus, 2 ... Light source part, 3 ... Sample-setting part, 4 ... Detection part, 5 ... Communication part, 11 ... Liver disease determination apparatus, 12 ... Control part, 13 ... Recording part, 14 ... Arithmetic part, 15 ... Display part, 16 ... Input part, 100 ... Liver disease determination system, D1 ... Acquisition unit, D2 ... Normalization unit, D3 ... Comparison unit, D4 ... Determination unit, D5 ... Display unit, S1 ... Acquisition step, S2 ... Normalization step, S3 ... Comparison step, S4 ... Determination step, S5 ... Display step

**Claims**

1. A method for discriminating between nonalcoholic fatty liver (NAFL) and nonalcoholic steatohepatitis (NASH), the method comprising:

   (1) a step of measuring quantities of marker molecules contained in blood collected from a subject, wherein the marker molecules to be measured are

   (A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2, at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40, at least two marker molecules selected from Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone, or
   at least two marker molecules selected from Group 4 consisting of AREG, BDNF, CD40-L, EREG, FGF2, HBEGF, IGFBP2, MMP9, PAI-1, PDGFB, PLGF, SAP, TGFA, TGFB1, THBS1, and VTN, or,
   (B) at least two marker molecules selected from one of two groups selected from the Group 1, the Group 2, the Group 3, and the Group 4 and at least two marker molecules selected from the other;

   (2) a step of determining an index value from a normalized score calculated based on the quantities of the marker molecules, wherein the normalized score based on the quantities of the marker molecules belonging to the same group is calculated by using the following formula 1:

$$M = \frac{1}{N}\sum_{i=1}^{N} \frac{1}{1 + exp\left(-\alpha_i \times \dfrac{m_i - mean(\mathrm{m}_i)}{sd(\mathrm{m}_i)}\right)}$$

$$\alpha_i = \begin{cases} 1 & \left(mean\left(\mathrm{m}_i^{NASH}\right) > mean\left(\mathrm{m}_i^{NAFL}\right)\right) \\ -1 & (otherwise) \end{cases}$$

$$\text{(formula 1)}$$

   wherein M denotes a normalized score; N denotes a number of marker molecules; $m_i$ denotes the quantity of each marker molecule measured; mean ($m_i$) denotes a predetermined mean of a quantity of each marker molecule; sd ($m_i$) denotes a predetermined standard deviation for each marker molecule; mean ($m_i^{NASH}$) denotes a predetermined mean of a quantity of each marker molecule for patients affected with NASH; and mean ($m_i^{NAFL}$) denotes a predetermined mean of a quantity of each marker molecule for patients affected with NAFL, and, in a case that the marker molecules correspond to the (A), the index value is the normalized score, and in a case that the marker molecules correspond to the (B), the index value is calculated from normalized scores for the two groups; and
   (3) a step of determining that the subject is affected with or possibly affected with NASH in a case that the index value is larger than a reference value.

2. The method according to claim 1, wherein, in a case of the (B), the index value is calculated from normalized scores for the two groups by using a linear equation or a nonlinear equation.

3. A method for discriminating between nonalcoholic fatty liver (NAFL) and nonalcoholic steatohepatitis (NASH), the method comprising:

(1) a step of measuring quantities of marker molecules contained in blood collected from a subject, wherein the marker molecules to be measured are

(A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2, at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40, at least two marker molecules selected from Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone, or
at least two marker molecules selected from Group 4 consisting of AREG, BDNF, CD40-L, EREG, FGF2, HBEGF, IGFBP2, MMP9, PAI-1, PDGFB, PLGF, SAP, TGFA, TGFB 1, THBS1, and VTN, or
(B) at least two marker molecules selected from one of two groups selected from the Group 1, the Group 2, the Group 3, and the Group 4 and at least two marker molecules selected from the other;

(2) a step of determining an index value from a normalized score calculated based on the quantities of the marker molecules, wherein the normalized score based on the quantities of the marker molecules belonging to the same group is calculated by using the following formula 1:

$$M = \frac{1}{N}\sum_{i=1}^{N}\frac{1}{1 + exp\left(-\alpha_i \times \frac{m_i - mean(\mathrm{m}_i)}{sd(\mathrm{m}_i)}\right)}$$

$$\alpha_i = \begin{cases} 1 & \left(mean(\mathrm{m}_i^{NASH}) > mean(\mathrm{m}_i^{NAFL})\right) \\ -1 & (otherwise) \end{cases}$$

(formula 1)

wherein M denotes a normalized score; N denotes a number of marker molecules; $m_i$ denotes the quantity of each marker molecule measured; mean $(m_i)$ denotes a predetermined mean of a quantity of each marker molecule; sd $(m_i)$ denotes a predetermined standard deviation for each marker molecule; mean $(m_i^{NASH})$ denotes a predetermined mean of a quantity of each marker molecule for patients affected with NASH; and mean $(m_i^{NAFL})$ denotes a predetermined mean of a quantity of each marker molecule for patients affected with NAFL, and, in a case that the marker molecules correspond to the (A), the index value is the normalized score, and in a case that the marker molecules correspond to the (B), the index value is calculated from normalized scores for the two groups by using a method with an equation for mean calculation, a method with a linear regression equation, a method with a linear discriminant, a method with a logistic regression equation, a method with a nonlinear equation for mean calculation, a method with a nonlinear regression equation, a method with a non-linear discriminant, or a method with a nonlinear logistic regression equation; and
(3) a step of determining that the subject is affected with or possibly affected with NASH in a case that the index value is larger than a reference value.

4. The method according to any one of claims 1 to 3, wherein, in a case of the (B), the index value is calculated from normalized scores for the two groups by using the following formula 2, as a method with a logistic regression equation:

$$I = a_1 \times M_{a1} + a_2 \times M_{a2} \text{ (formula 2)}$$

wherein I denotes an index value; $M_{a1}$ and $M_{a2}$ denote a normalized score for one of the two groups and a normalized score for the other, respectively; and $a_1$ and $a_2$ each denote a constant determined in logistic regression analysis.

5. The method according to any one of claims 1 to 3, wherein, in a case of the (B), the index value is calculated from normalized scores for the two groups by using any one of the following Decision trees 1 to 6, as a method with a nonlinear discriminant:

Decision tree 1: in a case that the marker molecules are selected from the Group 1 and Group 2, if $M1_a \geq 0.476$, then I = 2, if $M1_a < 0.476$ and $M2_a \geq 0.466$, then I = 1, and if $M1_a < 0.476$ and $M2_a < 0.466$, then I = 0;
Decision tree 2: in a case that the marker molecules are selected from the Group 1 and Group 3, if $M3_a \geq 0.409$ and $M1_a \geq 0.45$, then I = 2, if $M3_a \geq 0.409$ and $M1_a < 0.45$, then I = 1, and if $M3_a < 0.409$, then I = 0;
Decision tree 3: in a case that the marker molecules are selected from the Group 1 and Group 4, if $M4_a \geq 0.547$, then I = 2, if $M4_a < 0.547$ and $M1_a \geq 0.468$, then I = 1, and if $M4_a < 0.547$ and $M1_a < 0.468$, then I = 0;
Decision tree 4: in a case that the marker molecules are selected from the Group 2 and Group 3, if $M3_a \geq 0.409$ and $M2_a \geq 0.412$, then I = 2, if $M3_a \geq 0.409$ and $M2_a < 0.412$, then I = 1, and if $M3_a < 0.409$, then I = 0;
Decision tree 5: in a case that the marker molecules are selected from the Group 2 and Group 4, if $M4_a \geq 0.547$, then I = 2, if $M4_a < 0.547$ and $M2_a \geq 0.413$, then I = 1, and if $M4_a < 0.547$ and $M2_a < 0.413$, then I = 0; and
Decision tree 6: in a case that the marker molecules are selected from the Group 3 and Group 4, if $M3_a \geq 0.409$, then I = 2, if $M3_a < 0.409$ and $M4_a \geq 0.588$, then I = 1, and if $M3_a < 0.409$ and $M4_a < 0.588$, then I = 0,

wherein I denotes an index value; and $M1_a$, $M2_a$, $M3_a$, and $M4_a$ denote normalized scores for the Group 1, Group 2, Group 3, and Group 4, respectively.

6. The method according to any one of claims 1 to 3, wherein, in a case of the (A), the marker molecules are PROS1 and CLU, each belonging to the Group 1.

7. The method according to any one of claims 1 to 3, wherein, in a case of the (A), the marker molecules are VCAM1 and HA; VCAM1 and CTSD; VCAM1 and COL4; VCAM1 and COL4-7S; HA and COL4-7S; HA and CTSD; HA and COL4; CTSD and COL4; or CTSD and COL4-7S, each belonging to the Group 2.

8. The method according to any one of claims 1 to 3, wherein, in a case of the (A), the marker molecules are AST and PSAT; AST and LEP; AST and ICAM1; AST, PSAT, and LEP; AST, PSAT, and ICAM1; AST, LEP, and CK-18; AST, ICAM1, and GSTA1; or AST, PSAT, and 6Ckine, each belonging to the Group 3.

9. The method according to any one of claims 1 to 5, wherein, in a case of combination of the Group 1 and the Group 3 in the (B), the marker molecules selected from the Group 1 are PROS1 and CLU, and the marker molecules selected from the Group 3 are AST and PSAT; AST and LEP; AST and ICAM1; AST, PSAT, and LEP; AST, PSAT, and ICAM1; AST, LEP, and CK-18; AST, ICAM1, and GSTA1; or AST, PSAT, and 6Ckine.

10. A method for determining presence of hepatic fibrosis, the method comprising:

a step of measuring quantities or a quantity of marker molecules or a marker molecule contained in blood collected from a subject, wherein the marker molecules or marker molecule to be measured are/is at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40, or VCAM1;
a step of determining that the subject has or possibly has a symptom of hepatic fibrosis in a case that a normalized score calculated based on the quantities or quantity of the marker molecules or maker molecule is larger than a reference value, wherein the normalized score is calculated by using the following formula 1:

$$M = \frac{1}{N}\sum_{i=1}^{N} \frac{1}{1 + exp\left(-\alpha_i \times \dfrac{m_i - mean(m_i)}{sd(m_i)}\right)}$$

$$\alpha_i = \begin{cases} 1 & \left(mean\left(m_i^{NASH}\right) > mean\left(m_i^{NAFL}\right)\right) \\ -1 & (otherwise) \end{cases}$$

(formula 1)

wherein M denotes a normalized score; N denotes a number of marker molecules; $m_i$ denotes the quantity of each marker molecule measured; mean ($m_i$) denotes a predetermined mean of a quantity of each marker molecule; sd ($m_i$) denotes a predetermined standard deviation for each marker molecule; mean ($m_i^{NASH}$) denotes a predetermined mean of a quantity of each marker molecule for patients affected with NASH; and mean ($m_i^{NAFL}$) denotes a predetermined mean of a quantity of each marker molecule for patients affected with NAFL.

11. The method for determining presence of hepatic fibrosis according to claim 10, wherein
the marker molecules or marker molecule are/is VCAM1, HA, CTSD, COL4, COL4-7S, ALB, AFP, AXL, CCL19, CGB, CSF1, FAS, Mac-2bp, CA19-9, NRCAM, OPG, VWF, and YKL-40; VCAM1; VCAM1 and HA; VCAM1 and CTSD; VCAM1 and COL4; VCAM1 and COL4-7S; HA and CTSD; CTSD and COL4; HA and COL4; HA and COL4-7S; or CTSD and COL4-7S.

12. The method according to claim 10 or 11, wherein determination of presence of hepatic fibrosis is based on discrimination between symptoms of Type-1 to Type-3 and a symptom of Type-4 in Matteoni classification, or discrimination between a symptom of Stage 0, Stage 1, or Stage 2 and a symptom of Stage 3 or Stage 4 in Stages of fibrosis.

13. A method for determining a degree of progression of a symptom of a hepatic disease, the method comprising:

a step of measuring a quantity or quantities of a marker molecule or marker molecules contained in blood collected from a subject, wherein the marker molecule or marker molecules to be measured is/are VCAM1; VCAM1 and HA; HA and COL4; VCAM1 and COL4; HA and COL4-7S; or VCAM1 and COL4-7S; and
a step of determining that a degree of progression of hepatic fibrosis, a degree of progression of hepatocellular ballooning degeneration, or a degree of progression of hepatic inflammation in the subject is higher or possibly higher as a normalized score calculated based on the quantity or quantities of the marker molecule or molecules is higher, wherein the normalized score is calculated by using the following formula 1:

$$M = \frac{1}{N}\sum_{i=1}^{N} \frac{1}{1 + exp\left(-\alpha_i \times \dfrac{m_i - mean(m_i)}{sd(m_i)}\right)}$$

$$\alpha_i = \begin{cases} 1 & \left(mean\left(m_i^{NASH}\right) > mean\left(m_i^{NAFL}\right)\right) \\ -1 & (otherwise) \end{cases}$$

(formula 1)

wherein M denotes a normalized score; N denotes a number of marker molecules; $m_i$ denotes the quantity of each marker molecule measured; mean ($m_i$) denotes a predetermined mean of a quantity of each marker

molecule; sd ($m_i$) denotes a predetermined standard deviation for each marker molecule; mean ($m_i^{NASH}$) denotes a predetermined mean of a quantity of each marker molecule in patients presenting with NASH; and mean ($m_i^{NAFL}$) denotes a predetermined mean of a quantity of each marker molecule in patients presenting with NAFL.

**14.** A method for determining a degree of progression of a symptom of nonalcoholic fatty liver disease (NAFLD), the method comprising:

   a step of measuring a quantity or quantities of a marker molecule or marker molecules contained in blood collected from a subject, wherein the marker molecule or marker molecules to be measured is/are VCAM1; VCAM1 and HA; HA and COL4; VCAM1 and COL4; HA and COL4-7S; or VCAM1 and COL4-7S; and
   a step of determining that a degree of progression of hepatic fibrosis, a degree of progression of activity of nonalcoholic fatty liver disease, a degree of progression of hepatocellular ballooning degeneration, or a degree of progression of hepatic inflammation in the subject is higher or possibly higher as a normalized score calculated based on the quantity or quantities of the marker molecule or molecules is higher, wherein the normalized score is calculated by using the following formula 1:

$$M = \frac{1}{N} \sum_{i=1}^{N} \frac{1}{1 + exp\left(-\alpha_i \times \dfrac{m_i - mean(\mathrm{m}_i)}{sd(\mathrm{m}_i)}\right)}$$

$$\alpha_i = \begin{cases} 1 & \left(mean\left(\mathrm{m}_i^{NASH}\right) > mean\left(\mathrm{m}_i^{NAFL}\right)\right) \\ -1 & (otherwise) \end{cases}$$

(formula 1)

   wherein M denotes a normalized score; N denotes a number of marker molecules; $m_i$ denotes the quantity of each marker molecule measured; mean ($m_i$) denotes a predetermined mean of a quantity of each marker molecule; sd ($m_i$) denotes a predetermined standard deviation for each marker molecule; mean ($m_i^{NASH}$) denotes a predetermined mean of a quantity of each marker molecule in patients presenting with NASH; and mean ($m_i^{NAFL}$) denotes a predetermined mean of a quantity of each marker molecule in patients presenting with NAFL.

**15.** A kit for use in the method according to any one of claims 1 to 14, the kit comprising:

   a reagent for detecting the marker molecule or marker molecules; and
   an instruction for performing the method.

**16.** A method for discriminating between nonalcoholic fatty liver (NAFL) and nonalcoholic steatohepatitis (NASH), the method comprising:

   a step of measuring a quantity of VCAM1 contained in blood collected from a subject; and
   a step of determining that the subject is affected with or possibly affected with NASH in a case that the quantity of VCAM1 is larger than a reference value.

**17.** A method for determining presence of hepatic fibrosis, the method comprising:

   a step of measuring a quantity of VCAM1 contained in blood collected from a subject; and
   a step of determining that the subject has or possibly has a symptom of hepatic fibrosis in a case that the quantity of VCAM1 is larger than a reference value.

**18.** A method for determining a degree of progression of a symptom of a hepatic disease, the method comprising:

   a step of measuring a quantity of VCAM1 contained in blood collected from a subject; and

a step of determining that a degree of progression of hepatic fibrosis, a degree of progression of hepatocellular ballooning degeneration, or a degree of progression of hepatic inflammation in the subject is higher or possibly higher as the quantity of VCAM1 is larger.

**19.** A method for determining a degree of progression of a symptom of nonalcoholic fatty liver disease (NAFLD), the method comprising:

a step of measuring a quantity of VCAM1 contained in blood collected from a subject; and
a step of determining that a degree of hepatic fibrosis, a degree of progression of activity of nonalcoholic fatty liver disease, a degree of progression of hepatocellular ballooning degeneration, or a degree of progression of hepatic inflammation in the subject is higher or possibly higher as the quantity of VCAM1 is larger.

**20.** A kit for use in the method according to any one of claims 16 to 19, the kit comprising:

a reagent for detecting VCAM1.

**21.** A program for use in the method according to any one of claims 1 to 14, wherein the program allows a computer to execute:

a normalization step of calculating a normalized score or normalized scores from measurement data on a quantity or quantities of the marker molecule or marker molecules contained in blood collected from a subject by using the formula 1 and then determining an index value from the normalized score or normalized scores calculated;
a comparison step of comparing the index value determined with a reference value; and
a determination step of determining whether the subject has a symptom of a hepatic disease based on a result of the comparison.

**22.** A program for use in the method according to any one of claims 16 to 19, wherein the program allows a computer to execute:

a comparison step of comparing a quantity of VCAM1 contained in blood collected from a subject with a reference value; and
a determination step of determining whether the subject has a symptom of a hepatic disease based on a result of the comparison.

**23.** The program according to claim 21 or 22, wherein the program allows a computer to further execute an acquisition step of acquiring measurement data on a quantity or quantities of the marker molecule or marker molecules contained in blood collected from a subject or a quantity of the VCAM1 contained in blood collected from a subject.

**24.** A measurement apparatus for use in the method according to any one of claims 1 to 14 and 16 to 19, the measuring apparatus comprising:

a sample-setting part configured to set a sample therein; a light source part configured to irradiate the sample with light; and a detection part configured to detect light transmitted through the sample.

**25.** A hepatic disease determination system for use in the method according to any one of claims 1 to 14, the hepatic disease determination system comprising:

a measurement apparatus configured to measure a quantity or quantities of the marker molecule or marker molecules contained in blood collected from a subject; and a hepatic disease determination apparatus configured to determine presence of a symptom of a hepatic disease based on the quantity or quantities of the marker molecule or marker molecules measured, wherein
the measuring apparatus comprises a sample-setting part configured to set a sample therein, a light source part configured to irradiate the sample with light, and a detection part configured to detect light transmitted through the sample, and
the hepatic disease determination apparatus comprises an acquisition unit configured to acquire measurement data from the measurement apparatus, a normalization unit configured to calculate a normalized score or normalized scores from the measurement data by using the formula 1 and then determine an index value from the normalized score or normalized scores calculated, a comparison unit configured to compare the index value determined with a reference value, and a determination unit configured to determine whether the subject has a symptom of a hepatic disease based on a result of the comparison.

26. A hepatic disease determination system for use in the method according to any one claims 16 to 19, the hepatic disease determination system comprising:

a measurement apparatus configured to measure a quantity of VCAM1 contained in blood collected from a subject; and a hepatic disease determination apparatus configured to determine presence of a symptom of a hepatic disease based on the quantity of VCAM1 measured, wherein
the measuring apparatus comprises a sample-setting part configured to set a sample therein, a light source part configured to irradiate the sample with light, and a detection part configured to detect light transmitted through the sample, and
the hepatic disease determination apparatus comprises an acquisition unit configured to acquire measurement data from the measurement apparatus, a comparison unit configured to compare the quantity of VCAM1 with a reference value, and a determination unit configured to determine whether the subject has a symptom of a hepatic disease based on a result of the comparison.

27. A diagnostic reagent for determining whether a subject is affected with or possibly affected with nonalcoholic steatohepatitis (NASH), a subject has or possibly has a symptom of hepatic fibrosis, a degree of progression of hepatic fibrosis is high or possibly high, a degree of progression of a symptom of hepatocellular ballooning degeneration is high or possibly high, a degree of progression of hepatic inflammation is high or possibly high, a subject has or possibly has a symptom of hepatomegaly, a subject has or possibly has a symptom of hepatocellular necrosis, a subject has or possibly has a symptom of hepatocellular apoptosis, a subject has or possibly has a symptom of hepatocellular degeneration, or a subject has or possibly has a symptom of hepatic steatosis, the diagnostic reagent comprising a reagent for detecting VCAM1.

28. The diagnostic reagent according to claim 27, wherein the reagent for detecting VCAM1 is an antibody.

29. Use of a reagent for detecting VCAM1, for in-vitro detection of nonalcoholic steatohepatitis (NASH), hepatic fibrosis, hepatocellular ballooning degeneration, hepatic inflammation, hepatomegaly, hepatocellular necrosis, hepatocellular apoptosis, hepatocellular degeneration, or hepatic steatosis.

30. The use according to claim 29, wherein an additional diagnostic reagent is concomitantly used.

31. The use according to claim 30, wherein the additional diagnostic reagent is a diagnostic reagent for detecting HA, COL4, or COL4-7S.

32. A program, wherein the program allows a computer to execute:

a comparison step of comparing an index value determined from measurement data on a quantity or quantities of a marker molecule or marker molecules contained in blood collected from a subject with a reference value, the marker molecule or marker molecules including VCAM1; and
a determination step of determining whether the subject has a symptom of a hepatic disease based on a result of the comparison.

33. The program according to claim 32, wherein the program allows a computer to further execute an acquisition step of acquiring measurement data on a quantity or quantities of the marker molecule or marker molecules contained in blood collected from a subject.

34. The program according to claim 32 or 33, wherein the program allows a computer to further execute a normalization step of calculating a normalized score or normalized scores from the measurement data and then determining an index value from the normalized score or normalized scores calculated.

35. The program according to claim 32 or 33, wherein the marker molecule or marker molecules further include HA, COL4, or COL4-7S.

36. A method for determining an effect of a therapeutic drug for nonalcoholic steatohepatitis (NASH), the method comprising:

a step of measuring a quantity or quantities of a marker molecule or marker molecules contained in blood collected from an NASH patient before and after application of the therapeutic drug to the NASH patient, wherein the marker molecule or marker molecules to be measured is/are VCAM1;, VCAM1 and HA; HA and COL4; VCAM1 and COL4; HA and COL4-7S; or VCAM1 and COL4-7S; and

a step of calculating a normalized score based on the quantity or quantities of the marker molecule or marker molecules before and after the application of the therapeutic drug and then determining that an effect of the application of the therapeutic drug is possibly present in a case that the normalized score after the application is lower than the normalized score before the application, wherein each normalized score is calculated by using the following formula 1:

$$M = \frac{1}{N}\sum_{i=1}^{N}\frac{1}{1 + exp\left(-\alpha_i \times \dfrac{m_i - mean(m_i)}{sd(m_i)}\right)}$$

$$\alpha_i = \begin{cases} 1 & \left(mean(m_i^{NASH}) > mean(m_i^{NAFL})\right) \\ -1 & (otherwise) \end{cases}$$

(formula 1)

wherein M denotes a normalized score; N denotes a number of marker molecules; $m_i$ denotes the quantity of each marker molecule measured; mean $(m_i)$ denotes a predetermined mean of a quantity of each marker molecule; sd $(m_i)$ denotes a predetermined standard deviation for each marker molecule; mean $(m_i^{NASH})$ denotes a predetermined mean of a quantity of each marker molecule in patients presenting with NASH; and mean $(m_i^{NAFL})$ denotes a predetermined mean of a quantity of each marker molecule in patients presenting with NAFL.

37. A method for determining an effect of a therapeutic drug for nonalcoholic steatohepatitis (NASH), the method comprising:

a step of measuring a quantity of VCAM1 contained in blood collected from an NASH patient before and after application of the therapeutic drug to the NASH patient; and

a step of determining that an effect of the application of the therapeutic drug is possibly present in a case that the quantity of VCAM1 after the application is lower than the quantity of VCAM1 before the application.

38. A method for determining an effect of a therapeutic drug for nonalcoholic fatty liver (NAFL) or nonalcoholic steatohepatitis (NASH), the method comprising:

(1) a step of measuring quantities of marker molecules contained in blood collected from a subject of an NAFL or NASH patient before and after application of the therapeutic drug to the NAFL or NASH patient, wherein the marker molecules to be measured are

(A) at least two marker molecules selected from Group 1 consisting of PROS1, CLU, ANG, APOC3, APOD, CFHR1, cortisol, EGFR, HPN, IGFBP3, IL1B, IL23A, MET, MMP10, tetranectin, TTR, VDBP, and VEGFR-2, at least two marker molecules selected from Group 2 consisting of VCAM1, HA, CTSD, COL4, COL4-7S, ALB, AFP, AXL, CCL19, CGB, CSF1, FAS, Mac-2bp, CA19-9, NRCAM, OPG, VWF, and YKL-40, at least two marker molecules selected from Group 3 consisting of AST, PSAT, LEP, ICAM1, CK-18, GSTA1, ALT, INS$^{IVD}$, INS$^{MAP}$, 6Ckine, AGT, CRP, CXCL10, FABP4, G6PI, HSP-60, P3NP, and testosterone, or
at least two marker molecules selected from Group 4 consisting of AREG, BDNF, CD40-L, EREG FGF2, HBEGF, IGFBP2, MMP9, PAI-1, PDGFB, PLGF, SAP, TGFA, TGFB1, THBS 1, and VTN, or,

(B) at least two marker molecules selected from one of two groups selected from the Group 1, the Group 2, the Group 3, and the Group 4 and at least two marker molecules selected from the other;

(2) a step of determining an index value from a normalized score calculated based on the quantities of the marker molecules before and after application of the therapeutic drug to the NAFL or NASH patient, wherein the normalized score based on the quantities of the marker molecules belonging to the same group is calculated by using the following formula 1:

$$M = \frac{1}{N} \sum_{i=1}^{N} \frac{1}{1 + exp\left(-\alpha_i \times \frac{m_i - mean(m_i)}{sd(m_i)}\right)}$$

$$\alpha_i = \begin{cases} 1 & \left(mean(m_i^{NASH}) > mean(m_i^{NAFL})\right) \\ -1 & (otherwise) \end{cases}$$

(formula 1)

wherein M denotes a normalized score; N denotes a number of marker molecules; $m_i$ denotes the quantity of each marker molecule measured; mean $(m_i)$ denotes a predetermined mean of a quantity of each marker molecule; sd $(m_i)$ denotes a predetermined standard deviation for each marker molecule; mean $(m_i^{NASH})$ denotes a predetermined mean of a quantity of each marker molecule for patients affected with NASH; and mean $(m_i^{NAFL})$ denotes a predetermined mean of a quantity of each marker molecule for patients affected with NAFL, and,
in a case that the marker molecules correspond to the (A), the index value is the normalized score, and
in a case that the marker molecules correspond to the (B), the index value is calculated from normalized scores for the two groups; and
(3) a step of determining that an effect of the application of the therapeutic drug is possibly present in a case that the normalized score after the application is lower than the normalized score before the application.

Fig.1

M1(All factors)

M2(All factors)

M3(All factors)

M4(All factors)

M1(All factors)+M2(All factors)

M1(All factors)+M3(All factors)

M1(All factors)+M4(All factors)

M2(All factors)+M3(All factors)

M2(All factors)+M4(All factors)

M3(全因子)+M4(全因子)

Fig.2-1

EP 3 282 256 A1

# Fig.2-2

**M2(VCAM1,HA)**

**M2(VCAM1,COL4-7S)**

**M2(COL4-7S,HA)**

**Measured value of VCAM1 (ng/ml)**

EP 3 282 256 A1

Fig.3

M3(AST,PSAT)   M3(AST,LEP)   M3(AST,ICAM1)

M3(AST,PSAT,LEP)   M3(AST,PSAT,ICAM1)   M3(AST,LEP,CK18)

M3(AST,ICAM1,GSTA1)   M3(AST,PSAT,6Ckine)

NAFL   NASH

*Fig.4*

Fig.5-1

EP 3 282 256 A1

**Fig.5-2**

Fig.6-1

EP 3 282 256 A1

# Fig.6-2

EP 3 282 256 A1

Fig.7

Fig.8

EP 3 282 256 A1

Fig.9

# Fig.10

Fig.11

# Fig.12

```
                                           ⌐⁀11
  ┌─────────────────────────────────────────────┐
  │  Liver disease determination apparatus       │
  │                                      ⌐⁀D1    │
  │   ┌──────────────────────────────────┐       │
  │   │        Acquisition unit          │       │
  │   └──────────────────────────────────┘       │
  │                    │                          │
  │                    ▼                 ⌐⁀D2     │
  │   ┌──────────────────────────────────┐       │
  │   │       Normalization unit         │       │
  │   └──────────────────────────────────┘       │
  │                    │                          │
  │                    ▼                 ⌐⁀D3     │
  │   ┌──────────────────────────────────┐       │
  │   │        Comparison unit           │       │
  │   └──────────────────────────────────┘       │
  │                    │                          │
  │                    ▼                 ⌐⁀D4     │
  │   ┌──────────────────────────────────┐       │
  │   │       Determination unit         │       │
  │   └──────────────────────────────────┘       │
  │                    │                          │
  │                    ▼                 ⌐⁀D5     │
  │   ┌──────────────────────────────────┐       │
  │   │         Display unit             │       │
  │   └──────────────────────────────────┘       │
  │                                               │
  └─────────────────────────────────────────────┘
```

# Fig.13

```
          ┌──────────┐
          │  Start   │
          └──────────┘
               │
   ┌────────────────────────────┐
   │  Acquire measurement data of│── S1
   │   marker molecule quantities│
   └────────────────────────────┘
               │
   ┌────────────────────────────┐
   │   Calculate normalized scores│── S2
   └────────────────────────────┘
               │
   ┌────────────────────────────┐
   │     Compare index values    │── S3
   └────────────────────────────┘
               │
   ┌────────────────────────────┐
   │   Determine hepatic diseases │── S4
   └────────────────────────────┘
               │
   ┌────────────────────────────┐
   │          Display            │── S5
   └────────────────────────────┘
               │
          ┌──────────┐
          │   End    │
          └──────────┘
```

# Fig.14

Normalized score "HA, COL4"

# *Fig.15*

Normalized score "HA, COL4"

## Fig.16

Normalized score "HA, COL4"

Fig.17

Normalized score "HA, COL4"

## INTERNATIONAL SEARCH REPORT

| International application No. |
|---|
| PCT/JP2016/061609 |

A. CLASSIFICATION OF SUBJECT MATTER
*G01N33/68*(2006.01)i, *G01N21/27*(2006.01)i, *G01N33/48*(2006.01)i, *G01N33/483*(2006.01)i, *G01N33/53*(2006.01)i

According to International Patent Classification (IPC) or to both national classification and IPC

B. FIELDS SEARCHED

Minimum documentation searched (classification system followed by classification symbols)
G01N33/68, G01N21/27, G01N33/48, G01N33/483, G01N33/53

Documentation searched other than minimum documentation to the extent that such documents are included in the fields searched
Jitsuyo Shinan Koho        1922–1996    Jitsuyo Shinan Toroku Koho    1996–2016
Kokai Jitsuyo Shinan Koho  1971–2016    Toroku Jitsuyo Shinan Koho    1994–2016

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | JP 2005-519271 A  (Prometheus Laboratories, Inc.), 30 June 2005 (30.06.2005), entire text & US 2003/0175686 A1 entire text & US 2006/0084057 A1    & WO 2003/073822 A2 & EP 1487395 A1 | 1-38 |
| A | JP 2007-315752 A  (Ajinomoto Co., Inc.), 06 December 2007 (06.12.2007), entire text & WO 2006/019037 A1 | 1-38 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 June 2016 (22.06.16) | 05 July 2016 (05.07.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/061609

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2011-252868 A  (Yamaguchi University), 15 December 2011 (15.12.2011), entire text; all drawings (Family: none) | 1-38 |
| A | WO 2010/073870 A1  (Keio University), 01 July 2010 (01.07.2010), entire text & JP 5676275 B2          & JP 2015-57616 A & US 2011/0256561 A1 entire text & US 2013/0330749 A1     & WO 2010/073870 A1 & EP 2381259 A1 | 1-38 |
| A | WO 2009/090882 A1  (The University of Tokyo), 23 July 2009 (23.07.2009), entire text (Family: none) | 1-38 |
| A | WO 2007/122799 A1  (Yamasa Corp.), 01 November 2007 (01.11.2007), entire text & JP 5227168 B2          & US 2009/0111136 A1 entire text & WO 2007/122799 A1      & EP 2003453 A2 | 1-38 |
| A | Yoshio SUMIDA et al., "Noninvasive scoring systems for the diagnosis of NASH/NAFLD", Kan Tan Sui, 28 June 2010 (28.06.2010), vol.60, no.6, pages 963 to 970 | 1-38 |
| P,X | YOSHIMURA K et al., Identification of novel noninvasive markers for diagnosing nonalcoholic steatohepatitis and related fibrosis by data mining., Hepatology., 2016.02, Vol.63,No.2, Page.462-473 | 1-38 |

Form PCT/ISA/210 (continuation of second sheet) (January 2015)

## INTERNATIONAL SEARCH REPORT

| International application No. |
|---|
| PCT/JP2016/061609 |

**Box No. II**      **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:
     See extra sheet.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
     ☐ The additional search fees were accompanied by the applicant's protest and, where applicable, the payment of a protest fee.

     ☐ The additional search fees were accompanied by the applicant's protest but the applicable protest fee was not paid within the time limit specified in the invitation.

     ☒ No protest accompanied the payment of additional search fees.

Form PCT/ISA/210 (continuation of first sheet (2)) (January 2015)

**INTERNATIONAL SEARCH REPORT**

International application No.

PCT/JP2016/061609

Continuation of Box No.III of continuation of first sheet(2)

It is considered that the inventions of claims 1-38 comprise the following nine invention groups.

1st Invention: it is regarded that the technical feature of the inventions described in claims 1-15, 21, 23-25, 36 and 38 resides in a matter that the amount of a marker molecule contained in blood collected from a subject is measured, an index value is determined from the molecular weight and a normalized score calculated in accordance with formula 1, and then it is determined whether the subject is suffering from NASH, or the subject has a clinical symptom of hepatic fibrosis, or there is a high probability of occurring one of the above-mentioned conditions on the basis of the degrees of the index value and a reference value, and a matter that the efficacy of a therapeutic agent for NASH or the like is determined by the method.

2nd Invention: it is regarded that the technical feature of the invention described in claim 16 resides in a matter that the amount of VCAM1 contained in blood collected from a subject is measured and then it is determined that the subject is suffering from NASH or there is a possibility that the subject is suffering from NASH when the amount of VCAM1 is greater than a reference value.

3rd Invention: it is regarded that the technical feature of the invention described in claim 17 resides in a matter that the amount of VCAM1 contained in blood collected from a subject is measured and then it is determined that the subject has a clinical symptom of hepatic fibrosis or there is a possibility that the subject has a clinical symptom of hepatic fibrosis when the amount of VCAM1 is greater than a reference value.

4th Invention: it is regarded that the technical feature of the invention described in claim 18 resides in a matter that the amount of VCAM1 contained in blood collected from a subject is measured and then it is determined that the degree of progression of hepatic fibrosis, the degree of progression of ballooning degeneration of hepatocytes or the degree of progression of inflammation of liver in the subject increases or there is a possibility of the increase in any one of the above-mentioned progressions in the subject with the increase in the amount of VCAM1.

5th Invention: it is regarded that the technical feature of the inventions described in claims 19, 20, 22, 23 and 26 resides in a matter that the amount of VCAM1 contained in blood collected from a subject is measured and then it is determined that the degree of progression of hepatic fibrosis, the degree of progression of activity of a non-alcoholic fatty liver disease, the degree of progression of ballooning degeneration of hepatocytes or the degree of progression of inflammation of liver in the subject increases or there is a possibility of the increase in any one of the above-mentioned progressions in the subject with the increase in the amount of VCAM1.

(Continued to next extra sheet)

Form PCT/ISA/210 (extra sheet) (January 2015)

## INTERNATIONAL SEARCH REPORT

International application No.

PCT/JP2016/061609

6th Invention: it is regarded that the technical feature of the inventions described in claims 27 and 28 resides in a reagent for detecting VCAM1, wherein the reagent can be used as a diagnosis agent for determining whether there is a clinical symptom of fibrosis of liver suffering from non-alcoholic steatohepatitis (NASH), the degree of progression of hepatic fibrosis is high, the degree of progression of a clinical symptom of ballooning degeneration of hepatocytes is high, the degree of progression of inflammation of liver is high, there is a clinical symptom of hepatomegaly, there is a clinical symptom of necrosis of hepatocytes, there is a clinical symptom of apoptosis of hepatocytes, there is a clinical symptom of degeneration of hepatocytes, there is a clinical symptom of fatty deposition in liver or there is a possibility of any one of the above-mentioned conditions.

7th Invention: it is regarded that the technical feature of the inventions described in claims 29-31 resides in a matter that a reagent for detecting VCAM1 is used for the *in vitro* detection of non-alcoholic steatohepatitis (NASH), fibrosis of liver, ballooning degeneration of hepatocytes, inflammation of liver, hepatomegaly, necrosis of hepatocytes, apoptosis of hepatocytes, degeneration of hepatocytes, or fatty deposition in liver.

8th Invention: it is regarded that the technical feature of the inventions described in claims 32-35 resides in a program for carrying out a comparison step of measuring the amount of VCAM1 molecules contained in blood collected from a subject to determine an index value and then comparing the index value with a reference value and a determination step of determining whether the subject has a clinical symptom of a liver disease on the basis of the result of the comparison.

9th Invention: it is regarded that the technical feature of the invention described in claim 37 resides in a matter that the efficacy of a therapeutic agent for non-alcoholic steatohepatitis (NASH) is determined by carrying out the step of measuring the amount of VCAM1 contained in blood collected from an NASH patient before and after the application of the therapeutic agent to the patient and the step of determining that there is a possibility that the application of the therapeutic agent is effective when the amount of VCAM1 after the application of the therapeutic agent is reduced compared with that before the application of the therapeutic agent.

Therefore, there is no single general inventive concept between the 1st Invention and the 2nd to 9th Inventions.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **POYNARD, T. et al.** Diagnostic value of biochemical markers (NashTest) for the prediction of non alcoholo steato hepatitis in patients with non-alcoholic fatty liver disease. *BMC Gastroenterol.,* 2006, vol. 6, 34
- **YOUNOSSI, Z. M. et al.** A novel diagnostic biomarker panel for obesity-related nonalcoholic steatohepatitis (NASH). *Obes. Surg.,* 2008, vol. 18, 1430-7
- **SUMIDA, Y. et al.** A simple clinical scoring system using ferritin, fasting insulin, and type IV collagen 7S for predicting steatohepatitis in nonalcoholic fatty liver disease. *J. Gastroenterol.,* 2011, vol. 46, 257-68
- Evidence-based Clinical Practice Guidelines for NAFLD/NASH 2014. Nankodo Co., Ltd, 2014

- *J Hepatol.,* January 2005, vol. 42 (1), 132-8
- **MATTEONI, C. ; YOUNOSSI, Z. ; GRAMLICH, T.** Nonalcoholic fatty liver disease: a spectrum of clinical and pathological severity. *Gastroenterology,* 1999, 1413-1419
- **KLEINER.** Design and validation of a histological scoring system for nonalcoholic fatty liver disease. *Hepatology,* 2005, vol. 41, 1313-21
- *Hepatology,* June 2006, vol. 43 (6), 1317-25
- *Hepatogastroenterology,* March 2014, vol. 61 (130), 422-5
- *J Clin Gastroenterol.,* July 2010, vol. 44 (6), 440-7